# EUROPEAN PATENT APPLICATION

(11) **EP 4 617 279 A1**
(43) Date of publication of application: **17.09.2025**
(21) Application number: 23888791.3
(22) Date of filing: 10.11.2023
(51) Int. Cl.: C07H 19/06, A61K 48/00, C07C 335/38, C07H 19/16

(54) **MODIFIED NUCLEOSIDE HAVING GUANIDINO STRUCTURE IN BRIDGED PART AND METHOD FOR PRODUCING OLIGONUCLEOTIDE USING SAME**

(30) Priority: 10.11.2022 JP 2022180647
(71) Applicant: Luxna Biotech Co., Ltd., Suita-shi, Osaka 565-0871 (JP); Nissan Chemical Corporation, Tokyo 103-6119 (JP)
(72) Inventor: SHRESTHA, Ajaya Ram, Suita-shi, Osaka 565-0871 (JP); UMEMOTO, Tadashi, Suita-shi, Osaka 565-0871 (JP); MAEKAWA, Tsuyoshi, Suita-shi, Osaka 565-0871 (JP); NAKANO, Satoshi, Funabashi-shi, Chiba 274-8507 (JP)
(74) Representative: Müller-Boré & Partner Patentanwälte PartG mbB
(86) International application number: PCT/JP2023/040535
(87) International publication number: WO 2024/101446

(57) **Abstract**

A compound of the present invention or a salt thereof has a structure represented by a formula (I) and has excellent chemical stability. The present invention is useful, for example, as a material for nucleic acid drugs used in antisense therapies, antigene therapies, aptamer-based therapies, siRNA-based therapies, and the like, which are expected to be novel methods for treating or preventing a disease.

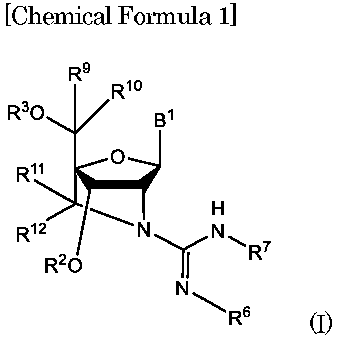

## Description

### Technical Field

The present invention relates to a modified nucleoside having a guanidino structure in a bridged part and a method for producing an oligonucleotide using the same.

### Background Art

Examples of methods for treating diseases using nucleic acid drugs include antisense therapies, antigene therapies, aptamer-based therapies, siRNA-based therapies, and the like. Of these, the antisense therapies are approaches for treating or preventing diseases, involving inhibiting a translation process of disease-causing RNAs by externally introducing oligonucleotides (antisense strands) that are complementary to disease-associated mRNAs to form duplexes. The siRNA-based therapies are similar to the antisense therapies, involving inhibiting translation from mRNAs to proteins by administering duplex RNAs into a living body. Meanwhile, the antigene therapies suppress transcription from DNAs to RNAs by externally introducing triplex-forming oligonucleotides corresponding to DNA sites that are to be transcribed into pathogenic RNAs. In addition, aptamers are short nucleic acid molecules (oligonucleotides) and exert their function by binding to bioloical components such as disease-associated proteins.

Various artificial nucleic acids have been developed as materials for such nucleic acid drugs. Examples of the materials developed for nucleic acid drugs to date include phosphorothioate (S-PO₃) oligonucleotide (S-oligo), 2',4'-bridged nucleic acid (BNA)/2',4'-locked nucleic acid (LNA) (Patent Documents 1 to 4 and Non-Patent Documents 1 to 4), and the like. Of these, S-oligo has high nuclease resistance, but has the drawback of low binding affinity towards target nucleic acid strand, and therefore, improvement is required. 2',4'-BNA/2',4'-LNA has high binding affinity towards target nucleic acid strands, and provides the most promising molecules as the materials for the future nucleic acid drugs. However, there is still room for improvement, as the nuclease resistance is not sufficient and the stability in a living body is poor.

Thus, there has been a demand for nucleic acid molecules for oligonucleotides that have high binding affinity and specificity for target nucleic acids and exhibit high nuclease resistance. To address this, in recent years, new nucleic acids in which a guanidine structure is introduced into the bridged part of the nucleic acid and improvements thereon have been proposed (Patent Document 5 and Non-Patent Documents 6 to 8).

However, none of the nucleic acid molecules described in Patent Document 5 and Non-Patent Documents 6 to 8 are sufficiently stable in terms of maintaining industrial productivity, and therefore, further improvements are desired.

### Related Art Documents

### Patent Documents

| | |
|---|---|
| [Patent Document 1] | WO 98/39352 |
| [Patent Document 2] | WO 2005/021570 |
| [Patent Document 3] | WO 2003/068795 |
| [Patent Document 4] | WO 2011/052436 |
| [Patent Document 5] | WO 2014/046212 |

### Non-Patent Documents

[Non-Patent Document 1] C. Wahlestedt et al., Proc. Natl. Acad. Sci. USA, 2000, Vol. 97, No. 10, pp. 5633-5638
[Non-Patent Document 2] Y. Hari et al., Bioorg. Med. Chem., 2006, Vol., pp. 1029-1038
[Non-Patent Document 3] K. Miyashita et al., Chem. Commun., 2007, pp. 3765-3767
[Non-Patent Document 4] S.M.A. Rahman et al., J. Am. Chem. Soc., 2008, Vol. 130, No. 14, 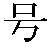 , pp. 4886-4896
[Non-Patent Document 5] M. Kuwahara et al., Nucleic Acids Res., 2008, Vol. 36, No. 13, pp. 4257-4265
[Non-Patent Document 6] S. Obika et al., Bioorg. Med. Chem., 2001, Vol. 9, pp. 1001-1011
[Non-Patent Document 7] A.R. Shreshta al., Chem. Commun., 2014, Vol. 50, No. 5, pp. 575-577
[Non-Patent Document 8] S. Kumagai et al., Org. Biomol. Chem., 2020, Vol. 18, No. 46 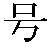 , pp. 9461-9472

### Summary of Invention

### Problem to be Solved by the Invention

The present invention was made to address the above problems and it is an object thereof to provide a modified nucleoside, an intermediate thereof, and a modified nucleotide that have a guanidino structure in a bridged part and have excellent chemical stability, as well as to provide methods for producing the modified nucleoside, intermediate thereof, and modified nucleotide. Furthermore, a method for producing an oligonucleotide using the modified nucleoside and/or the modified nucleotide is provided.

### Means for Solving the Problem

The present invention provides a compound represented by a formula (I) below or a salt thereof: 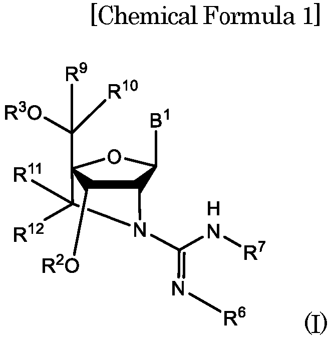 (where in the formula (I),
B¹ is
   (a) a purin-9-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms; or
   (b) a 1,2-dihydropyrimidin-1-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
   R² and R³ are each independently
      (a) a hydrogen atom;
      (b) a hydroxy group protecting group for nucleic acid synthesis;
      (c) an alkyl group having 1 to 7 carbon atoms that may form a branch or a ring;
      (d) an alkenyl group having 2 to 7 carbon atoms that may form a branch or a ring;
      (e) an aryl group having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (f) a heteroaryl group having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (g) an aralkyl group with an aryl moiety having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (h) a heteroaralkyl group with a heteroaryl moiety having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (i) an acyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (j) a silyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (k) a phosphate group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (l) a phosphate group protected by a protecting group for nucleic acid synthesis; or
      (m)-P(R⁴)R⁵,
         (where
      R⁴ and R⁵ are each independently
         (a) a hydroxy group;
         (b) a hydroxy group protected by a protecting group for nucleic acid synthesis;
         (c) a mercapto group;
         (d) a mercapto group protected by a protecting group for nucleic acid synthesis;
         (e) an amino group;
         (f) an alkoxy group having 1 to 5 carbon atoms;
         (g) an alkylthio group having 1 to 5 carbon atoms;
         (h) a cyanoalkoxy group having 1 to 6 carbon atoms;
         (i) a dialkylamino group having alkyl groups that each have 1 to 6 carbon atoms and may be the same or different; or
         (j) a cyclic alkylamino group having 3 to 6 carbon atoms; or
         R⁴ and R⁵ together constitute a formula below: 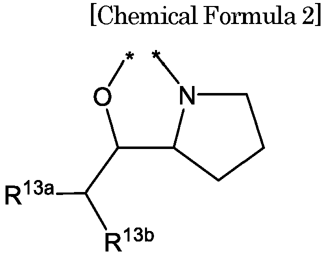
(where R^{13a} and R^{13b} are each independently a hydrogen atom, an alkyl group having 1 to 6 carbon atoms that may be branched, an aryl group having 6 to 12 carbon atoms, a nitro group, a halogen atom, a cyano group, Si(R^{a})₃ (where R^{a} is an alkyl group having 1 to 6 carbon atoms or an aryl group having 6 to 12 carbon atoms), a sulfonyl group, SO₂Ph, or SiMePh₂, and * is a bonding site); or 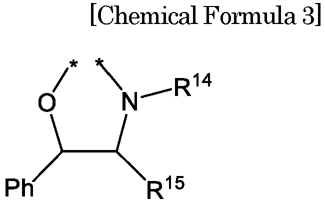
(where R¹⁴ and R¹⁵ are each independently a hydrogen atom, an alkyl group having 1 to 3 carbon atoms that may be branched, or a phenyl group, and * is a bonding site);
R⁶ is a group represented by a formula below: 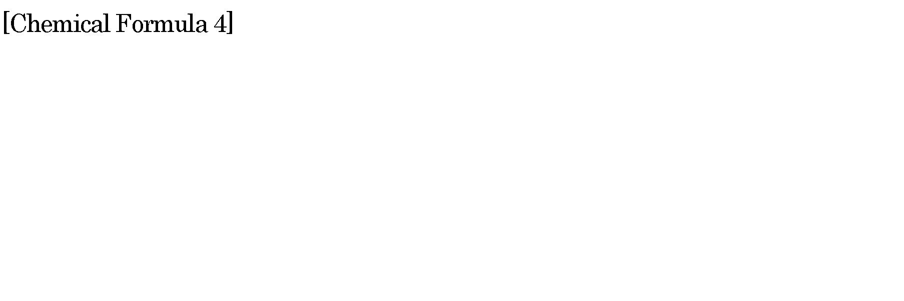 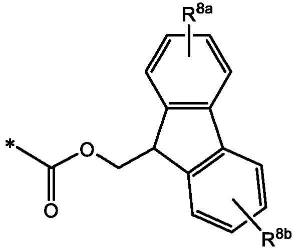
(where
   R^{8a} and R^{8b} are each independently
      (a) an alkyl group having 1 to 9 carbon atoms that may form a branch or a ring;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
      * is a bonding site); and
   R⁷ is
      (a) an alkyl group having 1 to 8 carbon atoms that may form a branch or a ring and may have undergone substitution by an alkoxy group having 1 to 8 carbon atoms that may form a branch or a ring;
      (b) a cyanoalkoxy group having 1 to 8 carbon atoms;
      (c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
      (d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
      (e) a group represented by a formula below: 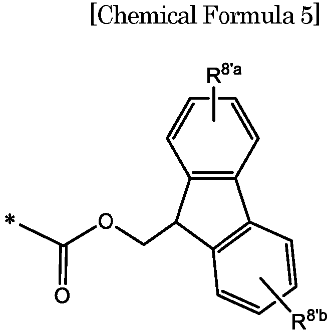
(where
   R^{8'a} and R^{8'b} are each independently
      (a) an alkyl group having 1 to 9 carbon atoms that may be branched;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
      * is a bonding site); or
   R⁶ is:
      (a) an alkyl group having 1 to 8 carbon atoms that may form a branch or a ring and may have undergone substitution by an alkoxy group having 1 to 8 carbon atoms that may form a branch or a ring;
      (b) a cyanoalkoxy group having 1 to 8 carbon atoms;
      (c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
      (d) a heteroaralkyl group with a heteroaryl moiety having 3 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
      (e) a group represented by a formula below: 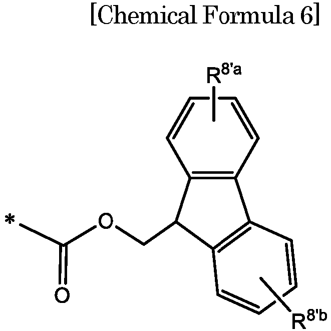
(where
   R^{8'a} and R^{8'b} are each independently
      (a) an alkyl group having 1 to 9 carbon atoms that may be branched;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, linear alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
      * is a bonding site); and
   R⁷ is a group represented by a formula below: 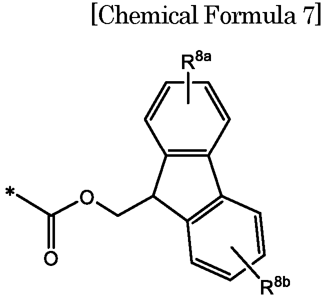
(where
   R^{8a} and R^{8b} are each independently
      (a) an alkyl group having 1 to 9 carbon atoms that may be branched;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
      * is a bonding site); and
   R⁹ and R¹⁰ are each independently a hydrogen atom or an alkyl group having 1 to 6 carbon atoms that may form a branch or a ring, or R⁹ and R¹⁰ together constitute - (CH₂)ₘ- (where m is an integer from 2 to 7), and
   R¹¹ and R¹² are each independently a hydrogen atom or an alkyl group having 1 to 6 carbon atoms that may form a branch or a ring, or
   R¹¹ and R¹² together constitute an oxygen atom part (=O) of a carbonyl group, or
   R¹¹ and R¹² together constitute -(CH₂)ₙ- (where n is an integer from 2 to 7).

In one embodiment, in the formula (I), R⁶ is a group represented by a formula below: 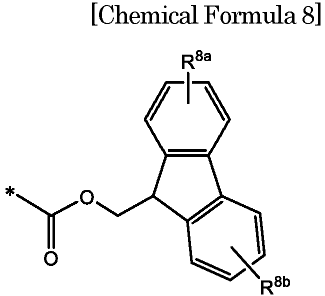 (where
R^{8a} and R^{8b} are each independently
   (a) an alkyl group having 1 to 9 carbon atoms that may be branched;
   (b) an alkoxy group having 1 to 8 carbon atoms that may form a branch or a ring;
   (c) an alkylamino group having 1 to 8 carbon atoms that may form a branch or a ring; or
   (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
   * is a bonding site); and
R⁷ is
   (a) an alkyl group having 1 to 8 carbon atoms that may form a branch or a ring and may have undergone substitution by an alkoxy group having 1 to 8 carbon atoms that may form a branch or a ring;
   (b) a cyanoalkoxy group having 1 to 8 carbon atoms;
   (c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
   (d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
   (e) a group represented by a formula below: 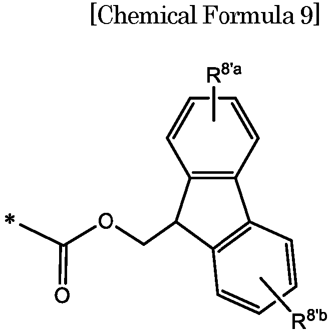 (where
      R^{8'a} and R^{8b} are each independently
      (a) an alkyl group having 1 to 9 carbon atoms that may be branched;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
      * is a bonding site).

In a further embodiment, the formula (I) is represented by a formula (I') below:

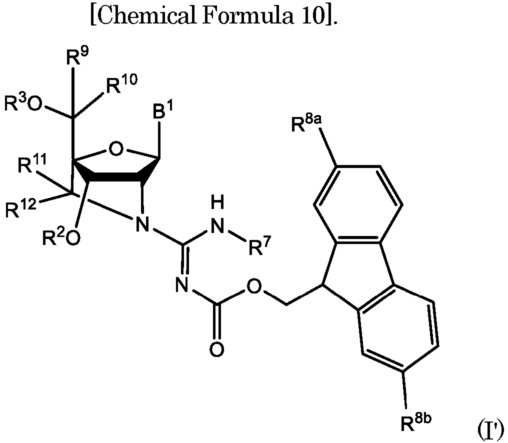

In a further embodiment, R⁷ in the formula (I) is an alkyl group having 1 to 8 carbon atoms that may form a branch or a ring.

In a further embodiment, both R^{8a} and R^{8b} in the formula (I) are each an alkyl group having 3 to 8 carbon atoms that may form a branch or a ring.

In a more further embodiment, both R^{8a} and R^{8b} in the formula (I) are each a t-butyl group.

The present invention also provides a method for producing an oligonucleotide or a pharmacologically acceptable salt thereof, the method comprising a step of synthesizing a nucleic acid using the above compound or salt thereof.

In one embodiment, the oligonucleotide is an oligonucleotide containing at least one phosphorothioate bond.

The present invention also provides a compound represented by a formula (II) below or a salt thereof: 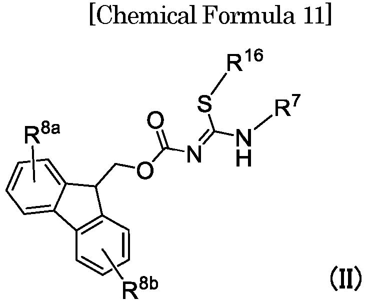 (where in the formula (II),
R⁷ is
(a) an alkyl group having 1 to 8 carbon atoms that may form a branch or a ring and may have undergone substitution by an alkoxy group having 1 to 8 carbon atoms that may form a branch or a ring;
(b) a cyanoalkoxy group having 1 to 8 carbon atoms;
(c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;

(d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
(e) a group represented by a formula below: 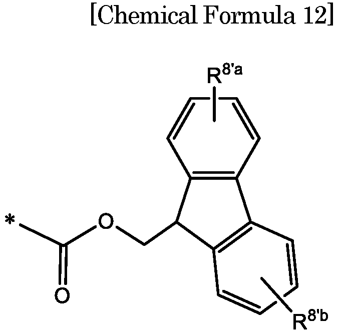 (where
   R^{8'a} and R^{8'b} are each independently
      (a) an alkyl group having 1 to 9 carbon atoms that may be branched;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
      * is a bonding site); and
   R^{8a} and R^{8b} are each independently
      (a) an alkyl group having 1 to 9 carbon atoms that may form a branch or a ring;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
   R¹⁶ is a linear or branched alkyl group having 1 to 6 carbon atoms or a linear or branched alkenyl group having 2 to 6 carbon atoms).

The present invention provides a method for producing a compound represented by a formula (II) or a salt thereof, 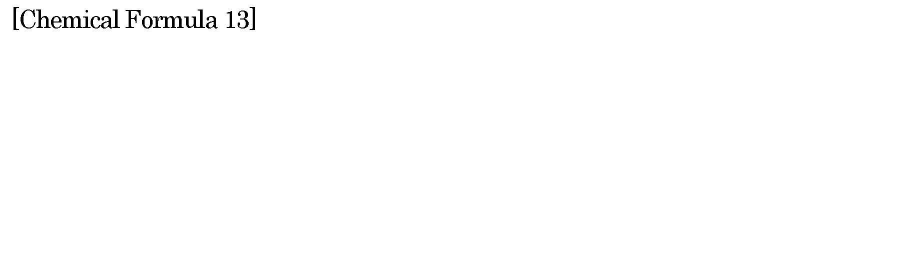 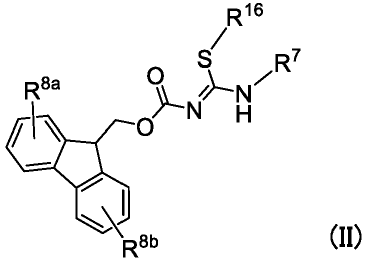 the method comprising a step of allowing a reaction product of a compound represented by a formula (III) below and a compound represented by a formula (IV) below to react with a compound represented by a formula (V) below: 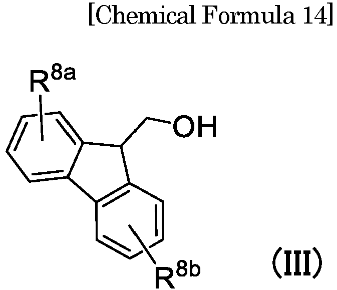 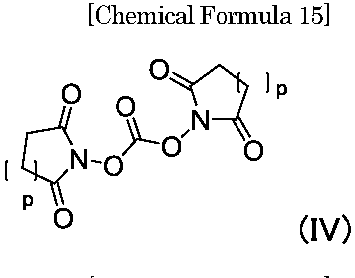 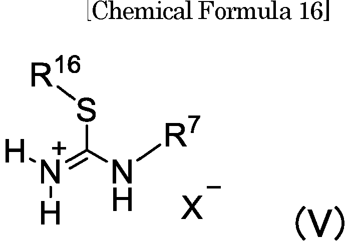 (where in the formula (III),
R^{8a} and R^{8b} are each independently
   (a) an alkyl group having 1 to 9 carbon atoms that may form a branch or a ring;
   (b) an alkoxy group having 1 to 8 carbon atoms that may form a branch or a ring;
   (c) an alkylamino group having 1 to 8 carbon atoms that may form a branch or a ring; or
   (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis;
in the formula (IV),
p is independently an integer from 0 to 3,
in the formula (V),
R⁷ is
   (a) an alkyl group having 1 to 8 carbon atoms that may form a branch or a ring and may have undergone substitution by an alkoxy group having 1 to 8 carbon atoms that may form a branch or a ring;
   (b) a cyanoalkoxy group having 1 to 8 carbon atoms;
   (c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
   (d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
   (e) a group represented by a formula below: 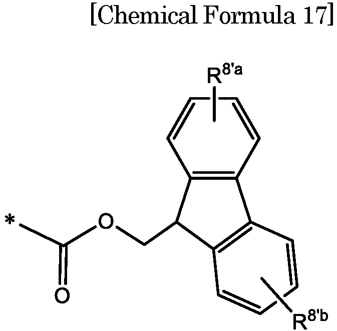 (where
      R^{8'a} and R^{8'b} are each independently
         (a) an alkyl group having 1 to 9 carbon atoms that may be branched;
         (b) an alkoxy group having 1 to 8 carbon atoms that may form a branch or a ring;
         (c) an alkylamino group having 1 to 8 carbon atoms that may form a branch or a ring; or
         (d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
      * is a bonding site); and
      R¹⁶ is a linear or branched alkyl group having 1 to 6 carbon atoms or a linear or branched alkenyl group having 2 to 6 carbon atoms, and
      X⁻ is a counter anion.

The present invention also provides a method for producing the compound represented by the formula (I) or salt thereof according to claim 1, the method comprising
a step of guanidinylating a compound represented by a formula (VI) below or a salt thereof: 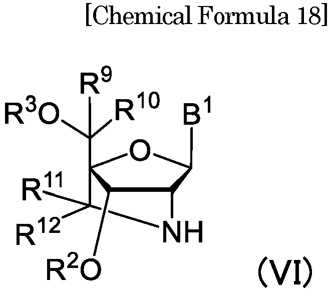
with a compound represented by a formula (II) or a salt thereof: 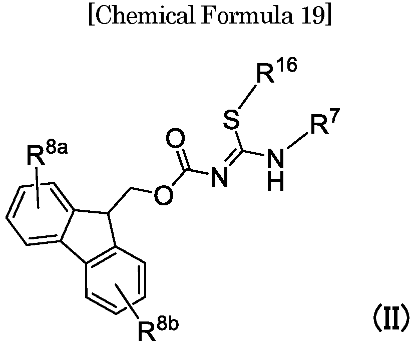
in the formula (VI),
   B¹ is
      (a) a purin-9-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms; or
      (b) a 1,2-dihydropyrimidin-1-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
   R² and R³ are each
      (a) a hydrogen atom;
      (b) a hydroxy group protecting group for nucleic acid synthesis;
      (c) an alkyl group having 1 to 7 carbon atoms that may form a branch or a ring;
      (d) an alkenyl group having 2 to 7 carbon atoms that may form a branch or a ring;
      (e) an aryl group having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (f) a heteroaryl group having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (g) an aralkyl group with an aryl moiety having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (h) a heteroaralkyl group with a heteroaryl moiety having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (i) an acyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (j) a silyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
      (k) a phosphate group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms; or
   (l) a phosphate group protected by a protecting group for nucleic acid synthesis;
   R⁹ and R¹⁰ are each independently a hydrogen atom or an alkyl group having 1 to 6 carbon atoms that may form a branch or a ring, or R⁹ and R¹⁰ together constitute - (CH₂)ₘ- (where m is an integer from 2 to 7), and
   R¹¹ and R¹² are each independently a hydrogen atom or an alkyl group having 1 to 6 carbon atoms that may form a branch or a ring, or
   R¹¹ and R¹² together constitute an oxygen atom part (=O) of a carbonyl group, or
   R¹¹ and R¹² together constitute -(CH₂)ₙ- (where n is an integer from 2 to 7), and
   in the formula (II),
   R⁷ is
      (a) an alkyl group having 1 to 8 carbon atoms that may form a branch or a ring and may have undergone substitution by an alkoxy group having 1 to 8 carbon atoms that may form a branch or a ring;
      (b) a cyanoalkoxy group having 1 to 8 carbon atoms;
      (c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
      (d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
      (e) a group represented by a formula below: 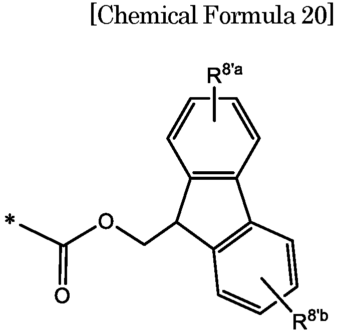 (where
         R^{8'a} and R^{8'b} are each independently
            (a) an alkyl group having 1 to 9 carbon atoms that may be branched;
            (b) an alkoxy group having 1 to 8 carbon atoms that may form a branch or a ring;
            (c) an alkylamino group having 1 to 8 carbon atoms that may form a branch or a ring; or
            (d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
            * is a bonding site); and
         R^{8a} and R^{8b} are each independently
            (a) an alkyl group having 1 to 9 carbon atoms that may form a branch or a ring;
            (b) an alkoxy group having 1 to 8 carbon atoms that may form a branch or a ring;
            (c) an alkylamino group having 1 to 8 carbon atoms that may form a branch or a ring; or
            (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
         R¹⁶ is a linear or branched alkyl group having 1 to 6 carbon atoms or a linear or branched alkenyl group having 2 to 6 carbon atoms.

In one embodiment, the guanidinylation step is carried out in the presence of a metal salt.

In a further embodiment, the metal salt is silver nitrate, silver chloride, silver bromide, silver iodide, silver trifluoromethanesulfonate, silver tetrafluoroborate, or silver hexafluorophosphate.

In a further embodiment, the metal salt is copper (I) chloride, copper (I) bromide, copper (I) iodide, copper (I) trifluoromethanesulfonate, copper (I) tetrafluoroborate, or copper (I) hexafluorophosphate.

In one embodiment, R² in the formula (VI) is a hydrogen atom.

In one embodiment, the method comprises a step of further converting, after the guanidinylation step, the resulting product into an amidite,
R² in the formula (I) being -P(R⁴)R⁵
   (where
   R⁴ and R⁵ are each independently
      (a) a hydroxy group;
      (b) a hydroxy group protected by a protecting group for nucleic acid synthesis;
      (c) a mercapto group;
      (d) a mercapto group protected by a protecting group for nucleic acid synthesis;
      (e) an amino group;
      (f) an alkoxy group having 1 to 5 carbon atoms;
      (g) an alkylthio group having 1 to 5 carbon atoms;
      (h) a cyanoalkoxy group having 1 to 6 carbon atoms;
      (i) a dialkylamino group having alkyl groups that each have 1 to 6 carbon atoms and may be the same or different; or
      (j) a cyclic alkylamino group having 3 to 6 carbon atoms; or
   R⁴ and R⁵ together constitute a formula below: 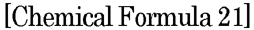 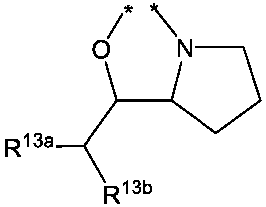
(where R^{13a} and R^{13b} are each independently a hydrogen atom, an alkyl group having 1 to 6 carbon atoms that may be branched, an aryl group having 6 to 12 carbon atoms, a nitro group, a halogen atom, a cyano group, Si(R^{a})₃ (where R^{a} is an alkyl group having 1 to 6 carbon atoms or an aryl group having 6 to 12 carbon atoms), a sulfonyl group, SO₂Ph, or SiMePh₂, and * is a bonding site); or 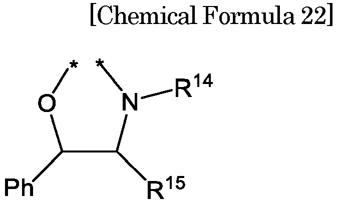
(where R¹⁴ and R¹⁵ are each independently a hydrogen atom, an alkyl group having 1 to 3 carbon atoms that may be branched, or a phenyl group, and * is a bonding site).

In one embodiment, the method further comprises a step of deprotecting a hydroxy group protecting group for nucleic acid synthesis, the protecting group being R² in the formula (VI).

In a further embodiment, the method comprises a step of further converting, after the deprotection step, the resulting product into an amidite,
R² in the formula (I) being -P(R⁴)R⁵
   (where
   R⁴ and R⁵ are each independently
      (a) a hydroxy group;
      (b) a hydroxy group protected by a protecting group for nucleic acid synthesis;
      (c) a mercapto group;
      (d) a mercapto group protected by a protecting group for nucleic acid synthesis;
      (e) an amino group;
      (f) an alkoxy group having 1 to 5 carbon atoms;
      (g) an alkylthio group having 1 to 5 carbon atoms;
      (h) a cyanoalkoxy group having 1 to 6 carbon atoms;
      (i) a dialkylamino group having alkyl groups that each have 1 to 6 carbon atoms and may be the same or different; or
      (j) a cyclic alkylamino group having 3 to 6 carbon atoms; or
   R⁴ and R⁵ together constitute a formula below: 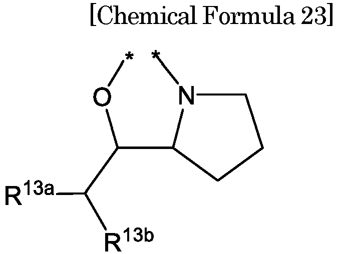
(where R^{13a} and R^{13b} are each independently a hydrogen atom, an alkyl group having 1 to 6 carbon atoms that may be branched, an aryl group having 6 to 12 carbon atoms, a nitro group, a halogen atom, a cyano group, Si(R^{a})s (where R^{a} is an alkyl group having 1 to 6 carbon atoms or an aryl group having 6 to 12 carbon atoms), a sulfonyl group, SO₂Ph, or SiMePh₂, and * is a bonding site); or 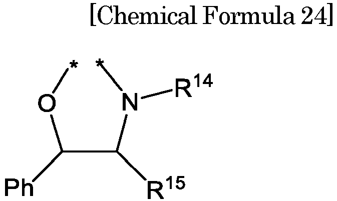
(where R¹⁴ and R¹⁵ are each independently a hydrogen atom, an alkyl group having 1 to 3 carbon atoms that may be branched, or a phenyl group, and * is a bonding site).

### Effects of the Invention

According to the present invention, it is possible to increase the stability of a protecting group that constitutes the guanidino structure of the bridged part. The compound of the present invention can also provide nucleic acid molecules for oligonucleotides that have a favorable binding affinity to target nucleic acids and exhibit a favorable nuclease resistance.

### Brief Description of Drawings

FIG. 1 is a ¹H-NMR spectrum of a compound (compound a2) obtained in Example 1(1-1(1)).
FIG. 2 is a ¹H-NMR spectrum of a compound (amidite a3) obtained in Example 1(1-2).
FIG. 3 is a ¹H-NMR spectrum of a compound (compound b2) obtained in Example 2(2-1(1)).
FIG. 4 is a ¹H-NMR spectrum of a compound (amidite b3) obtained in Example 2(2-2).
FIG. 5 is a ¹H-NMR spectrum of a compound (compound c1) obtained in Example 3(3-1).
FIG. 6 is a ¹H-NMR spectrum of a compound (compound c2) obtained in Example 3(3-2).
FIG. 7 is a ¹H-NMR spectrum of a compound (compound c3) obtained in Example 3(3-3).
FIG. 8 is a ¹H-NMR spectrum of a compound (compound c4) obtained in Example 3(3-4).
FIG. 9 is a ¹H-NMR spectrum of a compound (amidite c5)) obtained in Example 3(3-5).
FIG. 10 is a ¹H-NMR spectrum of a compound (compound d2) obtained in Example 4(4-1(1)).
FIG. 11 is a ¹H-NMR spectrum of a compound (amidite d3) obtained in Example 4(4-2).
FIG. 12 is the result of UPHLC-MS of the compound a3 in a stability test carried out in Example 5.
FIG. 13 is the result of UPHLC-MS of the compound a3' in the stability test carried out in Example 5.
FIG. 14 is a ¹H-NMR spectrum of a compound (compound e4) obtained in Example 6(6-2).
FIG. 15 is a ¹H-NMR spectrum of a compound (compound e5) obtained in Example 6(6-3).
FIG. 16 is a ¹H-NMR spectrum of a compound (compound e6) obtained in Example 6(6-4).
FIG. 17 is a ¹H-NMR spectrum of a compound (compound e7) obtained in Example 6(6-5).
FIG. 18 is a ¹H-NMR spectrum of a compound (compound B2)) obtained in Example 8(8-1).
FIG. 19 is a ¹H-NMR spectrum of a compound (compound B3)) obtained in Example 8(8-2).
FIG. 20 is a ¹H-NMR spectrum of a compound (compound B5)) obtained in Example 8(8-3).
FIG. 21 is a ¹H-NMR spectrum of a compound (compound B6)) obtained in Example 8(8-4).
FIG. 22 is a ¹H-NMR spectrum of a compound (compound B7)) obtained in Example 8(8-5).

### Description of Embodiments

The following definitions shall apply throughout the specification.

The term "linear alkyl group having 1 to 6 carbon atoms" as used herein refers to any linear alkyl group having 1 to 6 carbon atoms, and specifically to a methyl group, an ethyl group, an n-propyl group, an n-butyl group, an n-pentyl group, or an n-hexyl group. On the other hand, the term "alkyl group having 1 to 6 carbon atoms" refers to any linear, branched, or cyclic alkyl group having 1 to 6 carbon atoms.

The term "linear alkoxy group having 1 to 6 carbon atoms" as used herein encompasses alkoxy groups including any linear alkyl groups having 1 to 6 carbon atoms. Examples thereof include a methoxy group, an ethoxy group, and an n-propoxy group. On the other hand, the term "alkoxy group having 1 to 6 carbon atoms" refers to any linear, branched, or cyclic alkoxy group having 1 to 6 carbon atoms. The term "linear alkoxy group having 1 to 6 carbon atoms that may have undergone substitution by a linear alkoxy group having 1 to 6 carbon atoms" refers to the "linear alkoxy group having 1 to 6 carbon atoms" as well as an alkyl group obtained by substituting one or more hydrogen atoms included in the "linear alkoxy group having 1 to 6 carbon atoms" with another or other "linear alkoxy groups having 1 to 6 carbon atoms" that may be the same or different. Examples of such "linear alkoxy group having 1 to 6 carbon atoms that may have undergone substitution by a linear alkoxy group having 1 to 6 carbon atoms" include a methoxy group, an ethoxy group, an n-propoxy group, a methoxymethoxy group, an ethoxymethoxy group, an n-propoxymethoxy group, a methoxyethoxy group (e.g., a 2-methoxyethoxy group), an ethoxyethoxy group (e.g., a 2-ethoxyethoxy group), and an n-propoxyethoxy group.

The term "cyanoalkoxy group having 1 to 6 carbon atoms" as used herein refers to a group obtained by substituting at least one hydrogen atom included in any linear, branched, or cyclic alkoxy group having 1 to 6 carbon atoms with a cyano group.

The term "linear alkylthio group having 1 to 6 carbon atoms" as used herein encompasses alkylthio groups including any linear alkyl groups having 1 to 6 carbon atoms. Examples thereof include a methylthio group, an ethylthio group, and an n-propylthio group. On the other hand, the term "alkylthio group having 1 to 6 carbon atoms" refers to any linear, branched, or cyclic alkylthio group having 1 to 6 carbon atoms.

The term "linear alkylamino group having 1 to 6 carbon atoms" as used herein encompasses alkylamino groups including one or two alkylamino groups with any linear alkyl group having 1 to 6 carbon atoms. Examples thereof include a methylamino group, a dimethylamino group, an ethylamino group, a methylethylamino group, and a diethylamino group.

The term "alkyl group having 1 to 7 carbon atoms that may form a branch or a ring" as used herein encompasses any linear alkyl groups having 1 to 7 carbon atoms, any branched alkyl groups having 3 to 7 carbon atoms, and any cyclic alkyl groups having 3 to 7 carbon atoms. Such groups may also be simply referred to as "lower alkyl groups". Examples of any linear alkyl groups having 1 to 7 carbon atoms include a methyl group, an ethyl group, an n-propyl group, an n-butyl group, an n-pentyl group, an n-hexyl group, and an n-heptyl group; examples of any branched alkyl groups having 3 to 7 carbon atoms include an isopropyl group, an isobutyl group, a tert-butyl group, and an isopentyl group; and examples of any cyclic alkyl groups having 3 to 7 carbon atoms include a cyclopropylmethyl group, a cyclobutyl group, a cyclopentyl group, a cyclopentylmethyl group, and a cyclohexyl group.

The term "alkenyl group having 2 to 7 carbon atoms that may form a branch or a ring" as used herein encompasses any linear alkenyl groups having 2 to 7 carbon atoms, any branched alkenyl groups having 3 to 7 carbon atoms, and any cyclic alkenyl groups having 3 to 7 carbon atoms. Such groups may also be simply referred to as "lower alkenyl groups". Examples of any linear alkenyl groups having 2 to 7 carbon atoms include an ethenyl group, a 1-propenyl group, a 2-propenyl group, a 1-butenyl group, a 2-butenyl group, a 1-pentenyl group, a 2-pentenyl group, a 3-pentenyl group, a 4-pentenyl group, and a 1-hexenyl group; examples of any branched alkenyl groups having 3 to 7 carbon atoms include an isopropenyl group, a 1-methyl-1-propenyl group, a 1-methyl-2-propenyl group, a 2-methyl-1-propenyl group, a 2-methyl-2-propenyl group, and a 1-methyl-2-butenyl group; and examples of any cyclic alkenyl groups having 3 to 7 carbon atoms include a cyclobutenyl group, a cyclopentenyl group, and a cyclohexenyl group.

The term "aryl group having 6 to 12 carbon atoms" as used herein refer to any aryl groups that are constituted by only a hydrocarbon and have 6 to 12 carbon atoms, and examples thereof include a phenyl group, a naphthyl group, an indenyl group, and an azulenyl group.

The term "heteroaryl group having 1 to 12 carbon atoms" as used herein encompasses any heteroaryl groups having 1 to 12 carbon atoms obtained by substituting a heteroatom (e.g., a nitrogen atom, an oxygen atom, a sulfur atom, and a combination thereof) for at least one carbon atom included in the ring structure of any aryl group that is constituted by only a hydrocarbon and has 5 to 14 carbon atoms. Examples of the heteroaryl groups having 1 to 12 carbon atoms include a pyridyl group, a pyrrolyl group, a quinolyl group, an indolyl group, an imidazolyl group, a furyl group, and a thienyl group.

Examples of the term "aralkyl group with an aryl moiety that has 6 to 12 carbon atoms" as used herein include a benzyl group, a phenethyl group, a naphthylmethyl group, a 3-phenylpropyl group, a 2-phenylpropyl group, a 4-phenylbutyl group, and a 2-phenylbutyl group.

Examples of the term "heteroaralkyl group with a heteroaryl moiety that has 1 to 12 carbon atoms" as used herein include a pyridylmethyl group, an indolylmethyl group, a furylmethyl group, a thienylmethyl group, a pyrrolylmethyl group, a 2-pyridylethyl group, a 1-pyridylethyl group, and a 3-thienylpropyl group.

Examples of the term "acyl group" as used herein include aliphatic acyl groups and aromatic acyl groups. Specifically, examples of the aliphatic acyl groups include alkylcarbonyl groups such as a formyl group, an acetyl group, a propionyl group, a butyryl group, an isobutyryl group, a pentanoyl group, a pivaloyl group, a valeryl group, an isovaleryl group, an octanoyl group, a nonanoyl group, a decanoyl group, a 3-methylnonanoyl group, a 8-methylnonanoyl group, a 3-ethyloctanoyl group, a 3,7-dimethyloctanoyl group, an undecanoyl group, a dodecanoyl group, a tridecanoyl group, a tetradecanoyl group, a pentadecanoyl group, a hexadecanoyl group, a 1-methylpentadecanoyl group, a 14-methylpentadecanoyl group, a 13,13-dimethyltetradecanoyl group, a heptadecanoyl group, a 15-methylhexadecanoyl group, an octadecanoyl group, a 1-methylheptadecanoyl group, a nonadecanoyl group, an eicosanoyl group, and a heneicosanoyl group; carboxylated alkylcarbonyl groups such as a succinoyl group, a glutaroyl group, and an adipoyl group; halogeno lower-alkyl-carbonyl groups such as a chloroacetyl group, a dichloroacetyl group, a trichloroacetyl group, and a trifluoroacetyl group; lower-alkoxy-lower-alkyl-carbonyl groups such as a methoxyacetyl group; and unsaturated alkylcarbonyl groups such as an (E)-2-methyl-2-butenoyl group. Examples of the aromatic acyl groups include aryl carbonyl groups such as a benzoyl group, an α-naphthoyl group, and a β-naphthoyl group; halogeno aryl carbonyl groups such as a 2-bromobenzoyl group and a 4-chlorobenzoyl group; low-alkylated aryl carbonyl groups such as a 2,4,6-trimethylbenzoyl group and a 4-toluoyl group; low-alkoxylated aryl carbonyl groups such as a 4-anisoyl group; carboxylated aryl carbonyl groups such as a 2-carboxybenzoyl group, a 3-carboxybenzoyl group, and a 4-carboxybenzoyl group; nitrated aryl carbonyl groups such as a 4-nitrobenzoyl group and a 2-nitrobenzoyl group; low-alkoxycarbonylated aryl carbonyl groups such as a 2-(methoxycarbonyl)benzoyl group; and arylated aryl carbonyl groups such as a 4-phenylbenzoyl group.

Examples of the term "silyl group" as used herein include tri-lower-alkyl-silyl groups such as a trimethylsilyl group, a triethylsilyl group, an isopropyldimethylsilyl group, a t-butyldimethylsilyl group, a methyldiisopropylsilyl group, a methyldi-t-butylsilyl group, and a triisopropylsilyl group; silyl groups that have undergone substitution by one or two aryl groups, such as a diphenylmethylsilyl group (SiMePh₂ group), a butyldiphenylbutylsilyl group, a diphenylisopropylsilyl group, and a phenyldiisopropylsilyl group; and triarylsilyl groups that have undergone substitution by three aryl groups, such as a triphenylsilyl group. Examples of the term "sulfonyl group" include arylsulfonyl groups such as a phenylsulfonyl group (SO₂Ph group) and a 4-methylphenylsulfonyl group; and lower alkylsulfonyl groups such as a methylsulfonyl group and a trifluoromethanesulfonyl group.

Examples of the term "halogen atom" as used herein include a fluorine atom, a chlorine atom, a bromine atom, and an iodine atom.

"Protecting groups" in the terms "amino group protecting group for nucleic acid synthesis", "hydroxy group protecting group for nucleic acid synthesis", "hydroxy group protected by a protecting group for nucleic acid synthesis", "phosphate group protected by a protecting group for nucleic acid synthesis", and "mercapto group protected by a protecting group for nucleic acid synthesis" as used herein are not particularly limited as long as they can stably protect an amino group, a hydroxy group, a phosphate group, or a mercapto group during nucleic acid synthesis. Specifically, the protecting groups are stable during synthesis of oligonucleotides and can be cleaved using chemical techniques such as hydrogenolysis, hydrolysis, electrolysis, and photolysis. Examples of such protecting groups include lower alkyl groups, lower alkenyl groups, acyl groups, tetrahydropyranyl or tetrahydrothiopyranyl groups, tetrahydrofuranyl or tetrahydrothiofuranyl groups, silyl groups, lower-alkoxy-methyl groups, low-alkoxylated lower-alkoxy-methyl groups, halogeno lower-alkoxy-methyl groups, low-alkoxylated ethyl groups, halogenated ethyl groups, methyl groups that have undergone substitution by 1 to 3 aryl groups, "methyl groups that have undergone substitution by 1 to 3 aryl groups in which an aryl ring has undergone substitution by a lower alkyl group, lower alkoxy group, halogen atom, or cyano group", lower-alkoxy-carbonyl groups, "aryl groups that have undergone substitution by a halogen atom, lower alkoxy group, or nitro group", "lower-alkoxy-carbonyl groups that have undergone substitution by a halogen atom or tri-lower-alkyl-silyl group", alkenyloxycarbonyl groups, "aralkyloxycarbonyl groups in which an aryl ring may have undergone substitution by a lower alkoxy group or nitro group", a dimethylformamidyl group, and a diphenylcarbamoyl group.

More specific examples of the tetrahydropyranyl or tetrahydrothiopyranyl groups include a tetrahydropyran-2-yl group, a 3-bromotetrahydropyran-2-yl group, a 4-methoxytetrahydropyran-4-yl group, a tetrahydrothiopyran-4-yl group, and a 4-methoxytetrahydrothiopyran-4-yl group. Examples of the tetrahydrofuranyl or tetrahydrothiofuranyl groups include a tetrahydrofuran-2-yl group and a tetrahydrothiofuran-2-yl group. Examples of the lower-alkoxy-methyl groups include a methoxymethyl group, a 1,1-dimethyl-1-methoxymethyl group, an ethoxymethyl group, a propoxymethyl group, an isopropoxymethyl group, a butoxymethyl group, and a t-butoxymethyl group. An example of the low-alkoxylated lower-alkoxy-methyl groups is a 2-methoxyethoxymethyl group. Examples of the halogeno lower-alkoxy-methyl groups include a 2,2,2-trichloroethoxymethyl group and a bis(2-chloroethoxy)methyl group. Examples of the low-alkoxylated ethyl groups include a 1-ethoxyethyl group and a 1-(isopropoxy)ethyl group. An example of the halogenated ethyl groups is a 2,2,2-trichloroethyl group. Examples of the methyl groups that have undergone substitution by 1 to 3 aryl groups include a benzyl group, an α-naphthylmethyl group, a β-naphthylmethyl group, a diphenylmethyl group, a triphenylmethyl group, an α-naphthyldiphenylmethyl group, and a 9-anthrylmethyl group. Examples of the "methyl groups that have undergone substitution by 1 to 3 aryl groups in which an aryl ring has undergone substitution by a lower alkyl group, lower alkoxy group, halogen atom, or cyano group" include a 4-methylbenzyl group, a 2,4,6-trimethylbenzyl group, a 3,4,5-trimethylbenzyl group, a 4-methoxybenzyl group, a 4-methoxyphenyldiphenylmethyl group, a 4,4'-dimethoxytriphenylmethyl group, a 2-nitrobenzyl group, a 4-nitrobenzyl group, a 4-chlorobenzyl group, a 4-bromobenzyl group, and a 4-cyanobenzyl group. Examples of the lower-alkoxy-carbonyl groups include a methoxycarbonyl group, an ethoxycarbonyl group, and a t-butoxycarbonyl group. Examples of the aryl groups that have undergone substitution by a nitro group" include a 4-chlorophenyl group, a 2-fluorophenyl group, a 4-methoxyphenyl group, a 4-nitrophenyl group, and a 2,4-dinitrophenyl group. Examples of the "lower-alkoxy-carbonyl groups that have undergone substitution by a halogen atom or tri-lower-alkyl-silyl group" include a 2,2,2-trichloroethoxycarbonyl group and 2-trimethylsilylethoxycarbonyl group. Examples of the alkenyloxycarbonyl groups include a vinyloxycarbonyl group and an aryloxycarbonyl group. Examples of the "aralkyloxycarbonyl groups in which an aryl ring may have undergone substitution by a lower alkoxy group or nitro group" include a benzyloxycarbonyl group, a 4-methoxybenzyloxycarbonyl group, a 3,4-dimethoxybenzyloxycarbonyl group, a 2-nitrobenzyloxycarbonyl group, and a 4-nitrobenzyloxycarbonyl group.

In an embodiment, examples of the "hydroxy group protecting group for nucleic acid synthesis" include aliphatic acyl groups, aromatic acyl groups, methyl groups that have undergone substitution by 1 to 3 aryl groups, "methyl groups that have undergone substitution by 1 to 3 aryl groups in which an aryl ring has undergone substitution by a lower alkyl, lower alkoxy, halogen, or cyano group", and silyl groups. Alternatively, in an embodiment, examples of the "hydroxy group protecting group for nucleic acid synthesis" include an acetyl group, a benzoyl group, a benzyl group, a p-methoxybenzoyl group, a p-methoxybenzyl group, a trityl group, a 4,4'-dimethoxytrityl (DMTr) group, a 4-monomethoxytrityl group, a tert-butyldiphenylsilyl group, a trimethylsilyl group, a triethylsilyl group, an isopropyldimethylsilyl group, a methyldiisopropylsilyl group, a methyldi-t-butylsilyl group, a triisopropylsilyl group, a tert-butyldimethylsilyl (TBDMS) group, a [(triisopropylsilyl)oxy]methyl (TOM) group, a [(2-nitrobenzyl)oxy]methyl (NBOM) group, a bis(acetoxyethoxy)methyl ether (ACE) group, a tetrahydro-4-methoxy-2H-pyran-2-yl (Mthp) group, a 1-(2-cyanoethoxy)ethyl (CEE) group, a 2-cyanoethoxymethyl (CEM) group, a tert-butyldithiomethyl (DTM) group, a 2-(4-tolylsulfonyl)ethoxymethyl (TEM) group, and a 4-(N-dichloroacetyl-N-methylamino)benzyloxymethyl (4-MABOM) group.

In an embodiment, examples of the protecting group used for the "hydroxy group protected by a protecting group for nucleic acid synthesis" include aliphatic acyl groups, aromatic acyl groups, "methyl groups that have undergone substitution by 1 to 3 aryl groups", "aryl groups that have undergone substitution by a halogen atom, lower alkoxy group, or nitro group", lower alkyl groups, and lower alkenyl groups. Alternatively, in an embodiment, examples of the protecting group used for the "hydroxy group protected by a protecting group for nucleic acid synthesis" include a benzoyl group, a benzyl group, a 2-chlorophenyl group, a 4-chlorophenyl group, and a 2-propenyl group.

In an embodiment, examples of the "amino group protecting group for nucleic acid synthesis" include acyl groups (preferably a benzoyl group), a dimethylformamidyl group, and a diphenylcarbamoyl group.

In an embodiment, examples of the "amino group protected by a protecting group for nucleic acid synthesis" include an amino group protected by an acyl group, and an amino group protected by a benzoyl group is favorable.

In an embodiment, examples of the "protecting group" used for the "phosphate group protected by a protecting group for nucleic acid synthesis" include lower alkyl groups, lower alkyl groups that have undergone substitution by a cyano group, aralkyl groups, "aralkyl groups in which an aryl ring has undergone substitution by a nitro group or halogen atom", and "aryl groups that have undergone substitution by a lower alkyl group, halogen atom, or nitro group". Alternatively, in an embodiment, examples of the "protecting group" used for the "phosphate group protected by a protecting group for nucleic acid synthesis" include a 2-cyanoethyl group, a 2,2,2-trichloroethyl group, a benzyl group, a 2-chlorophenyl group, and a 4-chlorophenyl group.

In an embodiment, examples of the "protecting group" used for the "mercapto group protected by a protecting group for nucleic acid synthesis" include aliphatic acyl groups and aromatic acyl groups, and a benzoyl group is favorable.

In this specification, among groups represented by -P(R⁴)R⁵, where R⁴ and R⁵ each independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 6 carbon atoms, an alkylthio group having 1 to 6 carbon atoms, a cyanoalkoxy group having 1 to 6 carbon atoms, or a dialkylamino group that has an alkyl group having 1 to 6 carbon atoms, a group in which R⁴ is OR^{4a} and R⁵ is NR^{5a} is referred to as a "phosphoramidite group" (where an example of R^{4a} is a cyanoalkoxy group having 1 to 6 carbon atoms, and an example of R^{5a} is an alkyl group having 1 to 6 carbon atoms). Favorable examples of the phosphoramidite group include a group represented by a formula -P(OC₂H₄CN)(N(iPr)₂) and a group represented by a formula -P(OCH₃)(N(iPr)₂). In these formulas, iPr represents an isopropyl group.

The terms "nucleoside" and "nucleoside analogue" as used herein refer to non-naturally occurring nucleosides of "nucleosides" in which a purine base or a pyrimidine base binds to sugar, as well as those in which a heteroaromatic ring or an aromatic hydrocarbon ring other than purine and pyrimidine that can serve as a substitute for a purine or pyrimidine base binds to sugar.

The terms "artificial oligonucleotide" and "oligonucleotide analogue" as used herein refer to non-naturally occurring derivatives of "oligonucleotides" in which, for example, two to fifty of the same or different "nucleosides" or "nucleoside analogues" are bound via phosphodiester bonds. Favorable examples of such analogues include sugar derivatives with sugar moieties modified; thioated derivatives with phosphodiester moieties thioated; esters with terminal phosphate moieties esterificated; and amides in which amino groups on purine bases are amidated. A sugar derivative in which the sugar moiety is modified is more favorable.

The term "salt thereof" as used herein refers to a salt of a compound represented by the formula (I) according to the present invention. Examples of such salt include metal salts including alkali metal salts such as sodium salts, potassium salts, and lithium salts, alkali earth metal salts such as calcium salts and magnesium salts, and aluminum salts, iron salts, zinc salts, copper salts, nickel salts, and cobalt salts; amine salts including inorganic salts such as ammonium salts, and organic salts such as t-octylamine salts, dibenzylamine salts, morpholine salts, glucosamine salts, phenylglycine alkylester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts including hydrohalogenic acid salts such as hydrofluoric acid salts, hydrochloric acid salt, hydrobromic acid salts, and hydroiodic acid salts, nitrates, perchlorates, sulfates, and phosphates; organic acid salts including lower-alkane-sulfonates such as methanesulfonates, trifluoromethanesulfonates, and ethanesulfonates, aryl sulfonates such as benzenesulfonates and p-toluenesulfonates, acetates, malates, fumarates, succinates, citrates, tartrates, oxalates and maleates; and amino acid salts such as glycine salts, lysine salts, arginine salts, ornithine salts, glutamates, and aspartates.

The term "pharmacologically acceptable salt thereof" as used herein refers to a salt of an oligonucleotide analogue containing at least one nucleoside structure derived from the compound represented by the formula (I) according to the present invention. Examples of such salt include metal salts including alkali metal salts such as sodium salts, potassium salts, and lithium salts, alkali earth metal salts such as calcium salts and magnesium salts, and aluminum salts, iron salts, zinc salts, copper salts, nickel salts, and cobalt salts; amine salts including inorganic salts such as ammonium salts, and organic salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts including hydrohalogenic acid salts such as hydrofluoric acid salts, hydrochloric acid salt, hydrobromic acid salts, and hydroiodic acid salts, nitrates, perchlorates, sulfates, and phosphates; organic acid salts including lower-alkane-sulfonates such as methanesulfonates, trifluoromethanesulfonates, and ethanesulfonates, aryl sulfonates such as benzenesulfonates and p-toluenesulfonates, acetates, malates, fumarates, succinates, citrates, tartrates, oxalates and maleates; and amino acid salts such as glycine salts, lysine salts, arginine salts, ornithine salts, glutamates, and aspartates.

Hereinafter, the present invention will be described in detail.

### (Nucleosides and Salts thereof)

Acompound (nucleoside) of the present invention is a compound represented by a formula (I) below or a salt thereof.

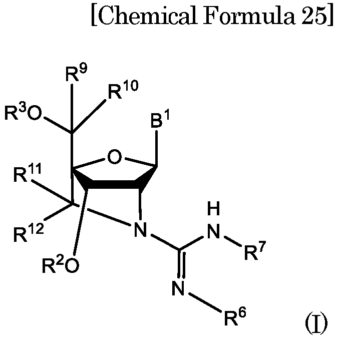

In the formula (I), B¹ is
(a) a purin-9-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms; or
(b) a 1,2-dihydropyrimidin-1-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms.

In the formula (I) above, specific examples of the "B¹" above include an adeninyl group, a guaninyl group, a cytosinyl group, an uracinyl group, a thyminyl group, a 6-aminopurin-9-yl group, a 2,6-diaminopurin-9-yl group, a 2-amino-6-chloropurin-9-yl group, a 2-amino-6-fluoropurin-9-yl group, a 2-amino-6-bromopurin-9-yl group, a 2-amino-6-hydroxypurin-9-yl group, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl group, a 4-amino-2-oxo-5-fluoro-1,2-dihydropyrimidin-1-yl group, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl group, a 2-oxo-4-hydroxy-5-methyl-1,2-dihydropyrimidin-1-yl group, and a 4-amino-5-methyl-2-oxo-1,2-dihydropyrimidin-1-yl group.

Alternatively, because the compound of the present invention can be effectively introduced as a nucleic acid drug, B¹ is preferably a group represented by each of the following structural formulas: 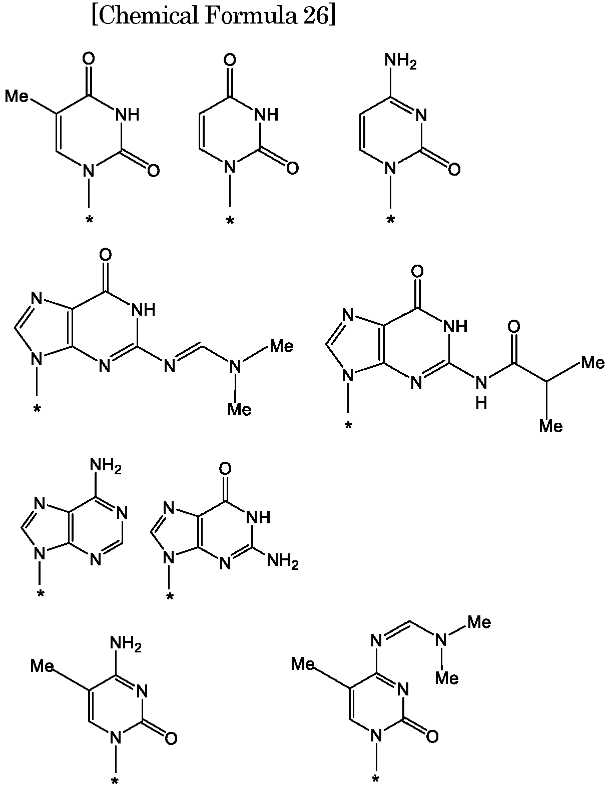 (where * is a bonding site) as well as a 2-oxo-4-hydroxy-5-methyl-1,2-dihydropyrimidin-1-yl group, a 2-oxo-4-amino-1,2-dihydropyrimidin-1-yl group, a 6-aminopurin-9-yl group, a 2-amino-6-hydroxypurin-9-yl group, a 4-amino-5-methyl-2-oxo-1,2-dihydropyrimidin-1-yl group, and a 2-oxo-4-hydroxy-1,2-dihydropyrimidin-1-yl group. It is preferable that a hydroxy group and an amino group included in the above-mentioned groups serving as B¹ are protected by a protecting group during oligonucleotide synthesis.

In the formula (I), R² and R³ are each independently
(a) a hydrogen atom;
(b) a hydroxy group protecting group for nucleic acid synthesis;
(c) an alkyl group having 1 to 7 carbon atoms that may form a branch or a ring;
(d) an alkenyl group having 2 to 7 carbon atoms that may form a branch or a ring;
(e) an aryl group having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(f) a heteroaryl group having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(g) an aralkyl group with an aryl moiety having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(h) a heteroaralkyl group with a heteroaryl moiety having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(i) an acyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(j) a silyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(k) a phosphate group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(l) a phosphate group protected by a protecting group for nucleic acid synthesis; or
(m) -P(R⁴)R⁵,
   where
   R⁴ and R⁵ are each independently
      (a) a hydroxy group;
      (b) a hydroxy group protected by a protecting group for nucleic acid synthesis;
      (c) a mercapto group;
      (d) a mercapto group protected by a protecting group for nucleic acid synthesis;
      (e) an amino group;
      (f) an alkoxy group having 1 to 5 carbon atoms;
      (g) an alkylthio group having 1 to 5 carbon atoms;
      (h) a cyanoalkoxy group having 1 to 6 carbon atoms;
      (i) a dialkylamino group having alkyl groups that each have 1 to 6 carbon atoms and may be the same or different; or
      (j) a cyclic alkylamino group having 3 to 6 carbon atoms; or
   R⁴ and R⁵ together constitute the following formula:

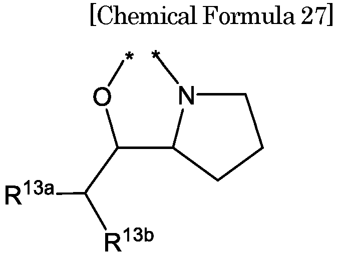

(where R^{13a} and R^{13b} are each independently a hydrogen atom, an alkyl group having 1 to 6 carbon atoms that may be branched, an aryl group having 6 to 12 carbon atoms, a nitro group, a halogen atom, a cyano group, Si(R^{a})₃ (where R^{a} is an alkyl group having 1 to 6 carbon atoms or an aryl group having 6 to 12 carbon atoms), a sulfonyl group, SO₂Ph, or SiMePh₂, where Ph represents a phenyl group, and * is a bonding site); or 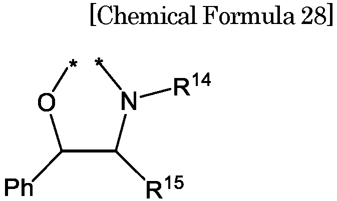
(where R¹⁴ and R¹⁵ are each independently a hydrogen atom, an alkyl group having 1 to 3 carbon atoms that may be branched, or a phenyl group, and * is a bonding site).

In an embodiment, in the formula (I), either R² or R³ is preferably
(m)-P(R⁴)R⁵,
where
R⁴ and R⁵ each independently represent
   (a) a hydroxy group;
   (b) a hydroxy group protected by a protecting group for nucleic acid synthesis;
   (c) a mercapto group;
   (d) a mercapto group protected by a protecting group for nucleic acid synthesis;
   (e) an amino group;
   (f) an alkoxy group having 1 to 5 carbon atoms;
   (g) an alkylthio group having 1 to 5 carbon atoms;
   (h) a cyanoalkoxy group having 1 to 6 carbon atoms;
   (i) a dialkylamino group having alkyl groups that have 1 to 6 carbon atoms and may be the same or different; or
   (j) a cyclic alkylamino group having 3 to 6 carbon atoms. A specific example of the "-P(R⁴)R⁵" is a phosphoramidite group, and specific examples thereof include the following groups: 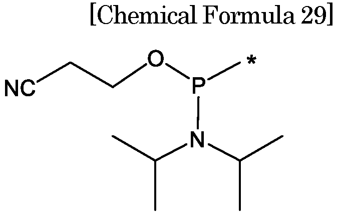
(where * is a bonding site).

In the formula (I), R⁶ and R⁷ are constituted by either the following "combination A" or "combination B".

First, as the "combination A", in an embodiment, in the formula (I), R⁶ is a group represented by the following formula: 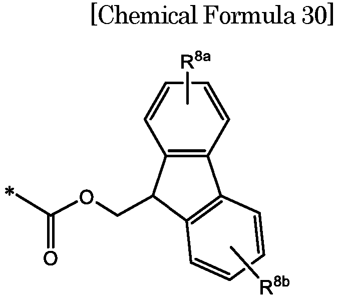 (where
R^{8a} and R^{8b} are each independently
   (a) an alkyl group having 1 to 9 carbon atoms that may form a branch or a ring;
   (b) an alkoxy group having 1 to 8 carbon atoms that may form a branch or a ring;
   (c) an alkylamino group having 1 to 8 carbon atoms that may form a branch or a ring; or
   (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site); and
R⁷ is
   (a) an alkyl group having 1 to 8 carbon atoms that may form a branch or a ring and may have undergone substitution by an alkoxy group having 1 to 8 carbon atoms that may form a branch or a ring;
   (b) a cyanoalkoxy group having 1 to 8 carbon atoms;
   (c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
   (d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
   (e) a group represented by the following formula: 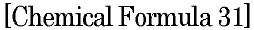 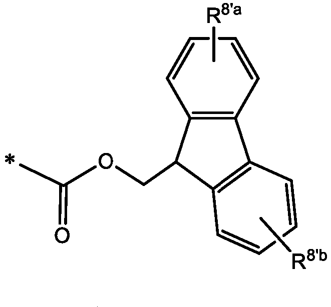 (where
      R^{8'a} and R^{8'b} are each independently
         (a) an alkyl group having 1 to 9 carbon atoms that may be branched;
         (b) an alkoxy group having 1 to 8 carbon atoms that may form a branch or a ring;
         (c) an alkylamino group having 1 to 8 carbon atoms that may form a branch or a ring; or
         (d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
         * is a bonding site):
In the "combination A", R⁷ is preferably an alkyl group having 1 to 8 carbon atoms that may form a branch or a ring. In the "combination A", both R^{8a} and R^{8b} are each preferably an alkyl group having 3 to 8 carbon atoms that may form a branch or a ring, and more preferably a t-butyl group.

Alternatively, as the "combination B", in an embodiment, R⁶ is:
(a) an alkyl group having 1 to 8 carbon atoms that may form a branch or a ring and may have undergone substitution by an alkoxy group having 1 to 8 carbon atoms that may form a branch or a ring;
(b) a cyanoalkoxy group having 1 to 8 carbon atoms;
(c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
(d) a heteroaralkyl group with a heteroaryl moiety having 3 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
(e) a group represented by the following formula: 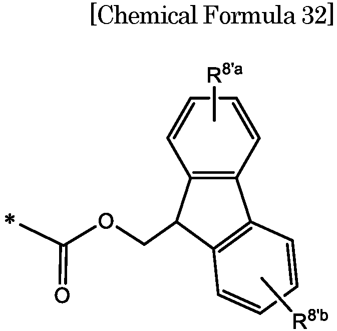 (where
   R^{8'a} and R^{8b} are each independently
      (a) an alkyl group having 1 to 9 carbon atoms that may be branched;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, linear alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
   * is a bonding site); and
   R⁷ is a group represented by the following formula: 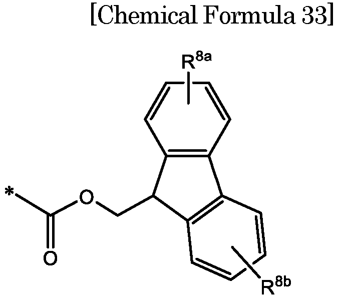 (where
      R^{8a} and R^{8b} are each independently
         (a) an alkyl group having 1 to 9 carbon atoms that may be branched;
         (b) an alkoxy group having 1 to 8 carbon atoms that may form a branch or a ring;
         (c) an alkylamino group having 1 to 8 carbon atoms that may form a branch or a ring; or
         (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy group having 1 to 8 carbon atoms that may form a branch or a ring, alkylamino groups having 1 to 8 carbon atoms that may form a branch or a ring, and an amino group protected by a protecting group for nucleic acid synthesis; and
      * is a bonding site).

In the present invention, it is preferable that R⁶ and R⁷ have a group constituted by the "combination A" because it has a more stable chemical structure.

In the formula (I), R⁹ and R¹⁰ are each independently a hydrogen atom or an alkyl group having 1 to 6 carbon atoms that may form a branch or a ring, or R⁹ and R¹⁰ together constitute -(CH₂)ₘ- (where m is an integer from 2 to 7).

In the formula (I), R¹¹ and R¹² are each independently a hydrogen atom or an alkyl group having 1 to 6 carbon atoms that may form a branch or a ring. Alternatively, R¹¹ and R¹² together may constitute the oxygen atom part (=O) of a carbonyl group (-(CO)-). Alternatively, R¹¹ and R¹² together may constitute -(CH₂)ₙ- (where n is an integer from 2 to 7).

In an embodiment, the compound represented by the formula (I) according to the present invention or a salt thereof is preferably a compound represented by a formula (I') below or a salt thereof because the guanidine structure of the bridged part can be more stably maintained.

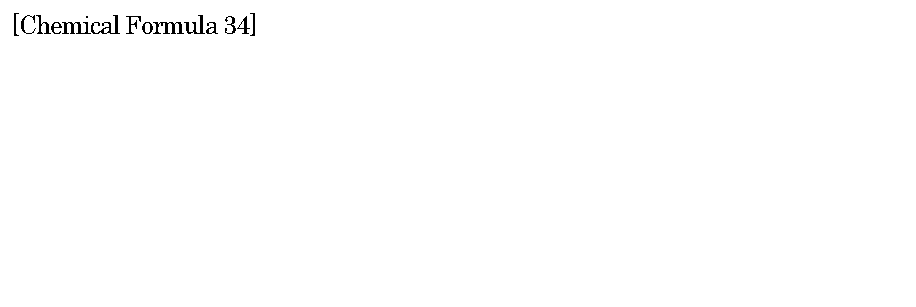

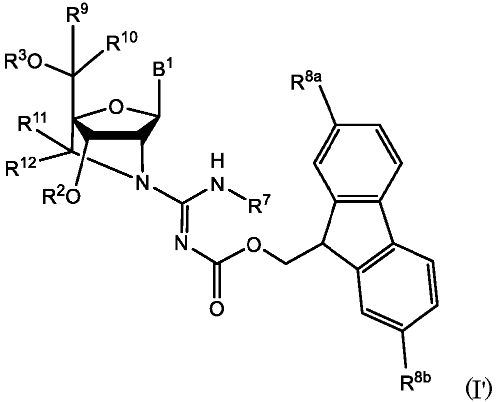

(In the formula (I'), B¹, R², R³, R⁷, R^{8a}, R^{8b}, R⁹, R¹⁰, R¹¹, and R¹² are each independently as defined above).

One of the structural features of the compound or salt thereof according to the present invention is that a guanidino structure is introduced into the bridged part of 2',4'-BNA/LNA. Because guanidine has positive electric charge, for example, the compound and salt thereof according to the present invention are expected that the ability to form a duplex with the target nucleic acid is improved due to an enhancement of the anionic repulsion suppression (electrostatic interaction) of the phosphodiester moiety and the hydration effect and that the enzyme resistance is improved.

The compound or salt thereof according to the present invention contains a protecting group having a predetermined substituent (9-fluorenylmethyloxycarbonyl group; Fmoc group) in R⁶ or R⁷ of the formula (I). Because the protecting group having such a predetermined substituent is contained, the resulting compound and a salt thereof can be maintained in a stable state for a long period of time, as compared with, for example, a compound or a salt thereof having an unsubstituted Fmoc group (9-fluorenylmethyloxycarbonyl group). Such a protecting group (Fmoc group) having such a predetermined substituent is not particularly limited, and an example thereof is a Fmoc that have undergone substitution by two t-butyl groups (di-tBu-Fmoc).

Note that a 2',4'-bridged artificial nucleotide can be easily synthesized using the compound represented by the formula (I) or salt thereof according to the present invention. For example, triphosphorylation of 2',4'-bridged artificial nucleotides can be easily carried out by those skilled in the art according to the method described in Non-Patent Document 5.

### (Guanidinylation Reagent)

The compound represented by the formula (I) and the salt thereof can be produced, for example, through a reaction of a compound having a predetermined nucleoside structure with a guanidinylation reagent, as exemplified in the Examples described later.

Examples of the guanidinylation reagent that may be used in such a reaction include compounds represented by a formula (II) below or salts thereof, for example. 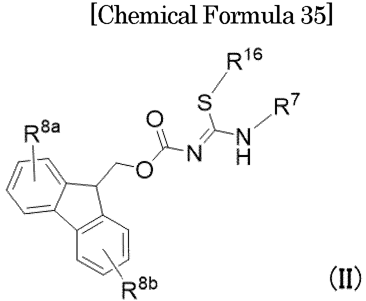 (where in the formula (II),
R⁷ is
(a) an alkyl group having 1 to 8 carbon atoms that may form a branch or a ring and may have undergone substitution by an alkoxy group having 1 to 8 carbon atoms that may form a branch or a ring;
(b) a cyanoalkoxy group having 1 to 8 carbon atoms;
(c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
(d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
(e) a group represented by the following formula: 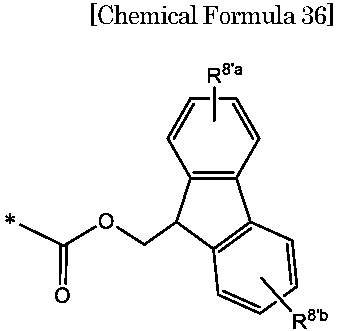 (where
   R^{8'a} and R^{8b} are each independently
      (a) an alkyl group having 1 to 9 carbon atoms that may be branched;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
   * is a bonding site); and
   R^{8a} and R^{8b} are each independently
      (a) an alkyl group having 1 to 9 carbon atoms that may form a branch or a ring;
      (b) an alkoxy group having 1 to 8 carbon atoms that may form a branch or a ring;
      (c) an alkylamino group having 1 to 8 carbon atoms that may form a branch or a ring; or
      (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
   R¹⁶ is a linear or branched alkyl group having 1 to 6 carbon atoms or a linear or branched alkenyl group having 2 to 6 carbon atoms).

In the present invention, the compound represented by the formula (II) above encompasses all of tautomers and geometric isomers thereof.

Specific examples of the compound represented by the formula (II) include compounds represented by a formula (II') below: 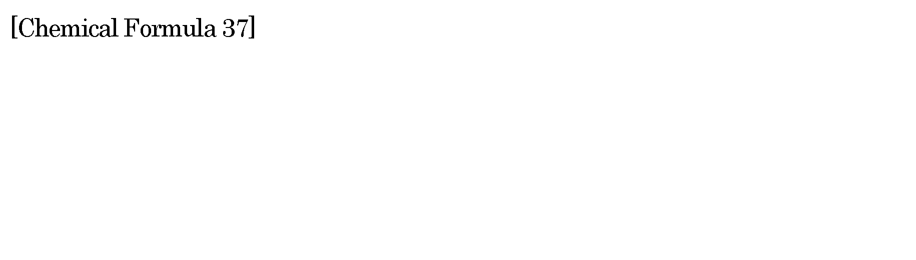 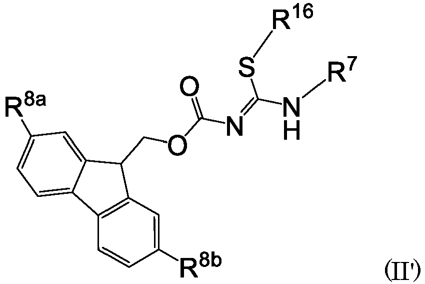 (where in the formula (II'), R⁷, R⁸a, R^{8b}, and R¹⁶ are as defined for the formula (II) above).

In the formulas (I), (I'), (II), and (II'), R^{8a} and R^{8b} are each preferably an alkyl group having 1 to 9 carbon atoms that may form a branch or a ring, more preferably a branched alkyl group having 1 to 6 carbon atoms, even more preferably a tertiary alkyl group having 4 to 6 carbon atoms, and particularly preferably a tert-butyl group.

In the formulas (I), (I'), (II), and (II'), R⁷ is an alkyl group having 1 to 8 carbon atoms that may form a branch or a ring and may have undergone substitution by an alkoxy group having 1 to 8 carbon atoms that may form a branch or a ring, more preferably an alkyl group having 1 to 8 carbon atoms that may form a branch or a ring, even more preferably an alkyl group having 1 to 8 carbon atoms that may be branched, and particularly preferably a tert-butyl group.

R¹⁶ is preferably a linear or branched alkyl group having 1 to 6 carbon atoms, more preferably a methyl group, an ethyl group, an n-propyl group, or an n-butyl group, and particularly preferably a methyl group.

In the present invention, the compound represented by the formula (II) above that can be used as a guanidinylation reagent can be produced, for example, through a reaction of a compound represented by a formula (III) below with a compound represented by a formula (IV) below, and a reaction of the reaction product with a compound represented by a formula (V) below. 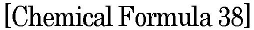 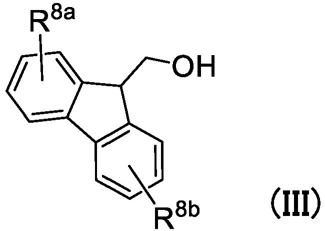
(where in the formula (III), R^{8a} and R^{8b} are each independently as defined for the formula (II) above), 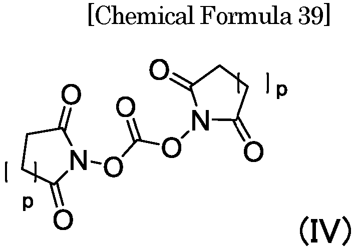
(where in the formula (IV), p is independently an integer from 0 to 3), 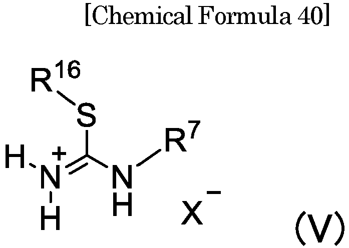
(where in the formula (V), R⁷ and R¹⁶ are each as defined for the formula (II) above, and X⁻ is a counter anion).

In the formula (IV), p is preferably independently 1 or 2, and particularly preferably 1. Specific examples of X include halogen atoms, a p-toluenesulfonyloxy group, a methanesulfonyloxy group, and a trifluoromethanesulfonyloxy group. X is preferably a halogen atom, and particularly preferably an iodine atom.

The reaction product of the compound represented by the formula (III) and the compound represented by the formula (IV) is represented by the following formula. For example, the reaction product can be obtained through a reaction of the compound represented by the formula (III) with the compound represented by the formula (IV) in an appropriate solvent in the presence of a base.

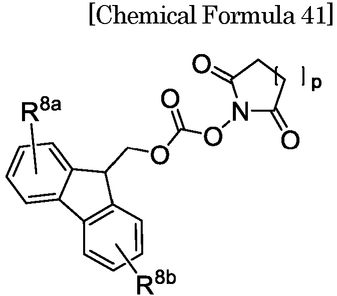

There is no particular limitation on the solvent as long as reactions favorably proceed, and specific examples thereof include halogenated hydrocarbon solvents (such as dichloromethane (DCM) and chloroform), lower aliphatic acid ester solvents (such as ethyl acetate), nitrile solvents (such as acetonitrile), and combinations thereof. The solvent is preferably an organic solvent, more preferably a halogenated hydrocarbon solvent, and particularly preferably dichloromethane. Examples of the base include pyridine, N-methylmorpholine, and combinations thereof, and the base is preferably pyridine. The amount of the base is, for example, 1 to 5 equivalents, and preferably 1 to 2 equivalents, with respect to 1 mole of the compound represented by the formula (III).

The amount of the compound represented by the formula (IV) used is, for example, 1 to 5 equivalents, and preferably 1 to 2 equivalents, with respect to 1 mole of the compound represented by the formula (III).

The reaction temperature is, for example, from -78°C to a solvent reflux temperature, preferably from 0°C to 50°C, and particularly preferably from 10°C to 40°C.

The reaction time is preferably 10 minutes to 48 hours, more preferably 20 minutes to 10 hours, and even more preferably 30 minutes to 5 hours.

As for the reaction conditions of the reaction between the reaction product and the compound represented by the formula (V), the solvent is not particularly limited as long as the reaction favorably proceeds. Specific examples of the solvent include water and solvents that are similar to those used in the step of allowing the compound represented by the formula (III) and the compound represented by the formula (IV) to react with each other. The solvent is preferably a mixed solvent of a halogenated hydrocarbon solvent and water, and particularly preferably a mixed solvent of dichloromethane and water. A mixed solvent of a halogenated hydrocarbon solvent and water is a bilayer solvent.

Examples of the base include alkali metal carbonates (such as sodium carbonate and potassium carbonate) and alkali metal hydrogen carbonates (such as sodium hydrogen carbonate and potassium hydrogen carbonate). The base is preferably an alkali metal carbonate, and particularly preferably potassium carbonate. The amount of the base is, for example, 1 to 5 equivalents (preferably 1 to 2 equivalents) in the case of an alkali metal carbonate, and 2 to 10 equivalents (preferably 2 to 4 equivalents) in the case of an alkali metal hydrogen carbonate, with respect to 1 mole of the compound represented by the formula (III).

The reaction temperature is, for example, from 0°C to a solvent reflux temperature, preferably from 0°C to 50°C, and particularly preferably from 10°C to 40°C.

The reaction time is preferably 10 minutes to 48 hours, more preferably 20 minutes to 10 hours, and even more preferably 30 minutes to 5 hours.

The compound represented by the formula (II) can be purified through addition of an aliphatic hydrocarbon solvent such as heptane, heating, cooling, and then filtering. The heating temperature is, for example, from 40°C to a solvent reflux temperature, and preferably from 40°C to 60°C. The cooling temperature is, for example, from -20°C to 30°C, and is preferably -10°C to 20°C.

Note that the compound represented by the formula (III) above and the compound represented by the formula (V) can be synthesized, for example, as follows.

Regarding a procedure for synthesizing the compound represented by the formula (III), a fluorene compound represented by a formula (A-5) is allowed to react with a formic acid ester (ethyl formate, methyl formate, or the like) in a solvent in the presence of a base to obtain a compound represented by a formula (A-6). Then, the compound represented by the formula (A-6) is allowed to react with a reducing agent such as sodium borohydride or sodium triacetoxy borohydride to obtain the compound represented by the formula (III) above: 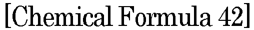 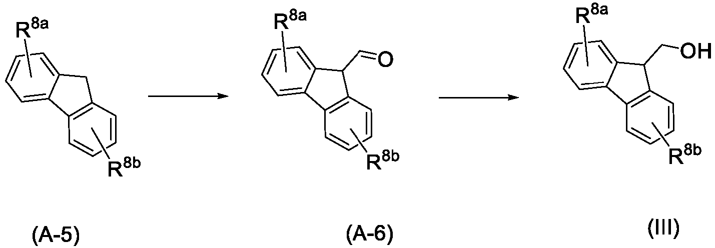 (where in the formulas (A-5) and (A-6), R^{8a} and R^{8b} are each independently as defined for the formula (II) above).

As for the reaction conditions under which the compound represented by the formula (A-5) is converted to the compound represented by the formula (A-6), the solvent is not particularly limited as long as the reaction favorably proceeds, and is preferably an organic solvent. Examples thereof include ether solvents (such as tetrahydrofuran and diethyl ether), and the solvent is particularly preferably tetrahydrofuran.

The base is not particularly limited as long as the reaction proceeds. Examples thereof include metal alkoxides (such as potassium t-butoxide, sodium t-butoxide, sodium ethoxide, and sodium methoxide), and the base is particularly preferably potassium t-butoxide. The amount of the base is, for example, 1 to 5 equivalents, and preferably 1 to 3 equivalents, with respect to 1 mole of the compound represented by the formula (A-5).

The reaction temperature is, for example, from -78°C to a solvent reflux temperature, preferably from -20°C to 50°C, more preferably -10°C to 30°C, and particularly preferably from -10°C to 10°C.

The reaction time is preferably 10 minutes to 48 hours, more preferably 10 minutes to 10 hours, and even more preferably 20 minutes to 2 hours.

As for the reaction conditions under which the compound represented by the formula (A-6) is converted to the compound represented by the formula (III), the solvent is not particularly limited as long as the reaction favorably proceeds. Examples of the solvent include water, alcohols (such as methanol, ethanol, and isopropyl alcohol), ether solvents (such as tetrahydrofuran and diethyl ether), and combinations thereof. The solvent is particularly preferably water or isopropyl alcohol, or a combination thereof.

It is preferable to use a base. The base is not particularly limited as long as the reaction proceeds, and examples thereof include metal hydroxides (such as potassium hydroxide and sodium hydroxide), and the base is particularly preferably sodium hydroxide. The amount of the base is, for example, 1 to 5 equivalents, and preferably 1 to 3 equivalents, with respect to 1 mole of the compound represented by the formula (A-6).

The reaction temperature is, for example, from -78°C to a solvent reflux temperature, preferably from 0°C to 50°C, and particularly preferably from 20°C to 40°C.

The reaction time is preferably 10 minutes to 48 hours, more preferably 20 minutes to 10 hours, and even more preferably 30 minutes to 5 hours.

After the reaction, a process for adding camphorsulfonic acid in an aromatic hydrocarbon solvent such as toluene and stirring the resulting mixture may be performed.

The compound represented by the formula (III) can be purified by adding an aliphatic hydrocarbon solvent such as heptane and then heating, cooling, and filtering the resulting mixture. The heating temperature is, for example, from 40°C to a solvent reflux temperature, preferably from 40°C to 70°C, and more preferably from 50°C to 70°C. The cooling temperature is, for example, from -20°C to 30°C, and preferably -10°C to 20°C.

On the other hand, regarding a procedure for synthesizing the compound represented by the formula (V), first, an amine represented by a formula (A-1) is allowed to react with benzoyl isothiocyanate in an appropriate solvent (e.g., acetonitrile) to produce a compound represented by the following formula (A-2). 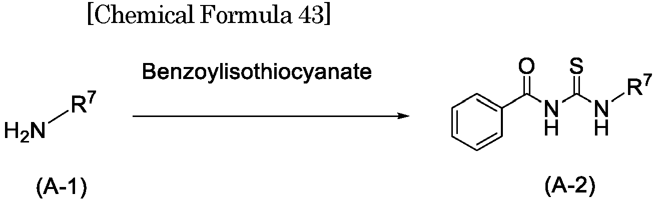 (where in the formulas (A-1) and (A-2), R⁷ is as defined for the formula (II) above).

The solvent is not particularly limited as long as the reaction favorably proceeds, and is preferably an organic solvent. Examples thereof include nitrile solvents (such as acetonitrile), and the solvent is particularly preferably acetonitrile.

The amount of benzoyl isothiocyanate is, for example, 1 to 5 equivalents, and preferably 1 to 3 equivalents, with respect to 1 mole of the compound represented by the formula (A-1).

The reaction temperature is, for example, from -78°C to a solvent reflux temperature, preferably from -20°C to 50°C, more preferably -10°C to 30°C, and particularly preferably from -10°C to 10°C.

The reaction time is preferably 10 minutes to 48 hours, more preferably 10 minutes to 10 hours, and even more preferably 30 minutes to 2 hours.

The compound represented by the formula (A-2) can be purified by washing with a nitrile solvent such as acetonitrile. Here, it is preferable to use cooled acetonitrile. The cooling temperature is, for example, from -20°C to 10°C, and preferably -10°C to 10°C.

The compound represented by the formula (A-2) is allowed to react with a nucleophile in a solvent to remove the benzoyl group, then react with an alkyl group-introducing reagent to obtain the compound represented by the formula (V) above via a compound represented by a formula (A-3). 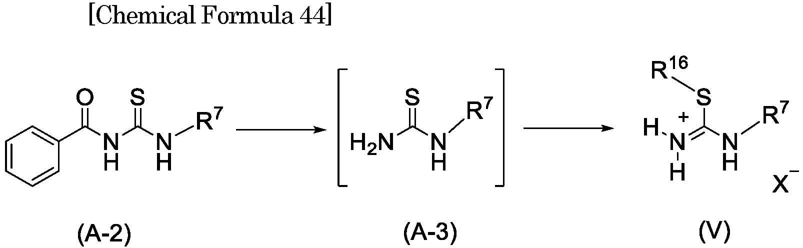 (where in the formulas (A-2), (A-3), and (V), R⁷ is as defined for the formula (II) above, and in the formula (V), R¹⁶ is as defined for the formula (II) above).

As for the reaction conditions under which the compound represented by the formula (A-2) is converted to the compound represented by the formula (A-3), the solvent is not particularly limited as long as the reactions favorably proceed, and is preferably an organic solvent. Examples thereof include alcohol solvents (such as methanol, ethanol, and n-propanol), and the solvent is particularly preferably methanol.

The nucleophile is not particularly limited as long as the reaction proceeds. Examples thereof include metal alkoxides (such as sodium ethoxide and sodium methoxide), and the nucleophile is particularly preferably sodium methoxide. The amount of the base is, for example, 0.001 to 1 equivalent, and preferably 0.01 to 0.1 equivalents, with respect to 1 mole of the compound represented by the formula (A-2). The reaction temperature is, for example, from -78°C to a solvent reflux temperature, preferably from 0°C to the solvent reflux temperature, and more preferably from 40°C to 70°C.

The reaction time is preferably 10 minutes to 48 hours, more preferably 10 minutes to 10 hours, and even more preferably 20 minutes to 3 hours.

As for the reaction conditions under which the compound represented by the formula (A-3) is converted to the compound represented by the formula (V), the solvent is not particularly limited as long as the reaction favorably proceeds, and is preferably an organic solvent. Examples thereof include alcohol solvents (such as methanol, ethanol, and n-propanol), and the solvent is more preferably the solvent that is the same as that used in the step of producing the compound represented by the formula (A-3) from the compound represented by the formula (A-2).

Examples of the alkyl group-introducing reagent include alkyl halides (such as methyl iodide, methyl bromide, methyl chloride, ethyl iodide, ethyl bromide, and ethyl chloride), sulfonates (such as methyl methanesulfonate, methyl p-toluenesulfonate, and methyl trifluoromethanesulfonate), and sulfate diesters (such as dimethyl sulfate and diethyl sulfate), and the alkyl group-introducing reagent is particularly preferably methyl iodide. The amount of the alkyl group-introducing reagent is, for example, 1 to 5 equivalents, and preferably 1 to 3 equivalents, with respect to 1 mole of the compound represented by the formula (A-3).

The reaction temperature is, for example, from -78°C to a solvent reflux temperature, preferably from 0°C to the solvent reflux temperature, and more preferably from 20°C to 40°C.

The reaction time is preferably 10 minutes to 48 hours, more preferably 10 minutes to 10 hours, and even more preferably 20 minutes to 3 hours.

The compounds represented by the formulas (III), (V), (A-2), and (A-6) can all be used in the next step without column purification. Also, the compound represented by the above formula (II) can be produced without using reagents that require careful handling (such as p-nitrophenyl chloroformate, n-butyllithium, and an ammonia-methanol solution). The method for producing the above-described compound represented by the formula (II) is applicable to an industrial production method.

### (Guanidinylation Step Using Guanidinylation Reagent)

The compound represented by the formula (II) or the salt thereof produced as above can be used as a guanidinylation reagent to react with a compound represented by a formula (VI) below, and thereby the compound represented by the formula (I) or the salt thereof can be produced through the following reaction step (guanidinylation step), for example. Note that the term "guanidinylation" as used herein can be expressed as "guanidylation" or "guanidination", which have the same meaning.

Specifically, the compound represented by the formula (VI) below or a salt thereof:

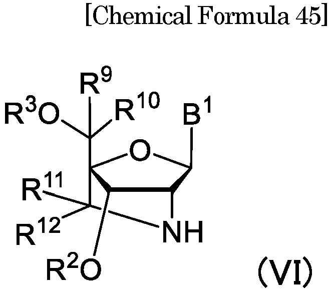

is guanidinylated using the compound represented by the formula (II) or the salt thereof: 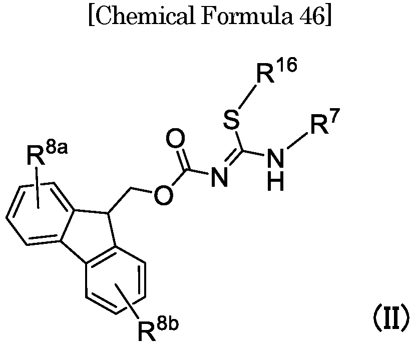 (where in the formula (VI),
B¹ is
   (a) a purin-9-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms; or
   (b) a 1,2-dihydropyrimidin-1-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms.
R² and R³ are each
   (a) a hydrogen atom;
   (b) a hydroxy group protecting group for nucleic acid synthesis;
   (c) an alkyl group having 1 to 7 carbon atoms that may form a branch or a ring;
   (d) an alkenyl group having 2 to 7 carbon atoms that may form a branch or a ring;
   (e) an aryl group having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
   (f) a heteroaryl group having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
   (g) an aralkyl group with an aryl moiety having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
   (h) a heteroaralkyl group with a heteroaryl moiety having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
   (i) an acyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
   (j) a silyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
   (k) a phosphate group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms; or
   (l) a phosphate group protected by a protecting group for nucleic acid synthesis;
R⁹ and R¹⁰ are each independently a hydrogen atom or an alkyl group having 1 to 6 carbon atoms that may form a branch or a ring, or R⁹ and R¹⁰ together constitute - (CH₂)ₘ- (where m is an integer from 2 to 7), and
R¹¹ and R¹² are each independently a hydrogen atom or an alkyl group having 1 to 6 carbon atoms that may form a branch or a ring, or
R¹¹ and R¹² together constitute the oxygen atom part (=O) of a carbonyl group, or
R¹¹ and R¹² together constitute -(CH₂)ₙ- (where n is an integer from 2 to 7), and
in the formula (II),
R⁷ is
   (a) an alkyl group having 1 to 8 carbon atoms that may form a branch or a ring and may have undergone substitution by an alkoxy group having 1 to 8 carbon atoms that may form a branch or a ring;
   (b) a cyanoalkoxy group having 1 to 8 carbon atoms;
   (c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
   (d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
   (e) a group represented by the following formula: 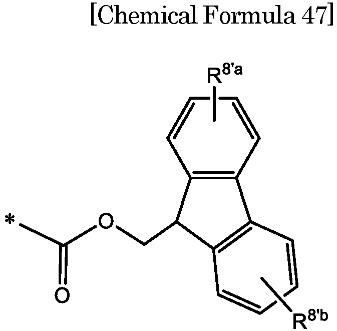 (where
      R^{8'a} and R^{8b} are each independently
         (a) an alkyl group having 1 to 9 carbon atoms that may be branched;
         (b) an alkoxy group having 1 to 8 carbon atoms that may form a branch or a ring;
         (c) an alkylamino group having 1 to 8 carbon atoms that may form a branch or a ring; or
         (d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
         * is a bonding site); and
      R^{8a} and R^{8b} are each independently
         (a) an alkyl group having 1 to 9 carbon atoms that may form a branch or a ring;
         (b) an alkoxy group having 1 to 8 carbon atoms that may form a branch or a ring;
         (c) an alkylamino group having 1 to 8 carbon atoms that may form a branch or a ring; or
         (d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
      R¹⁶ is a linear or branched alkyl group having 1 to 6 carbon atoms or a linear or branched alkenyl group having 2 to 6 carbon atoms).

The solvent that can be used in the guanidinylation step is not particularly limited as long as the reaction favorably proceeds, and is preferably an organic solvent. Examples thereof include ether solvents (such as diethyl ether, diisopropyl ether, tetrahydrofuran, and 4-methyltetrahydropyran), and the solvent is particularly preferably tetrahydrofuran.

The reaction is preferably carried out using a metal salt. The metal salt is not particularly limited as long as the reaction favorably proceeds, and is preferably a thiophilic metal salt. Examples thereof include silver salts (such as silver chloride, silver bromide, silver nitrate, silver iodide, silver trifluoromethanesulfonate, silver tetrafluoroborate, and silver hexafluorophosphate), copper (I) salts (such as copper (I) chloride, copper (I) bromide, copper (I) iodide, copper (I) trifluoromethanesulfonate, copper (I) tetrafluoroborate, and copper (I) hexafluorophosphate), and mercury (II) salts (such as mercury (II) chloride). The metal salt is preferably a silver salt or copper (I) salt, more preferably a copper (I) salt, and particularly preferably copper (I) chloride. Copper (I) salts are inexpensive and safe. The amount of the metal salt is, for example, 1 to 5 equivalents, and preferably 1 to 2 equivalents, with respect to 1 mole of the compound represented by the formula (II).

The term "thiophilic metal" as used herein refers to a soft metal to which a soft sulfide can bind. The term "thiophilic" is based on the HSAB principle. The "HSAB principle" is an acronym for hard and soft (Lewis) acids and bases. According to the HSAB principle, soft metal ions prefer soft sulfides as counter ions.

The reaction is preferably carried out in a nitrogen atmosphere. Furthermore, when a copper (I) salt is used, it is preferable to start the reaction under a nitrogen atmosphere and then add an oxygen-containing gas (e.g., air) during the reaction and stir the mixture. Copper ions are oxidized by oxygen from a monovalent state to a divalent state, increasing the reaction rate.

The reaction is preferably carried out in the presence of a base. The base is not particularly limited as long as the reaction favorably proceeds, and examples thereof include trialkylamines (such as triethylamine) and pyridine. The base is particularly preferably pyridine. The amount of the metal salt is, for example, 1 to 20 equivalents, and preferably 2 to 10 equivalents, with respect to 1 mole of the compound represented by the formula (II).

The reaction temperature is, for example, from -78°C to a solvent reflux temperature, preferably from 0°C to 70°C, more preferably 30°C to 70°C, and particularly preferably from 50°C to 70°C.

The reaction time is preferably 5 minutes to 48 hours, more preferably 10 minutes to 10 hours, even more preferably 30 minutes to 5 hours, and particularly preferably 1 to 2 hours.

The guanidinylation step may be carried out on the compound represented by the formula (I), where R² is a hydroxy group protecting group for nucleic acid synthesis (e.g., a silyl group such as a trimethylsilyl group, a triethylsilyl group (TMS group), or a t-butyldimethylsilyl group), and the protecting group may be removed with a fluorine reagent (e.g., tetrabutylammonium fluoride) or the like to produce the compound represented by the formula (I), where R² is a hydrogen atom.

As a post-treatment of the guanidinylation step, the solution containing the product compound represented by the formula (I) may be treated with a solution containing ethylenediaminetetraacetic acid (EDTA) and an aqueous solution of a metal hydroxide (such as sodium hydroxide or potassium hydroxide). This post-treatment is effective in removing metal salts. The amount of ethylenediaminetetraacetic acid is 1 to 5 equivalents, and preferably 1 to 2 equivalents, with respect to 1 mole of the metal salt used. The amount of the aqueous solution of metal hydroxide is 1 to 4 equivalents, and preferably 2 equivalents, with respect to 1 mole of ethylenediaminetetraacetic acid used.

Needless to say, the process for obtaining the compound represented by the formula (A-6) from the compound represented by the formula (A-5), the process for obtaining the compound represented by the formula (III) from the compound represented by the formula (A-6), the process for obtaining the compound represented by the formula (II) from the compound represented by the formula (III), and the process for obtaining the compound represented by the formula (I) from the compound represented by the formula (II) can also be applied to the case where R^{8a} and/or R^{8b} is a hydrogen atom, and can be carried out in the same manner as described above.

Note that between the guanidinylation step and the subsequent amidite formation step, a protection reaction and/or deprotection reaction of the nucleic acid base portion, or a protection reaction and/or deprotection reaction of the 3'-position and/or 5'-position hydroxy group may be carried out as needed. For example, the guanidinylation step may be performed using, as a raw material, a compound in which R² in the formula (VI) in the guanidinylation step is a hydroxy group protecting group for nucleic acid synthesis, and then the protecting group may be deprotected to obtain the compound represented by the formula (I), where R² is a hydrogen atom. Furthermore, the compound represented by the formula (I), where R² is a hydrogen atom, can be subjected to the subsequent amidite formation step.

### (Amidite Formation Step)

Then, a desired amidite can be produced by converting the above-mentioned guanidinylated compound represented by the formula (I), where R² and/or R³ is a hydrogen atom, or a salt thereof, into an amidite as follows, for example.

That is, an amidite (a compound in which R² and/or R³ in the formula (I) is represented by -P(R⁴)R⁵) (where R⁴ and R⁵ are as defined above) can be produced by converting, into -P(R⁴)R⁵, using an amidite reagent, a hydroxy group in the compound in which R² and/or R³ in the formula (I) is a hydroxy group.

This step can be performed under the conditions described in, for example, Org. Process Res. Dev. 2005, 9, 6, 730-737.

Specifically, for example, this step can be carried out using an activating agent (e.g., 4,5-dicyanoimidazole, 5-phenyltetrazole, or 1H-tetrazole) and an amidite reagent corresponding to -P(R⁴)R⁵ (e.g., 2-cyanoethoxy N,N,N',N'-tetraisopropylphosphorodiamidite, 2-cyanoethoxy N,N-diisopropylchlorophosphoroamidite, or the like).

The solvent that can be used in the above amidite formation step may be any solvent that does not affect the reaction. The solvent is preferably a halogenated hydrocarbon solvent such as dichloromethane or a nitrile solvent such as acetonitrile.

The reaction temperature is preferably 0°C to 50°C, and more preferably 20°C to 30°C.

The reaction time is, for example, 0.5 to 48 hours, preferably 1 to 36 hours, and more preferably 1 to 8 hours.

In this manner, an amidite (a compound in which R² in the formula (I) is represented by -P(R⁴)R⁵) can be produced from the above guanidinylated compound represented by the formula (I).

### (Oligonucleotide or Pharmacologically Acceptable Salt thereof)

In the present invention, an oligonucleotide or a pharmacologically acceptable salt thereof can be produced through nucleic acid synthesis using the compound represented by the above formula (I) or a salt thereof (hereinafter, may be referred to as "the compound of the formula (I) or the like").

Such an oligonucleotide has at least one nucleoside structure derived from the compound of the formula (I) or the like at any position. There is no particular limitation on the positions and number of the nucleoside structures contained in one oligonucleotide, and the oligonucleotide can be designed as appropriate depending on the purpose. The higher the number thereof is, the greater the oligonucleotide's binding affinity and specificity for the target nucleic acid, the higher the rates at which a duplex and a triplex are formed, and the higher the nuclease resistance. In this specification, 2',4'-bridged artificial nucleosides of the present invention and the above nucleoside structures contained in the oligonucleotide of the present invention are collectively referred to as "guanidine-bridged artificial nucleic acids" or "guanidine-bridged nucleic acids".

Oligonucleotides having such nucleoside structures and analogues thereof have a structure fixed by a bridge of a sugar moiety as described above, and therefore are resistant to degradation by various nucleases and can remain in the living body for a long period of time after administration to the living body. Furthermore, through electrostatic interactions caused by cationic guanidine present on the bridge of the sugar moiety, for example, a stable duplex is formed with mRNA, thereby inhibiting the biosynthesis of pathogenic proteins, or a triplex is formed with a double-stranded DNA in the genome, thereby inhibiting transcription into mRNA. Also, it is possible to suppress the proliferation of the infected virus.

With all these facts, oligonucleotides and analogues thereof synthesized using the compounds represented by the formula (I) according to the present invention or the like are useful as pharmaceutical agents (such as antisense molecules), such as antitumor agents and antiviral drugs, inhibiting the functions of specific genes to treat a disease.

In particular, for antisense therapies, the binding affinity for complementary sense strand RNAs and the resistance to *in vivo* DNA-degrading enzymes are both required. Generally, it is known that a nucleic acid in the form of a single strand constantly has a structural fluctuation of a sugar moiety between the form close to a sugar moiety in a DNA duplex and the form close to a sugar moiety in a DNA-RNA duplex or a RNA duplex. When a single-stranded nucleic acid forms a duplex with a complementary RNA strand, the sugar structure is fixed. In view of this, in the compound of the formula (I) according to the present invention and the like, the sugar moiety is fixed in advance in a state in which it forms a double strand, and thus is likely to form a duplex with a target RNA strand and can be stably present. It is also known that nucleic acid duplexes are stabilized by hydration water connected like chains, called a network of water molecules. The compound of the formula (I) according to the present invention and the like have a guanidino structure in its bridged part, and thus is expected to, for example, improve the ability to form duplexes due to electrostatic interactions and hydration effects, as well as improve enzyme resistance. Furthermore, by introducing a guanidino structure into the bridged part, the position of the cation can be fixed, and it is also expected that electrostatic interactions and hydration effects will be enhanced. The compound of the formula (I) according to the present invention and the like have positive charge derived from a guanidinium group in the molecule, and are expected to improve cellular uptake efficiency and improve the rate of hybridization for a target nucleic acid, compared to natural nucleic acids and artificial nucleic acids known so far. These are expected to enhance an antisense effect and increase the time the compound remains in the body, and it is also possible to reduce side effects and costs by reducing the dosage.

The oligonucleotide of the present invention may contain at least one phosphorothioate bond within its structure.

Additives typically used in the field of pharmaceutical formulation technology, such as excipients, binders, preservatives, oxidation stabilizers, disintegrants, lubricants, and flavoring substances, can be added to oligonucleotides that can be obtained using the compound of the formula (I) according to the present invention or the like or pharmacologically acceptable salts thereof to prepare parenteral formulations or liposomal formulations. Also, for example, topical formulations such as liquids, creams, and ointments can be prepared by adding pharmaceutical carriers typically used in the art.

### Examples

Hereinafter, the present invention will be described in greater detail using examples. However, the present invention is not limited to the examples below.

In the examples, "LC-MS" means liquid chromatography mass spectrometer, "NMR" means nuclear magnetic resonance, "CDCl₃" means deuterated chloroform, and "DMSO-d₆" means deuterated dimethyl sulfoxide. "(v/v)" means (volume/volume). In silica gel column chromatography purification, "qCV" (q is a number) means that the volume of the developing solvent used was q times the volume of the silica gel.

The measurement apparatuses and measurement conditions used in the following Examples 1 to 6 (excluding Example 1(1-1(2)), Example 2(2-1(2)), Example 2(2-1(3)), and Example 4(4-1(2)) are as follows.
(1) NMR
   Device used: JEOL ECX-400P and ECS-400 manufactured by JEOL Ltd.
   Measurement conditions: ¹H-NMR (DMSO-d₆, CDCl₃)
      ³¹P-NMR (CDCl₃)
(2) UHPLC
   Device used: ACQUITY Arc UHPLC system manufactured by Waters Corporation
   Column: Xbridge BEH C18, 2.5 pm, 3.0 × 100 mm, Column XP manufactured by Waters Corporation
   Mobile phase: Gradient

**[Table 1]**

| Time/Solvent | 10mM | CH₃CN(%) |
|---|---|---|
| | NH₄CO₃(%) | |
| 0 min | 50 | 50 |
| 6.00 min | 0 | 100 |
| 10.00 min | 0 | 100 |

Flow rate: 0.85 mL/min
Detection: PDA (200 to 400 nm)
Column temperature: 30°C
Analysis time: 10.00 min

The measurement apparatuses and measurement conditions used in the following Example 1(1-1(2)), Example 2(2-1(2)), Example 2(2-1(3)), Example 4(4-1(2)), and Example 7 are as follows.

### (1) NMR

Device used: JNM-ECP300, JNM-ECX300, or JNM-ECZ400S manufactured by JEOL Ltd.
Measurement conditions: ¹H-NMR (DMSO-d₆ or CDCl₃)

Tetramethylsilane (0.0 ppm) was used as an internal standard. J values are given in Hz, and chemical shifts are given in ppm. "s" stands for singlet, "d" stands for doublet, "t" stands for triplet, "q" stands for quartet, "quint" stands for quintet, "dd" stands for doublet of doublets, "m" stands for multiplet, "brs" stands for broad singlet, and "brm" stands for broad multiplet.

### (2) LC-MS

Unless otherwise specified, data was measured using ESI (electrospray ionization) under the following conditions. "ESI⁺" means ESI positive ion mode, and "ESI⁻" means ESI negative ion mode.

Unless otherwise specified, the LC purity was measured under either a condition A or B below, and calculated using the area percentage method.

LC-MS Analysis Condition A:
High-performance liquid chromatography: HPLC manufactured by Waters Corporation Column: ACQUITY UPLC BEH C18 1.7 pM 2.1 × 50 mm manufactured by Waters Corporation
Column oven temperature: 40°C
Eluent: Solution A: aqueous solution containing formic acid (0.1%)
   Solution B: acetonitrile solution containing formic acid (0.1%)
Gradient conditions:
   Measurement was started by mixing the solutions A and B in a ratio of 80/20 at a flow rate of 0.6 mL/min. The mixture ratio of solutions A and B was then changed linearly to 0/100 over a period of 3 minutes. Then, the mixture ratio of solutions A and B was fixed at 0/100 for 0.7 minutes. Thereafter, the mixture ratio of solutions A and B was changed linearly to 90/10 over 0.1 minutes at a flow rate of 0.8 mL/min. Then, the mixture ratio of solutions A and B was fixed at 90/10 for 1 minute.
   Detection wavelength: 254 nm

LC-MS Analysis Condition B:
High-performance liquid chromatography: HPLC manufactured by Waters Corporation
Column: ACQUITY UPLC BEH C18 1.7 pM 2.1 × 50 mm manufactured by Waters Corporation
Column oven temperature: 40°C
Eluent: Solution A: 10 mM aqueous solution of ammonium bicarbonate
   Solution B: 10 mM aqueous solution of ammonium bicarbonate / acetonitrile = 1:9
Gradient conditions:
   Measurement was started by mixing the solutions A and B in a ratio of 80/20 at a flow rate of 0.6 mL/min. The mixture ratio of solutions A and B was then changed linearly to 0/100 over a period of 3 minutes. Then, the mixture ratio of solutions A and B was fixed at 0/100 for 3.7 minutes. Thereafter, over 0.1 minutes, the mixture ratio of solutions A and B was changed linearly to 90/10, and the flow rate was changed linearly to 0.8 mL/min. Then, the mixture ratio of solutions A and B was fixed at 90/10 for 1 minute.
Detection wavelength: 254 nm

The measurement apparatuses and measurement conditions used in the following Example 8 are as follows.
(1) NMR
   Device used: JEOL ECX-400P and ECS-400 manufactured by JEOL Ltd.
   Measurement conditions: ¹H-NMR (DMSO-d₆, CDCl₃)
(2) UHPLC
   Device used: ACQUITY Arc UHPLC system manufactured by Waters Corporation
   Column: Xbridge BEH C18, 2.5 pm, 3.0 × 100 mm, Column XP manufactured by Waters Corporation
   Mobile phase: Gradient

**[Table 2]**

| Time/Solvent | H₂O(%) | CH₃CN(%) |
|---|---|---|
| 0 min | 95 | 0 |
| 0.60 min | 95 | 0 |
| 4.50 min | 0 | 95 |
| 7.50 min | 0 | 95 |
| 9.50 min | 0 | 100 |
| 10.00 min | 0 | 100 |

Flow rate: 0.85 mL/min
Detection: PDA(200 to 400 nm)
Column temperature: 40°C
Analysis time: 10.00 min

### (Reference Example 1: Preparation of Triethylamine-treated Silica Gel)

The triethylamine-treated silica gel used in Examples 2(2-1(2)), 2(2-1(3)), and 4(4-1(2)) described below was prepared as follows.

To 200 g of silica gel (FL 100D, manufactured by Fuji Silysia Chemical Ltd.), n-hexane (600 mL) was added, and the resulting mixture was stirred with a mechanical stirrer (170 rpm). Triethylamine (40 g) was added all at once, the mixture was stirred until the heat generation subsided (usually for about 1 hour), and then filtered through a glass filter. The filtered substance was transferred to a recovery flask and dried under reduced pressure using an evaporator to obtain 212 g of triethylamine-treated silica gel.

### (Example 1: Amidite (a3) Synthesis)

The following amidite (di-tBu-Fmoc-GuNA[t-Bu]-T amidite) (a3) was synthesized as follows:

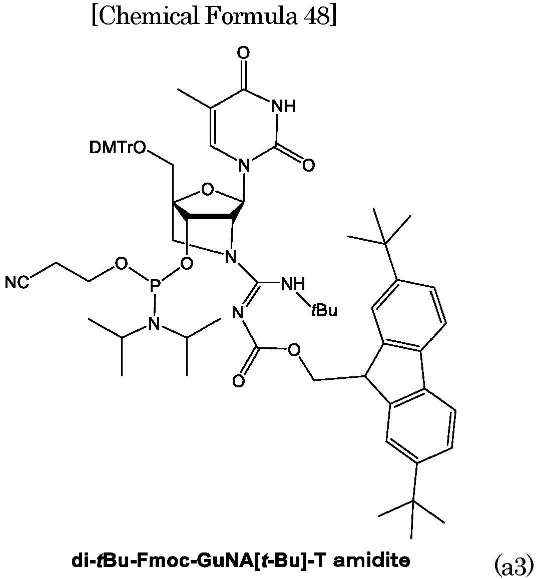

### (1-1(1): Compound a2 Synthesis (1))

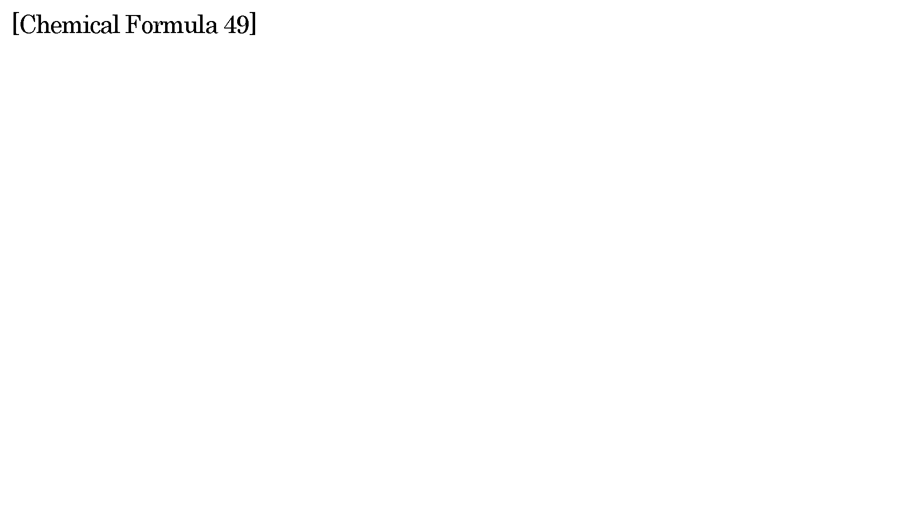

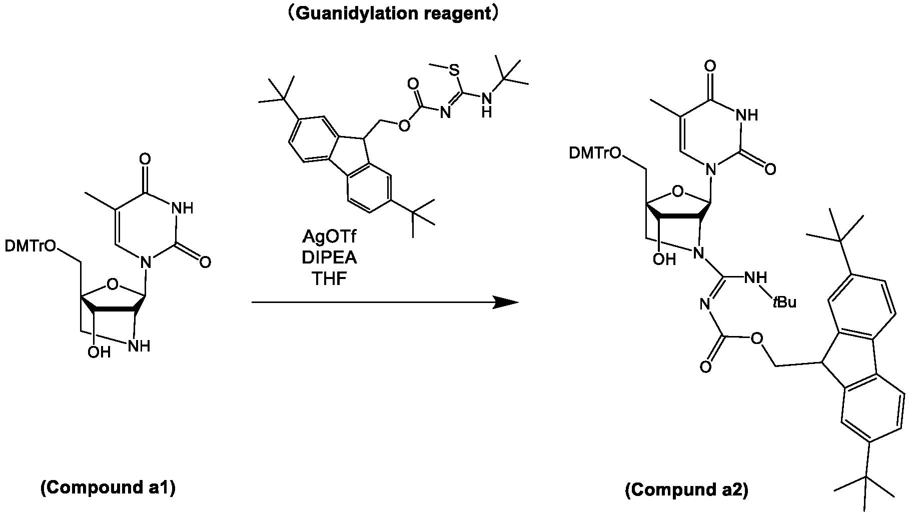

Under argon stream, a compound a1 (39.6 g, 69.3 mmol) obtained using the method described in A. S. Madsen et al., J. Org. Chem. 2012, 77, 10718-10728, and H. Sawamoto et al., Org. Lett. 2018, 20, 1928-1931, and the guanidinylation reagent (di-tBu-Fmoc-protected derivative) (40 g, 83.0 mmol) obtained in Examples described below were suspended in tetrahydrofuran (THF) (400 mL) and cooled to an internal temperature of 5°C or less in an ice bath. N,N-diisopropylethylamine (DIPEA) (18.2 mL, 104 mmol) and silver trifluoromethanesulfonate (AgOTf) (26.7 g, 104 mmol) were added, and the resulting mixture was stirred at room temperature for 2 hours and 35 minutes. Since the raw material remained, DIPEA (6.06 mL, 34.7 mmol) and AgOTf (8.91 g, 34.7 mmol) were added, and the resulting mixture was further stirred for 1 hour and 5 minutes.

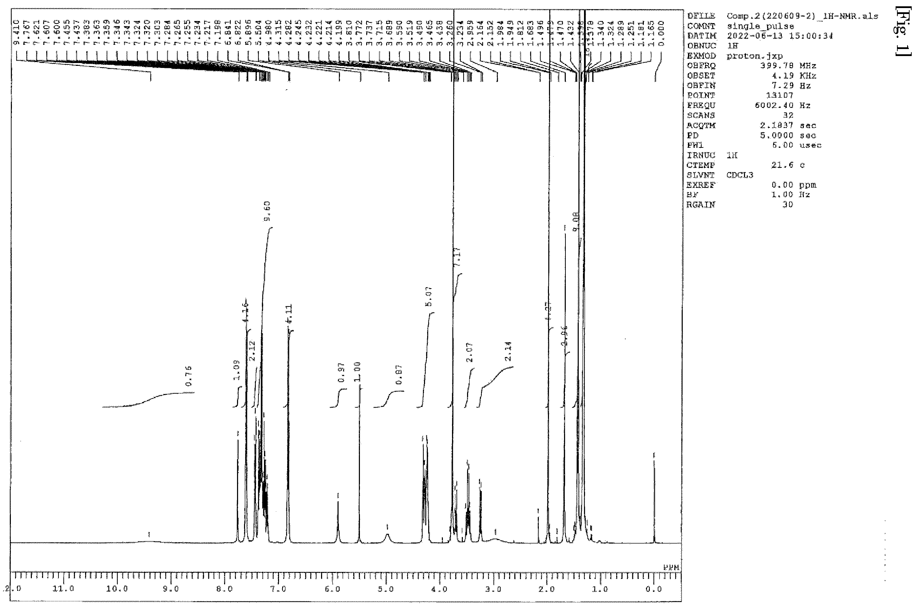

After the disappearance of the raw material was confirmed, ethyl acetate (250 mL) and a saturated aqueous NaCl solution (250 mL) were added to the reaction solution, the resulting mixture was stirred for a while and filtered through Celite. The residue was washed successively with ethyl acetate (300 mL) and water (250 mL), and the filtrate was separated. The organic layer was dried over Na₂SO₄, the drying agent was filtered off, and the filtrate was concentrated. The concentrated residue was purified through silica gel column chromatography (1.0 kg of neutral silica gel, developing solvent: n-heptane/acetone (1% triethylamine) = 80/20 → 70/30 → 60/40 → 50/50) and subjected to azeotropic distillation with acetonitrile to obtain the title compound a2 (68.8 g (containing acetonitrile in an amount of 3.87% by weight), 65.9 mmol, yield was 95%) as a white amorphous solid. FIG. 1 shows a ¹H-NMR spectrum of the compound a2 obtained in Example 1(1-1(1)).

### (1-1(2): Compound a2 Synthesis (2))

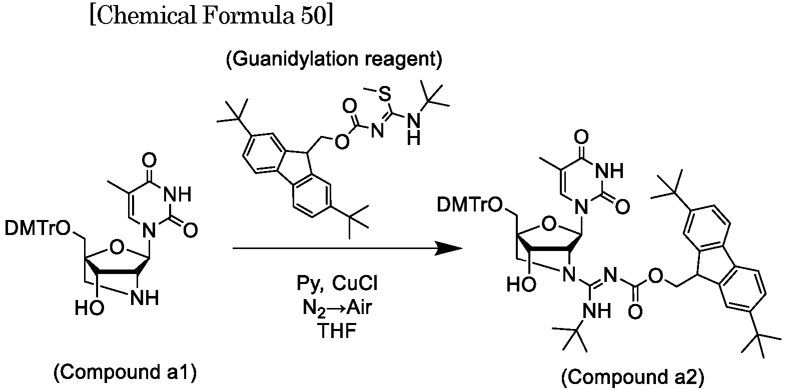

Pyridine (705 µL) was added to a suspension of the compound a1 (1.00 g) obtained using the method described in A. S. Madsen et al., J. Org. Chem. 2012, 77, 10718-10728 and H. Sawamoto et al., Org. Lett. 2018, 20, 1928-1931 in THF (15 mL) under a nitrogen atmosphere. The resulting mixture was stirred at room temperature for 7 minutes, 208 mg of copper (I) chloride was then added, and the resulting mixture was stirred for 1 hour. A solution of the guanidinylation reagent (di-tBu-Fmoc-protected derivative) (925 mg) in THF (2.0 mL) was added, the resulting mixture was stirred at 60°C for 30 minutes, the nitrogen atmosphere was changed to air, and the mixture was further stirred at 60°C for 2 hours.

The reaction mixture was cooled to room temperature in a water bath, and then insoluble matter was removed through filtration (washing: ethyl acetate 5 mL × 2). The filtrate was concentrated, and the residue was dissolved in ethyl acetate (15 mL), a solution prepared from EDTA (ethylenediaminetetraacetic acid) (511 mg), a 1 M aqueous solution of sodium hydroxide (3.5 mL), and water (10 mL) was added thereto, and the mixture was shaken well and allowed to stand.

The aqueous layer was removed, water (10 mL) was added to the organic layer, the resulting mixture was shaken well and allowed to stand. The aqueous layer was then removed, and the organic layer was concentrated under reduced pressure. The concentrated residue was purified through silica gel column chromatography (30 g of DIOL MB100-40/75 manufactured by Fuji Silysia Chemical Ltd., developing solvent: n-heptane/ethyl acetate = 90/10 (0.1 CV) → 70/30 (5 CV) → 30/70 (5 CV) → 0/100 (2 CV)) to obtain the title compound a2 (1.74 g, yield was 99%) as a cream-colored amorphous solid. Table 3 shows data on physical properties of the compound a2 obtained in Example 1(1-1(2)).

**[Table 3]**

| Data on Physical Properties of Compound a2 obtained in This Example 1 (1-1(2)) |
|---|
| LC/MS:Measuring conditions B, Retention time=4.15 min |
| LC/MS(ESI+) m/z;1004.5[M+H]⁺ |
| LC purity 99.4% |
| ¹H-NMR (CDCl₃,400MHz) δ: 1.34 (9H, s), 1.35 (9H, s), 1.46 (9H, s), 1.69 (3H, d, *J* = 0.9 Hz), 3.26 (1H, d, *J* = 10.3 Hz), 3.47 (1H, d, *J* = 11.0Hz), 3.51 (1H, d, *J* = 11.0Hz), 3.70 (1H, d, *J* = 10.1 Hz), 3.79 (6H, s), 4.21-4.34 (5H, m), 4.67 (1H, br s), 5.52 (1H, s), 5.86 (1H, br s), 6.84 (4H, d, *J* = 9.0Hz), 7.23 (1H, t, *J* = 7.0Hz), 7.30 (2H, t, *J* = 7.7 Hz), 7.34 (4H, d, *J* = 9.0Hz), 7.38 (1H, dd, *J* = 8.1, 2.0Hz), 7.38 (1H, dd, *J*= 8.1, 2.0Hz), 7.45 (2H, d, *J* = 8.4 Hz), 7.60 (1H, q, *J* = 1.1 Hz), 7.62 (2H, d, *J* = 8.1 Hz), 7.64 (1H, d, *J* = 1.5 Hz), 7.77 (1H, d, *J* = 1.3 Hz), 8.38 (1H, br s). |

### (1-2: Amidite (a3) Synthesis)

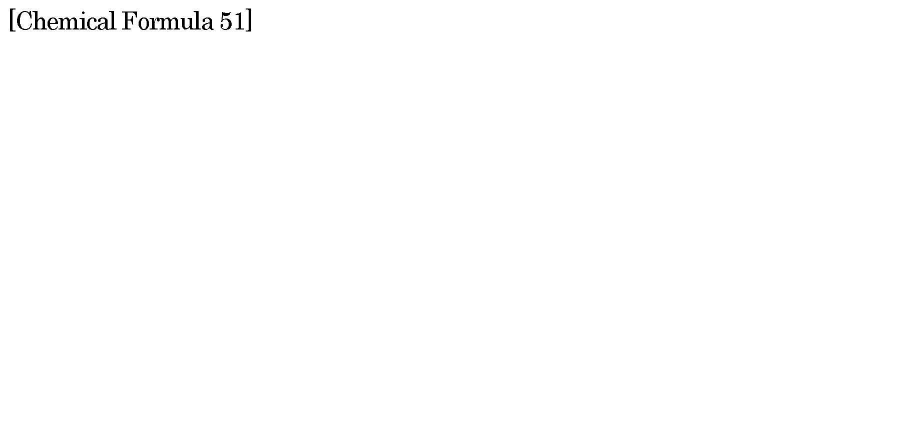

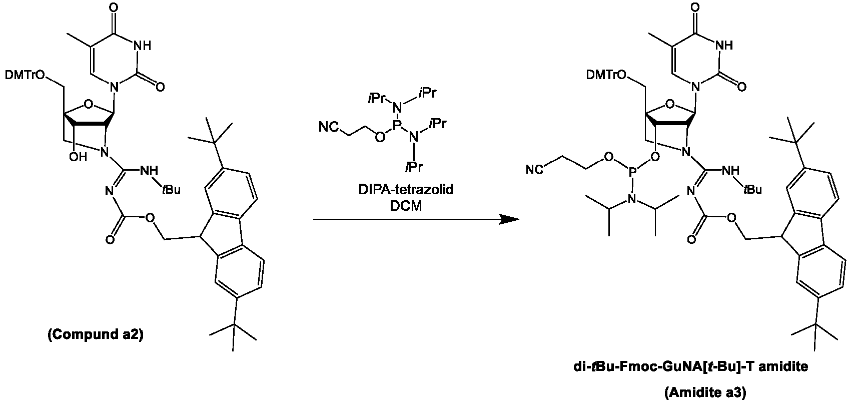

Under argon stream, the compound a2 (65.0 g (containing acetonitrile in an amount of 3.87% by weight), 62.2 mmol) obtained in Example 1(1-1(1)) above was charged into dry dichloromethane (DCM) (600 mL) and cooled to an internal temperature of 10°C or less in an ice bath. Then, 2-cyanoethyl N,N,N',N'-tetraisopropylphosphorodiamidite (37.5 g, 124 mmol) was added, and the resulting mixture was washed with dry DCM (30 mL). Diisopropylammonium tetrazolide (DIPA-tetrazolide) (32.0 g, 187 mmol) was added while the internal temperature was maintained at 6°C or less, the resulting mixture was washed with dry DCM (20 mL) and stirred for 15 minutes. The temperature of the reaction solution was increased to room temperature, and the reaction solution was stirred for 3 hours and 25 minutes.

After the disappearance of the raw material was confirmed, the reaction was quenched by pouring the reaction solution into a saturated aqueous NaHCO₃ solution (600 mL) containing ice, and the resulting mixture was separated. The aqueous layer was extracted with chloroform (500 mL). The organic layers were combined together, washed with a saturated aqueous NaCl solution (600 mL) and dried over Na₂SO₄, and the drying agent was filtered off, and the filtrate was concentrated. The concentrated residue was purified through silica gel column chromatography (1.0 kg of neutral silica gel, developing solvent: n-heptane/ethyl acetate (1% triethylamine) = 80/20 → 70/30 → 60/40 → 50/50) to obtain a crude product containing impurities derived from the amidite reagent.

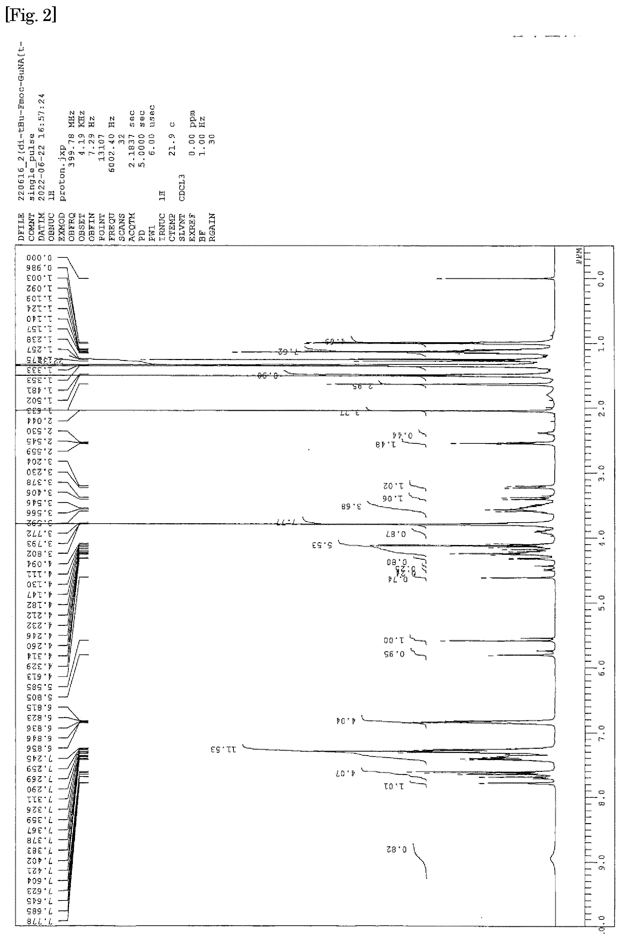

The obtained crude product was dissolved in n-heptane/ethyl acetate c = 1/1 (800 mL) and washed twice with water/N,N-dimethylformamide (DMF) = 1/1 (400 mL), twice with water (400 mL), and with a saturated aqueous NaCl solution (400 mL). The organic layer was dried over Na₂SO₄, the drying agent was filtered off, and the filtrate was concentrated to obtain the title amidite a3 (61.1 g (containing ethyl acetate in an amount of 8.42% by weight), 46.5 mmol, yield was 74%) as a white amorphous solid. FIG. 2 shows a ¹H-NMR spectrum of the obtained amidite a3. Also, data on physical properties of the obtained amidite a3 were as follows: ³¹P-NMR (CDCl₃): δ148.7, 150.6 ppm.

### (Example 2: Amidite (b3) Synthesis)

The following amidite (di-tBu-Fmoc-GuNA[t-Bu]-A amidite) (b3) was synthesized as follows:

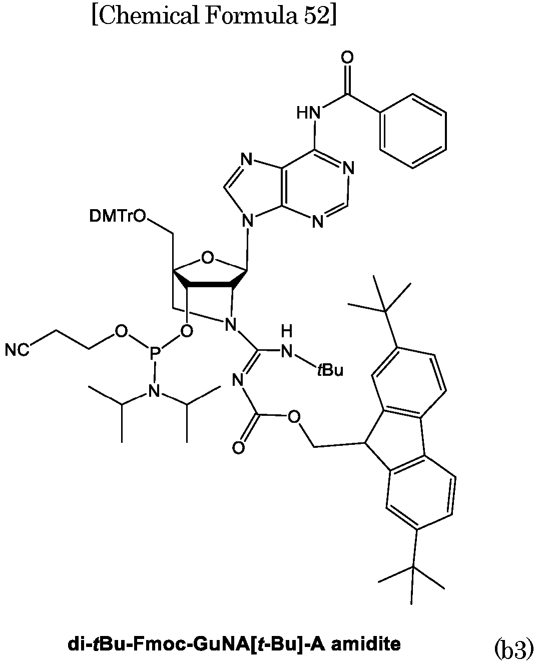

### (2-1(1): Compound b2 Synthesis (1))

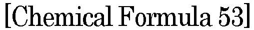

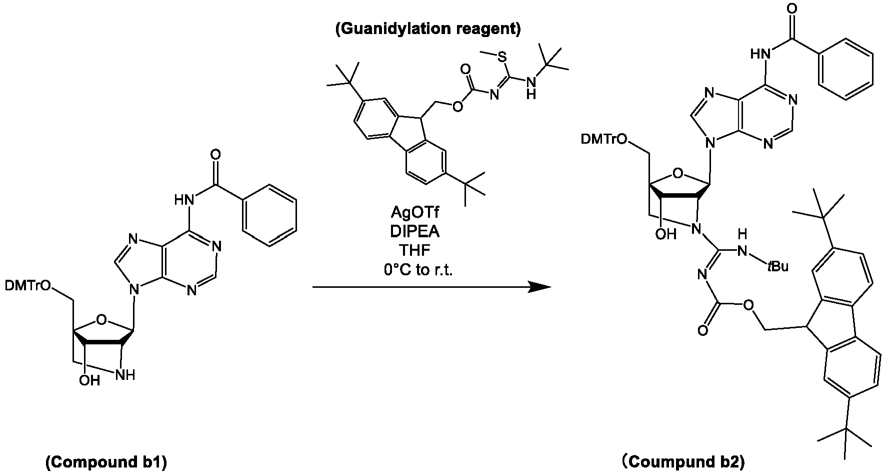

Under argon stream, a compound b1 (45.0 g, 65.7 mmol) obtained using the method described in S. Kumagai et al., Org. Biomol. Chem., 2020, 18, 9461-9471 and Org. Lett. 2018, 20, 1928-1931, and the guanidinylation reagent (di-tBu-Fmoc-protected derivative) (37.9 g, 79.0 mmol) obtained in Examples described below were suspended in THF (450 mL) and cooled to an internal temperature of 5°C or less in an ice bath. Then, DIPEA (17.2 mL, 99.0 mmol) and AgOTf (25.3 g, 99.0 mmol) were added, and the resulting mixture was stirred at room temperature for 2 hours and 50 minutes. Since the raw material remained, DIPEA (5.74 mL, 33.0 mmol) and AgOTf (8.44 g, 33.0 mmol) were added, and the resulting mixture was further stirred for 2 hours.

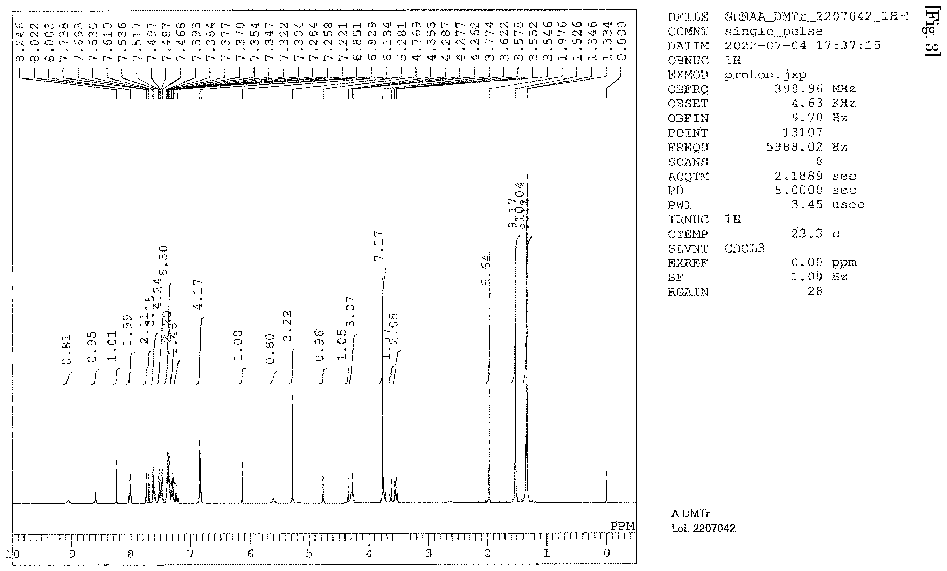

After the disappearance of the raw material was confirmed, ethyl acetate (450 mL) and a saturated aqueous NaCl solution (900 mL) were added to the reaction solution. After the addition, the resulting mixture was stirred for a while and filtered through Celite. The residue was washed successively with ethyl acetate (450 mL) and water (900 mL), and the filtrate was separated. The organic layer was dried over Na₂SO₄, the drying agent was filtered off, and the filtrate was concentrated. The concentrated residue was purified through silica gel column chromatography (400 g of neutral silica gel, developing solvent: n-heptane/acetone (1% triethylamine) = 80/20 → 70/30 → 60/40 → 50/50) and subjected to azeotropic distillation with acetonitrile and DCM to obtain a crude product (60.7 g (containing acetonitrile in an amount of 2.6% by weight and DCM in an amount of 7.4% by weight), 34.9 mmol, yield was 74%) as a white amorphous solid. FIG. 3 shows a ¹H-NMR spectrum of the compound b2 obtained in Example 2(2-1(1)).

### (2-1(2): Compound b2 Synthesis (2))

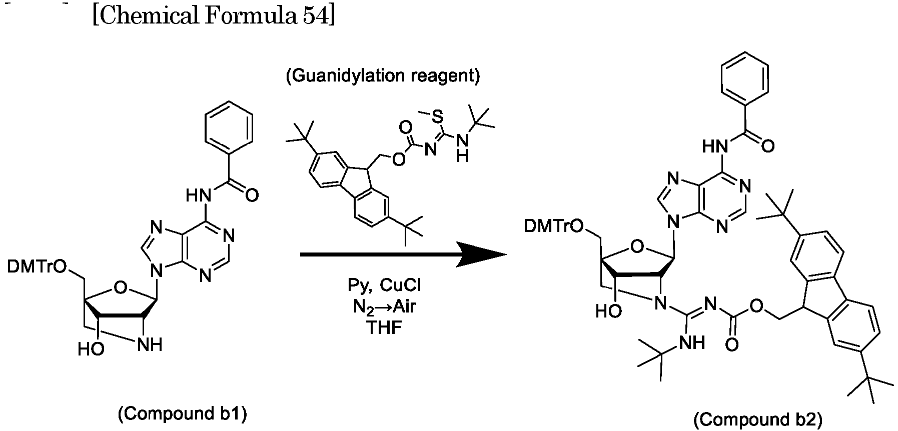

A suspension of the compound b1 (1.00 g) obtained using the method described in S. Kumagai et al., Org. Biomol. Chem., 2020, 18, 9461-9472 in THF (15 mL) was stirred at room temperature in a nitrogen atmosphere for 20 minutes, and pyridine (590 µL) and 361 mg of copper (I) chloride were added, and the resulting mixture was stirred for 2 hours. A solution of guanidinylation reagent (di-tBu-Fmoc-protected derivative) (722 mg) in THF (2.0 mL) was added, the resulting mixture was stirred at 60°C for 6 minutes, the nitrogen atmosphere was changed to air, and the mixture was further stirred at 60°C for 4 hours.

The reaction mixture was cooled to room temperature in a water bath, and then insoluble matter was removed through filtration (washing: toluene 10 mL × 1). The filtrate was concentrated under slightly reduced pressure and most of THF was distilled off, and then a solution prepared from EDTA (860 mg), a 1 M aqueous solution of sodium hydroxide (5.8 mL), and water (10 mL) was added to the residue, and the resulting mixture was stirred vigorously for 10 minutes and allowed to stand. The aqueous layer was removed, and a solution prepared from EDTA (860 mg), a 1 M aqueous solution of sodium hydroxide (5.8 mL), and water (10 mL) was added to the organic layer, and the resulting mixture was stirred vigorously for 10 minutes and allowed to stand. The aqueous layer was removed, water (10 mL) was added to the organic layer, the resulting mixture was stirred vigorously for 10 minutes and allowed to stand. The aqueous layer was then removed, and the organic layer was concentrated under reduced pressure.

The concentrated residue was purified through silica gel column chromatography (22 g of triethylamine-treated silica gel, developing solvent: n-hexane/ethyl acetate = 50/50 (4 CV) → 0/100 (3.5 CV)) to obtain the title compound b2 (949 mg, yield was 58%) as a white amorphous solid. Table 4 shows data on physical properties of the compound b2 obtained in Example 2(2-1(2)).

**[Table 4]**

| Data on Physical Properties of Compound b2 obtained in This Example 2 (2-1(2)) |
|---|
| LC/MS: Measuring condition A, Retention time=3.27 min |
| LC/MS(ESI+) m/z; 1117.4[M+H]⁺ |
| LC purity 94.9% |
| ¹H-NMR (CDCl₃,400MHz) δ: 1.34 (9H, s), 1.35 (9H, s), 1.55 (9H, s), 3.53 (1H, d, *J* = 11.0 Hz), 3.55 (1H, d, *J* = 9.9 Hz), 3.63 (1H, d, *J* = 11.0 Hz), 3.75 (1H, d, *J* = 9.9 Hz), 3.79 (6H, s), 4.23-4.37 (4H, m), 4.74 (1H, s), 5.31 (1H, br s), 5.70 (1H, br s), 6.13 (1H, s), 6.85 (4H, d, *J* = 9.0 Hz), 7.24 (1H, t, *J* = 7.5 Hz), 7.32 (2H, t, *J* = 7.9 Hz), 7.34-7.41 (6H, m), 7.48 (2H, d, *J* = 7.7 Hz), 7.55 (2H, t, *J* = 7.5 Hz), 7.63 (2H, d, *J* = 7.9 Hz), 7.63 (1H, t, *J* = 7.5 Hz), 7.69 (1H, d, *J* = 1.3 Hz), 7.75 (1H, d, *J* = 1.3 Hz), 8.05 (2H, d, *J* = 8.1 Hz), 8.26 (1H, s), 8.66 (1H, s), 9.10 (1H, s). |

### (2-1(3): Compound b2 Synthesis (3))

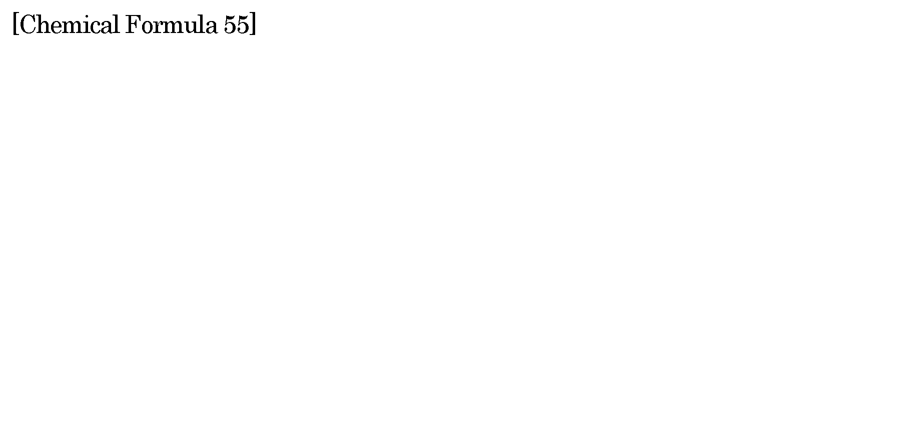

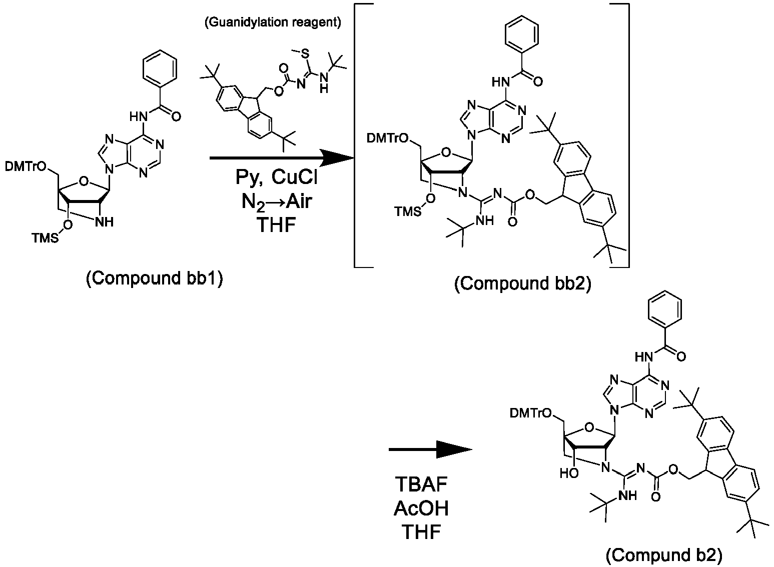

Pyridine (59 µL) and 36 mg of copper (I) chloride were added to a solution of the compound bb1 (111 mg) obtained using the method described in H. Sawamoto et al., Org. Lett. 2018, 20, 1928-1931 in THF (1.5 mL) in a nitrogen atmosphere, and the resulting mixture was stirred for 1 hour. A solution of the guanidinylation reagent (di-tBu-Fmoc-protected derivative) (77 mg) in THF (0.20 mL) was added, the resulting mixture was stirred at 60°C for 6 minutes, the nitrogen atmosphere was changed to air, and the mixture was further stirred at 60°C for 2 hours.

The reaction mixture was cooled to room temperature through air cooling and centrifuged, and the supernatant was removed. Toluene (1.5 mL) was added to the precipitate, and the resulting mixture was shaken well and centrifuged again, and the supernatant was removed. Toluene (1.5 mL) was added to the two supernatants combined, the resulting mixture was concentrated under slightly reduced pressure, and most of THF was distilled off. A solution prepared from EDTA (86 mg), a 1 M aqueous solution of sodium hydroxide (0.58 mL), and water (2.0 mL) was added to the residue, and the resulting mixture was shaken well for 10 minutes and allowed to stand. The aqueous layer was then removed, and the organic layer was concentrated under reduced pressure.

Acetic acid (8 µL) and tetrabutylammonium fluoride (ca. 1 mol/L in 146 µL of THF) were added to a solution of the concentrated residue in THF (1.5 mL), and the resulting mixture was stirred at room temperature for 15 hours. Toluene (2.0 mL) and water (2.0 mL) were added to the reaction solution, and the resulting mixture was then shaken well and allowed to stand. The aqueous layer was removed, water (2.0 mL) was added to the organic layer, the resulting mixture was shaken well and allowed to stand. The aqueous layer was then removed, and the organic layer was concentrated under reduced pressure. The concentrated residue was purified through silica gel column chromatography (5.0 g of triethylamine-treated silica gel, developing solvent: n-hexane/ethyl acetate = 50/50 (4 CV) → 0/100 (4 CV)) to obtain the title compound b2 (128 mg, yield was 79%) as a white amorphous solid. Table 5 shows data on physical properties of the compound b2 obtained in Example 2(2-1(3)).

**[Table 5]**

| Data on Physical Properties of Compound b2 obtained in This Example 2 (2-1(3)) |
|---|
| LC/MS: Measuring condition B, Retention time=4.19 min |
| LC/MS(ESI+) m/z; 1117.3[M+H]⁺ |
| LC purity 99.2% |
| ¹H-NMR (CDCl₃,400MHz) δ: 1.34 (9H, s), 1.36 (9H, s), 1.56 (9H, s), 3.53 (1H, d, *J* = 11.2 Hz), 3.55 (1H, d, *J* = 10.1 Hz), 3.63 (1H, d, *J* = 11.2 Hz), 3.75 (1H, d, *J* = 10.1 Hz), 3.79 (6H, s), 4.23-4.37 (4H, m), 4.74 (1H, s), 5.32 (1H, br s), 5.70 (1H, br s), 6.13 (1H, s), 6.85 (4H, d, *J* = 9.0 Hz), 7.24 (1H, t, *J* = 7.3 Hz), 7.32 (2H, t, *J* = 7.9 Hz), 7.34-7.41 (6H, m), 7.49 (2H, d, *J* = 7.9 Hz), 7.55 (2H, t, *J* = 7.7 Hz), 7.60-7.66 (3H, m), 7.69 (1H, d, *J* = 1.5 Hz), 7.75 (1H, d, *J* = 1.3 Hz), 8.05 (2H, d, *J* = 7.5 Hz), 8.26 (1H, s), 8.66 (1H, s), 9.11 (1H, s). |

### (2-2: Amidite (b3) Synthesis)

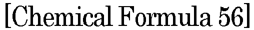

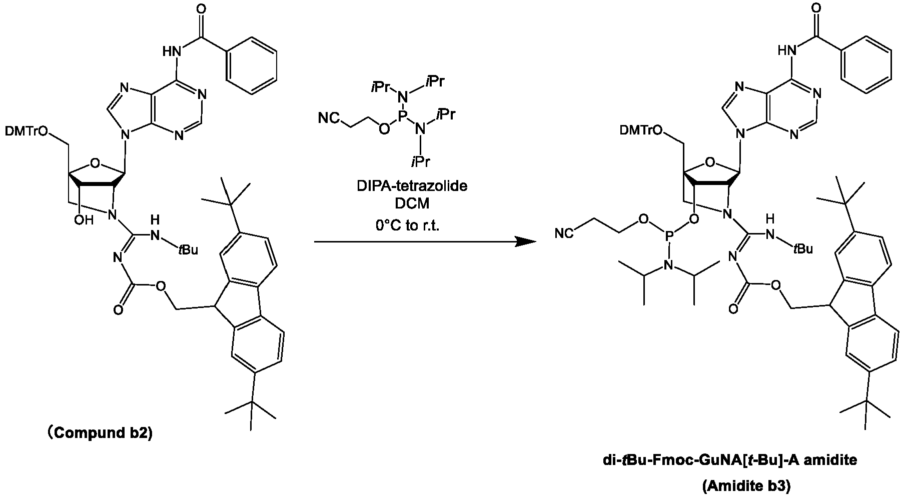

Under argon stream, the compound b2 (55.9 g (containing acetonitrile in an amount of 6.0% by weight and DCM in an amount of 7.3% by weight), 43.4 mmol) obtained in Example 2(2-1(1)) above was charged into dry DCM (485 mL) and cooled to an internal temperature of 3°C or less in an ice bath. DIPA-tetrazolide (22.3 g, 130 mmol) was added, and 2-cyanoethyl N,N,N',N'-tetraisopropylphosphorodiamidite (26.5 g, 87.0 mmol) dissolved in dry DCM (145 mL) was added while the internal temperature was maintained at 5°C or less. The temperature of the reaction solution was increased to room temperature, and the reaction solution was stirred for 3 hours and 15 minutes.

After the disappearance of the raw material was confirmed, the reaction was quenched by pouring the reaction solution into a saturated aqueous NaHCO₃ solution (600 mL) containing ice, and the resulting mixture was separated. The aqueous layer was extracted with DCM (550 mL). The organic layers were combined together, washed with a saturated aqueous NaCl solution (550 mL), dried over Na₂SO₄, and the drying agent was filtered off, and the filtrate was concentrated. The concentrated residue was purified through silica gel column chromatography (600 g of neutral silica gel, developing solvent: n-heptane/ethyl acetate (1% triethylamine) = 80/20 → 70/30 → 60/40 → 50/50) to obtain a crude product containing impurities derived from the amidite reagent.

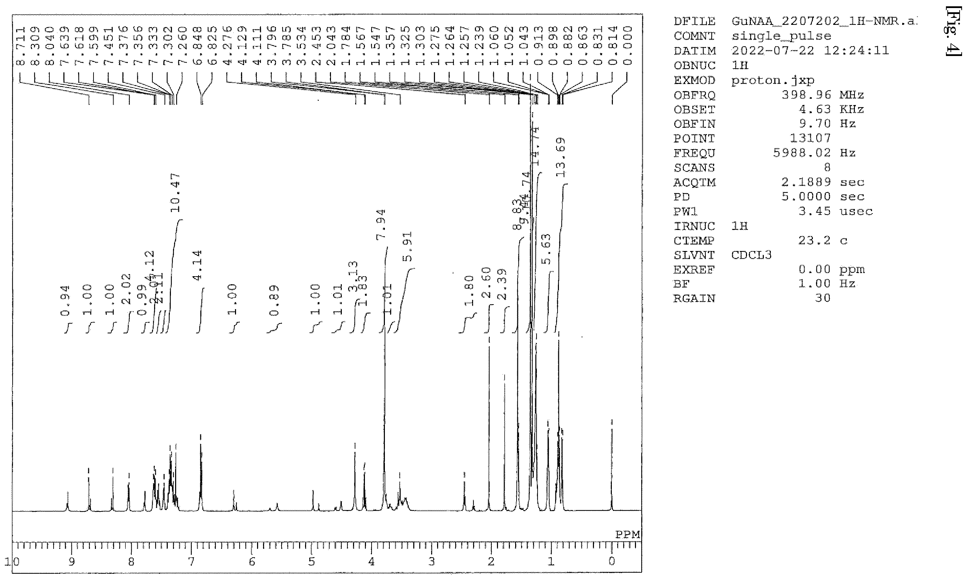

The obtained crude product was dissolved in n-heptane/ethyl acetate = 1/1 (600 mL) and washed twice with water/DMF = 1/1 (600 mL), twice with water (600 mL), and with a saturated aqueous NaCl solution (600 mL). The organic layer was dried over Na₂SO₄, the drying agent was filtered off, and the filtrate was concentrated to obtain a white amorphous solid of di-tBu-Fmoc-GuNA[t-Bu]-A amidite b3 (40.4 g (containing ethyl acetate in an amount of 5.6% by weight), 28.9 mmol, yield was 67%). FIG. 4 shows a ¹H-NMR spectrum of the obtained amidite b3. Also, data on physical properties of the obtained amidite b3 were as follows: ³¹P-NMR (CDCl₃): δ149.3, 150.8 ppm.

### (Example 3: Amidite (c5) Synthesis)

The following amidite (di-tBu-Fmoc-GuNA[t-Bu]-mC amidite) (c5) was synthesized as follows:

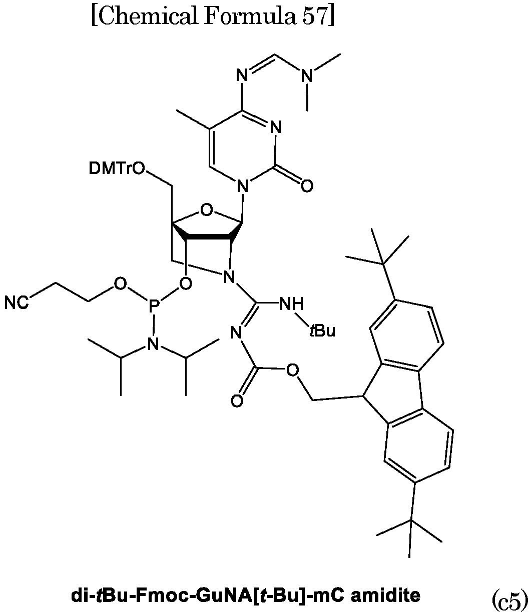

### (3-1: Compound c1 Synthesis)

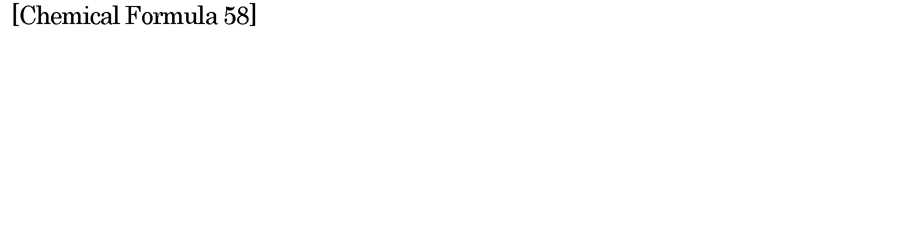

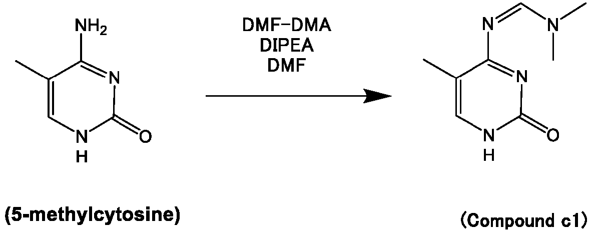

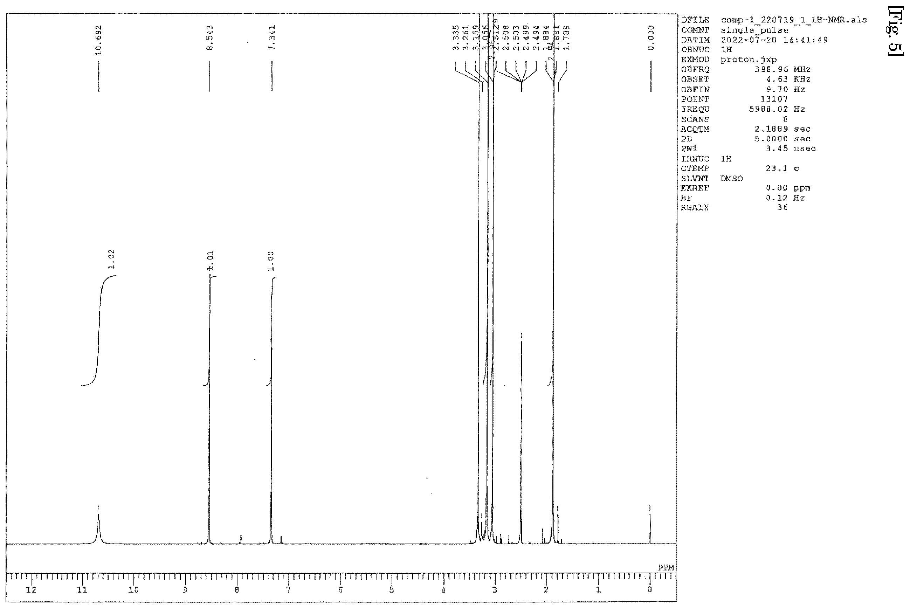

Under argon stream, 5-methylcytosine (100 g, 799 mmol) was suspended in DMF (1.00 L), DIPEA(140 mL, 799 mmol, 1.0 equivalent), and N,N-dimethylformamide dimethyl acetal (DMF-DMA) (531 mL, 4.00 mol, 5.0 equivalents) were added, and the resulting mixture was stirred at room temperature for 20 hours and 20 minutes. After the completion of the reaction was confirmed, methyl t-butyl ether (MTBE) (1.00 L) was added to the reaction solution. The resulting mixture was stirred for 1 hour and 20 minutes and filtered, and the filter cake was washed with MTBE (1.40 L). The resulting residue was dried at room temperature under reduced pressure to obtain the title compound c1 (109 g, 607 mmol, yield was 76%) as a white solid. FIG. 5 shows a ¹H-NMR spectrum of the compound c1 obtained.

### (3-2: Compound c2 Synthesis)

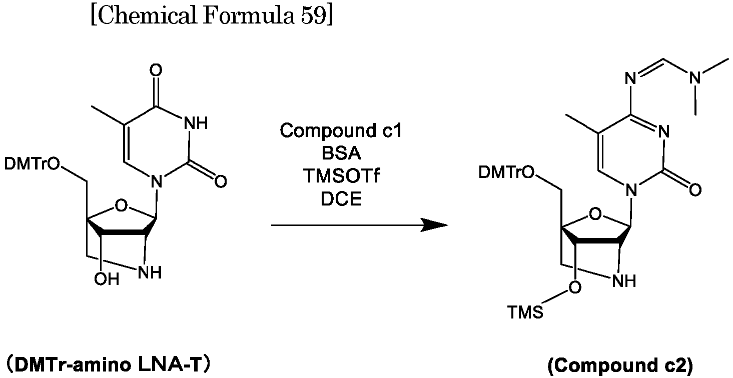

Under argon stream, DMTr-amino LNA-T (130 g, 227 mmol) obtained using the method described in A. S. Madsen et al., J. Org. Chem. 2012, 77, 10718-10728, and H. Sawamoto et al., Org. Lett. 2018, 20, 1928-1931, and the compound c1 (123 g, 682 mmol, 3.0 equivalents) obtained above were charged into 1,2-dichloroethane (DCE) (1.30 L) and cooled to an internal temperature of 5°C or less through immersion in an ice bath. N,O-bis(trimethylsilyl)acetamide (BSA) (416 g, 2.05 mol, 9.0 equivalents) was added over 1 hour and 5 minutes while the internal temperature was maintained at 10°C or less and the resulting mixture was then removed from the ice bath and stirred for 1 hour while the temperature thereof was increased to room temperature. The reaction solution was immersed in an ice bath to cool the internal temperature thereof to 5°C or less, and trimethylsilyl trifluoromethanesulfonate (TMSOTf) (7.58 g, 34.1 mmol, 0.15 equivalents) was added dropwise over 5 minutes. Thereafter, the mixture was stirred for 15 hours and 30 minutes while the mixture was heated in a water bath at 45°C.

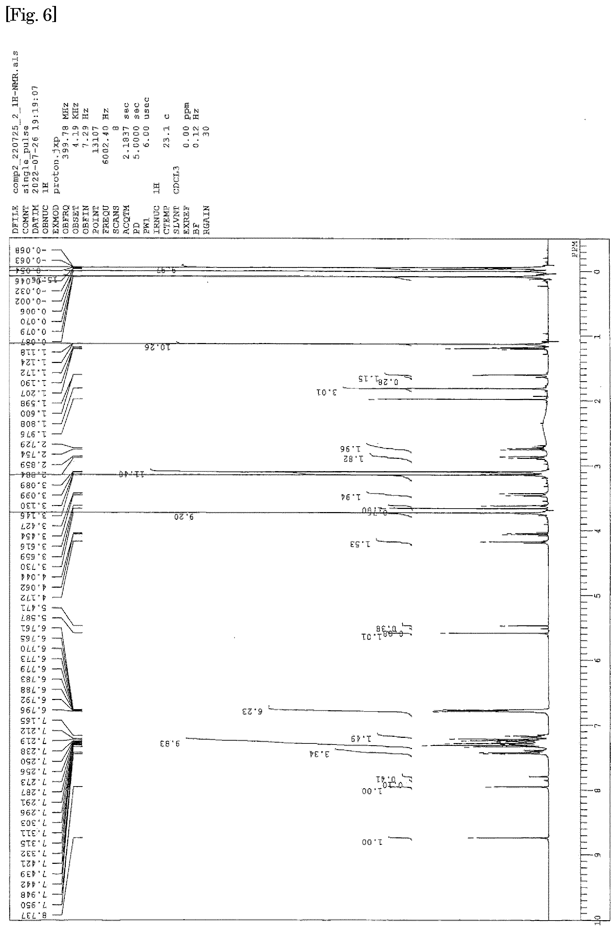

After the completion of the reaction was confirmed, the reaction solution was cooled to an internal temperature of 5°C. Ethyl acetate (1.30 L) was added, and the reaction was quenched by pouring the solution into a saturated aqueous NaHCO₃ solution (1.30 L) containing ice, and the resulting mixture was filtered through Celite. The filter cake was washed with ethyl acetate (500 mL), the filtrate was separated, and the aqueous layer was extracted once with ethyl acetate (700 mL). The organic layers were combined together and concentrated under reduced pressure. The resulting concentrated residue was dissolved in MTBE (2.00 L), and the solution was washed once with water (2.00 L) and once with a saturated aqueous NaCl solution (2.00 L). The organic layer was dried over Na₂SO₄, the drying agent was filtered off, and the filtrate was concentrated to obtain the title compound c2 (198 g (crude product)) containing impurities as a brown amorphous solid. FIG. 6 shows a ¹H-NMR spectrum of the compound c2 obtained.

### (3-3: Compound c3 Synthesis)

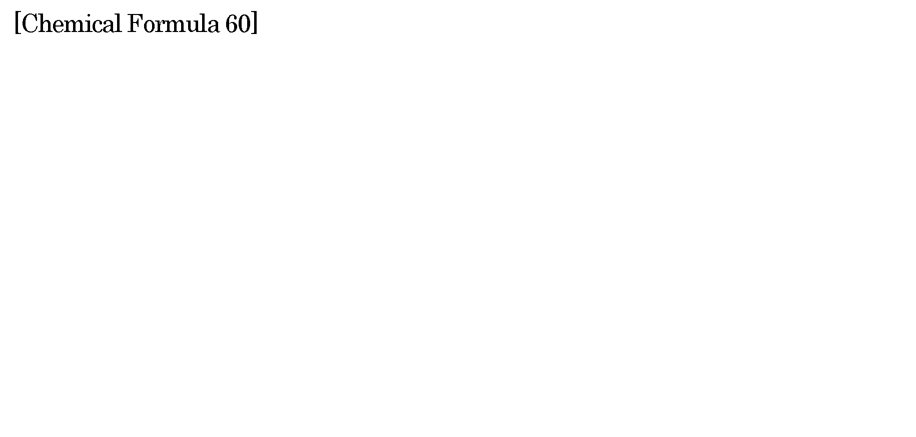

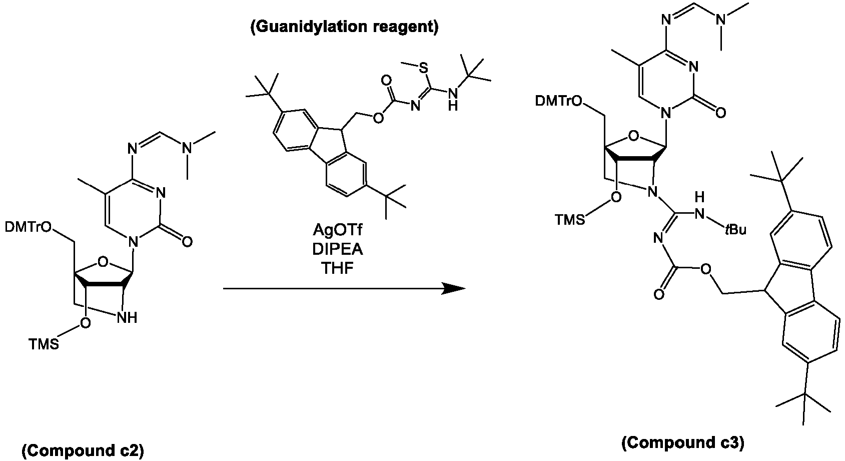

Under argon stream, the compound c2 (198 g (crude product), 227 mmol (since it was a crude product, the amount thereof was calculated based on the amount of the compound c2 (DMTr-ALNA-T) charged)) obtained above, the guanidinylation reagent (di-tBu-Fmoc-protected derivative) (120 g, 250 mmol, 1.1 equivalents) obtained in the examples described later, and DIPEA (59.5 mL, 341 mmol, 1.5 equivalents) were suspended in THF (1.50 L), and the resulting mixture was cooled to an internal temperature of 25°C or less in a water bath. AgOTf (87.5 g, 341 mmol) was added in portions over 5 minutes, washed with THF (100 mL), and stirred at room temperature for 1 hour and 50 minutes.

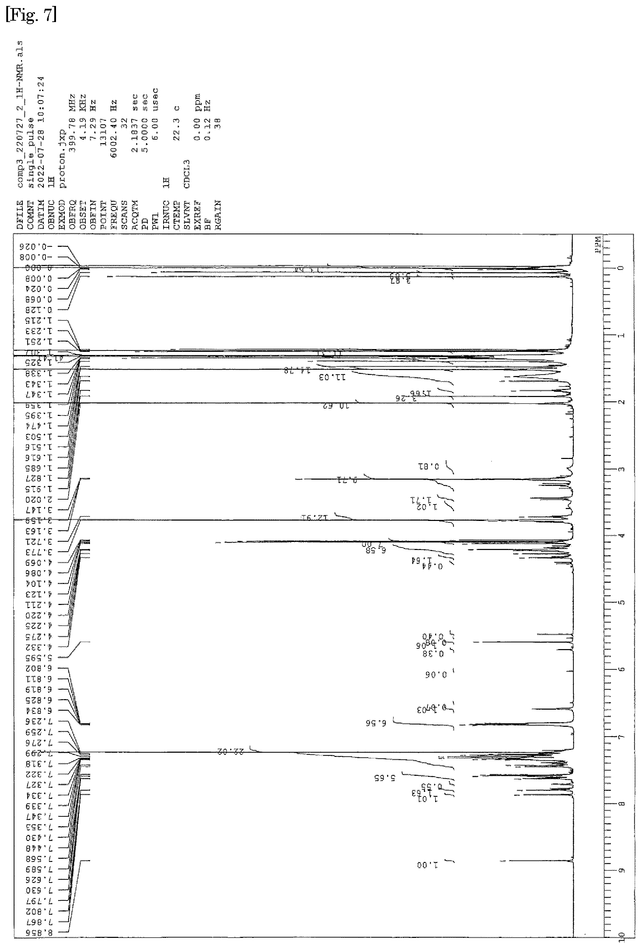

After the disappearance of the raw material was confirmed, ethyl acetate (1.30 L) and a saturated aqueous NaCl solution (1.30 L) were added to the reaction solution. After the addition, the resulting mixture was stirred for a while and filtered through Celite. The residue was washed with ethyl acetate (500 mL), and the filtrate was separated. The organic layer was washed with water (1.30 L) and dried over Na₂SO₄, the drying agent was filtered off, and the filtrate was concentrated to obtain the title compound c3 (241 g (crude product)) as an orange-brown amorphous solid. FIG. 7 shows a ¹H-NMR spectrum of the compound c3 obtained.

### (3-4: Compound c4 Synthesis)

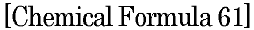

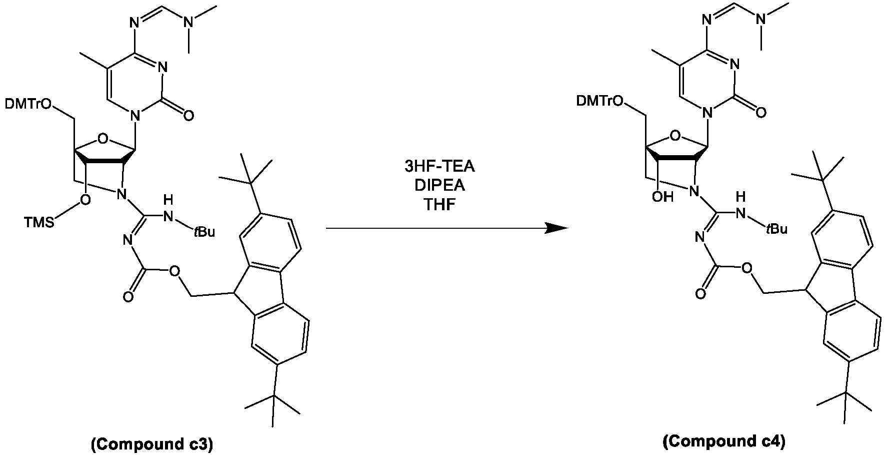

Under argon stream, the compound c3 (241 g (crude product), 227 mmol (since it was a crude product, the amount thereof was calculated based on the amount of the compound c3 (DMTr-ALNA-T) charged)) obtained above was charged into THF (2.50 L), and the resulting mixture was cooled to an internal temperature of 20°C or less in a water bath. DIPEA (119 mL, 681 mmol, 3.0 equivalents) and triethylamine trihydrofluoride (3HF-TEA) (54.9 g, 341 mmol, 1.5 equivalents) were added, and the resulting mixture was washed with THF (100 mL) and directly stirred at an internal temperature of 20°C or less for 2 hours.

After the disappearance of the raw material was confirmed, the reaction solution was poured into a saturated aqueous NaHCO₃ solution (2.00 L) containing ice. Ethyl acetate (1.30 L) was added, the resulting mixture was separated, and the organic layer was washed with a saturated aqueous NaCl solution (2.00 L). The organic layer was dried over Na₂SO₄, the drying agent was filtered off, and the filtrate was concentrated. The concentrated residue was purified through silica gel column chromatography (3.00 kg of neutral silica gel manufactured by Kanto Chemical Co., Inc., developing solvent: MTBE/ethyl acetate (0.5% pyridine) = 70/30 → 50/50 → 30/70 → 10/90) to obtain a crude product (146 g).

A portion (19 g) of the obtained crude product was purified again through silica gel column chromatography (285 g of neutral silica gel manufactured by Kanto Chemical Co., Inc., developing solvent: MTBE/THF (1.0% pyridine) = 90/10 → 70/30 → 50/50 → 30/70). The obtained pyridine-containing compound c4 was dissolved in ethyl acetate (200 mL), and the resulting mixture was washed twice with water (200 mL) and once with a saturated aqueous NaCl solution (200 mL). The organic layer was dried over Na₂SO₄, the drying agent was filtered off, and the filtrate was concentrated to obtain the title compound c4 (15.6 g (containing ethyl acetate in an amount of 9.29% by weight, dibutylhydroxytoluene (BHT) in an amount of 1.49% by weight) as a white amorphous solid.

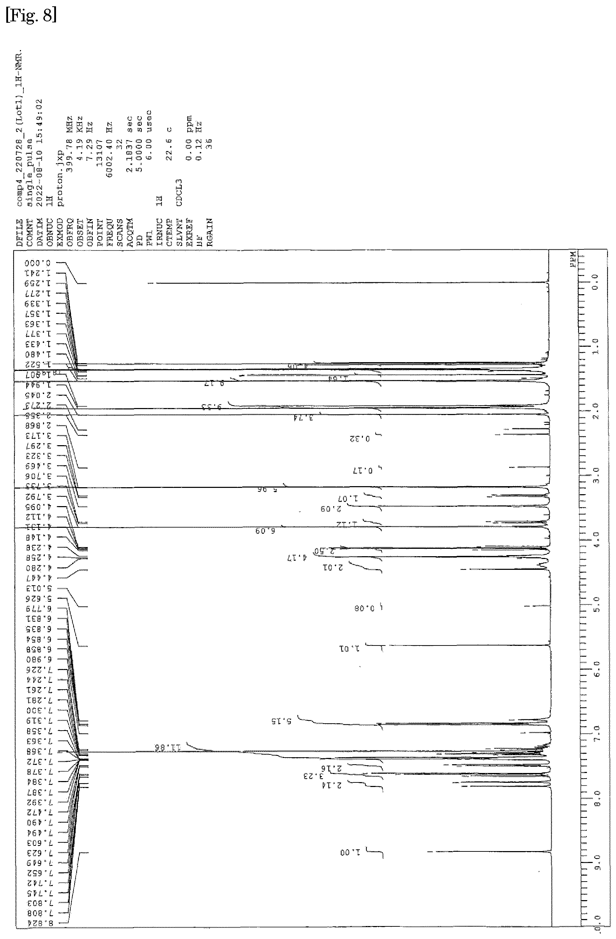

A portion (127 g) of the obtained crude product was purified again through silica gel column chromatography (2.00 kg of neutral silica gel manufactured by Kanto Chemical Co., Inc., developing solvent: MTBE/THF (1.0% pyridine) = 90/10 → 70/30 → 60/40 → 50/50 → 30/70). The obtained pyridine-containing compound c4 was dissolved in ethyl acetate (1.50 L), and the resulting mixture was washed twice with water (1.50 L) and once with a saturated aqueous NaCl solution (1.50 L). The organic layer was dried over Na₂SO₄, the drying agent was filtered off, and the filtrate was concentrated. Since pyridine remained in the obtained residue, the residue was dissolved again in ethyl acetate (1.50 L), and washed twice with water (1.50 L) and once with a saturated aqueous NaCl solution (1.50 L). The organic layer was dried over Na₂SO₄, the drying agent was filtered off, and the filtrate was concentrated to obtain the compound c4 (95.8 g (containing ethyl acetate in an amount of 6.70% by weight, BHT in an amount of 1.79% by weight)) as a white amorphous solid. The total amount of the compound c4 obtained was 101 g in terms of pure content (96.0 mmol, 3 steps, yield was 42%). FIG. 8 shows a ¹H-NMR spectrum of the compound c4 obtained.

### (3-5: Amidite c5 Synthesis)

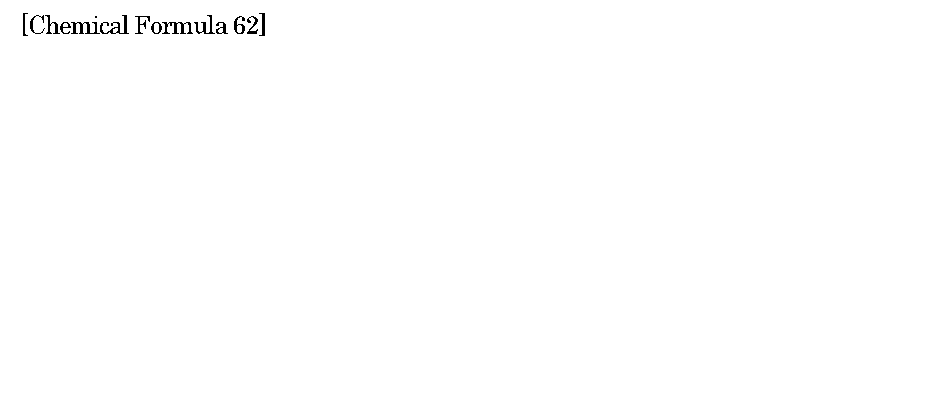

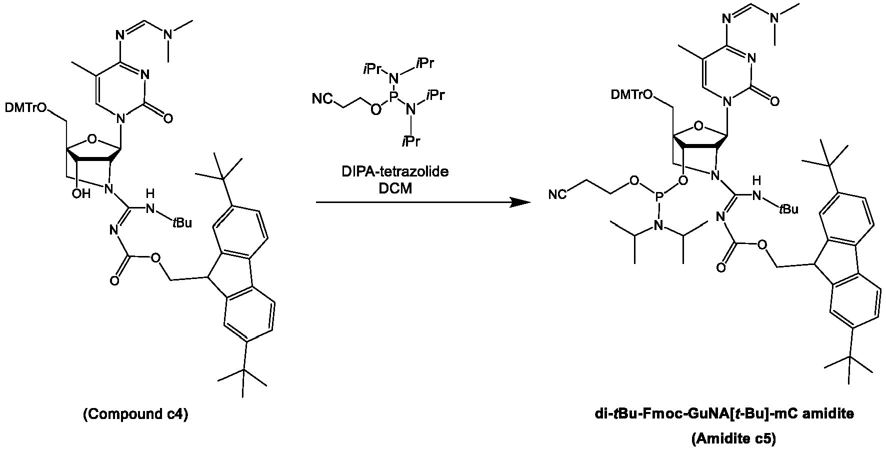

Under argon stream, the compound c4 (60.0 g (containing ethyl acetate in an amount of 6.70% by weight and BHT in an amount of 1.79% by weight), 51.9 mmol) obtained above was charged into dry DCM (500 mL) and cooled to an internal temperature of 10°C or less in an ice bath. Then, 2-cyanoethyl N,N,N',N'-tetraisopropylphosphorodiamidite (31.3 g, 104 mmol) was added, and the resulting mixture was washed with dry DCM (50 mL). DIPA-tetrazolide (26.6 g, 156 mmol) was added while the internal temperature was maintained at 6°C or less, the resulting mixture was washed with dry DCM (50 mL) and directly stirred for 10 minutes. The temperature of the reaction solution was increased to room temperature, and the reaction solution was stirred for 2 hours and 20 minutes.

After the disappearance of the raw material was confirmed, the reaction was quenched by pouring the reaction solution into a saturated aqueous NaHCO₃ solution (600 mL) containing ice, and the resulting mixture was separated. The aqueous layer was extracted with CHCl₃ (500 mL). The organic layers were combined together, washed with a saturated aqueous NaCl solution (600 mL) and dried over Na₂SO₄, and the drying agent was filtered off, and the filtrate was concentrated. The concentrated residue was purified through silica gel column chromatography (600 g of diol silica gel manufactured by Fuji Silysia Chemical Ltd., developing solvent: n-heptane/ethyl acetate (1% pyridine) = 70/30 → 60/40 → 50/50 → 40/60) to obtain the title amidite c5 (di-tBu-Fmoc-GuNA[t-Bu]-mC amidite) containing impurities.

The obtained amidite c5 was dissolved in n-heptane/ethyl acetate = 1/1 (2.00 L) and washed twice with water/DMF = 1/1 (1.50 L), twice with water (1.50 L), and once with a saturated aqueous NaCl solution (1.50 L). The organic layer was dried over Na₂SO₄, the drying agent was filtered off, and the filtrate was concentrated. The concentrated residue was purified through silica gel column chromatography (600 g of neutral silica gel manufactured by Kanto Chemical Co., Inc., developing solvent: n-heptane/ethyl acetate (1% pyridine) = 50/50 → 40/60 → 30/70 → 20/80) to obtain the amidite c5 (di-tBu-Fmoc-GuNA[t-Bu]-mC amidite) containing pyridine.

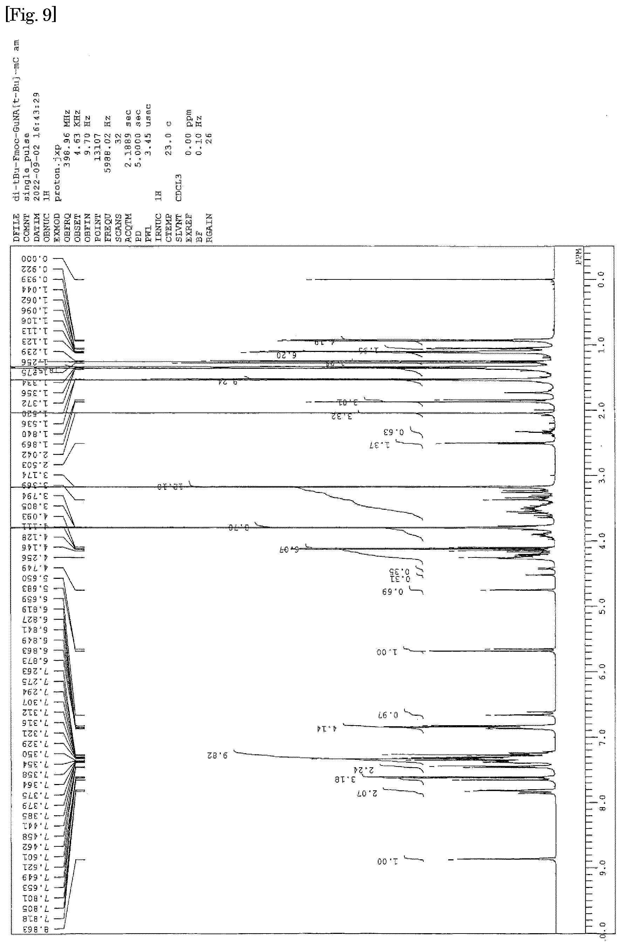

The obtained amidite c5 was dissolved in n-heptane/ethyl acetate = 1/1 (2.00 L) and washed twice with water/DMF = 1/1 (1.50 L), twice with water (1.50 L), and once with a saturated aqueous NaCl solution (1.50 L). The organic layer was dried over Na₂SO₄, the drying agent was filtered off, and the filtrate was concentrated to obtain the amidite c5 (62.7 g (containing ethyl acetate in an amount of 9.56% by weight), 45.0 mmol, yield was 89%) as a white amorphous solid. FIG. 9 shows a ¹H-NMR spectrum of the obtained amidite c5. Also, data on physical properties of the obtained amidite c5 were as follows: ³¹P-NMR (CDCl₃): δ148.3, 150.7 ppm.

### (Example 4: Amidite d3 Synthesis)

The following amidite (di-tBu-Fmoc-GuNA[t-Bu]-G amidite) (d3) was synthesized as follows:

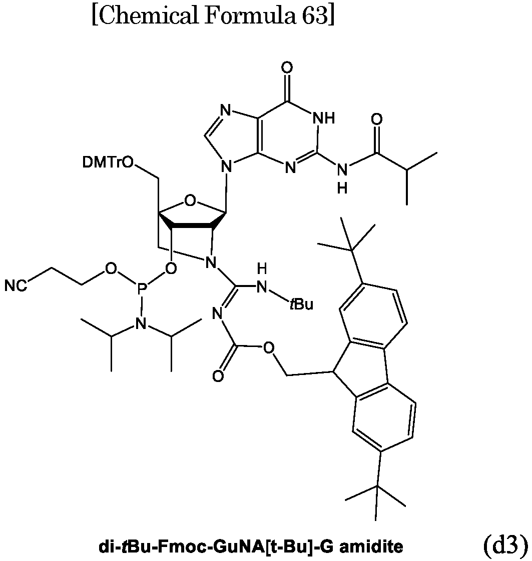

### (4-1(1): Compound d2 Synthesis (1))

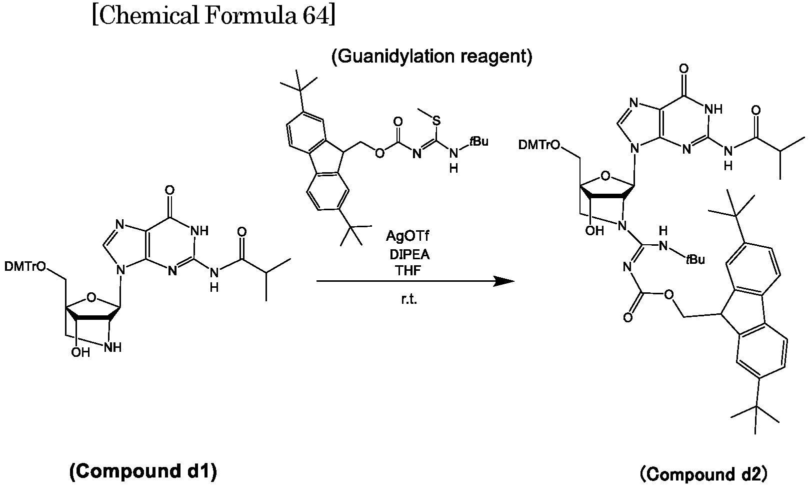

In an argon atmosphere, the compound d1 (9.00 g, 13.5 mmol) obtained in the Example described later and the guanidinylation reagent (7.79 g, 16.2 mmol) obtained in the Example described later were dissolved in dry THF (90 mL). DIPEA (3.54 mL, 20.3 mmol) and AgOTf (5.20 g, 20.3 mmol) were added, and the resulting mixture was stirred at room temperature for 1 hour and 40 minutes. The residue of the raw material was confirmed, and thus DIPEA (3.54 mL, 20.3 mmol) and AgOTf (5.20 g, 20.3 mmol) were added, and the resulting mixture was further stirred at room temperature for 1 hour and 40 minutes.

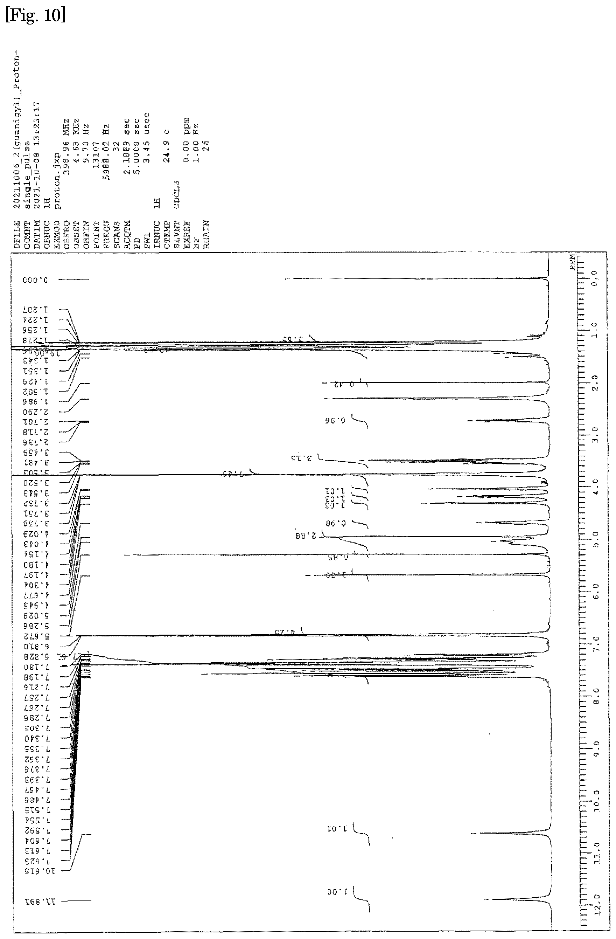

After the disappearance of the raw material was confirmed, the reaction was quenched by adding the reaction solution to the ethyl acetate / saturated aqueous NaCl solution = 1/1 (600 mL). After the addition, the resulting mixture was stirred for a while and filtered through Celite. The residue was washed with ethyl acetate (100 mL), and the filtrate was separated. The aqueous layer was extracted with ethyl acetate (200 mL), the combined organic layers were dried over Na₂SO₄, the drying agent was filtered off, and the filtrate was concentrated. The concentrated residue was purified through silica gel column chromatography (first: SI 50, SIZE 200 + SIZE 200 manufactured by Fuji Silysia Chemical Ltd., developing solvent: CHCl₃/methanol = 100/0 → 97/3 → 96/4 → 95/5, second: SI 50, SIZE 200 + SIZE 200 manufactured by Fuji Silysia Chemical Ltd., developing solvent: CHCl₃/methanol = 100/0 → 97/3 → 96/4 → 95/5). Fractions containing the target compound were concentrated to obtain the title compound d2 (11.63 g, 10.2 mmol (in terms of pure content), yield was 75%) as a light brown amorphous solid. FIG. 10 shows a ¹H-NMR spectrum of the compound d2 obtained in Example 4(4-1(1)).

### (4-1(2): Compound d2 Synthesis (2))

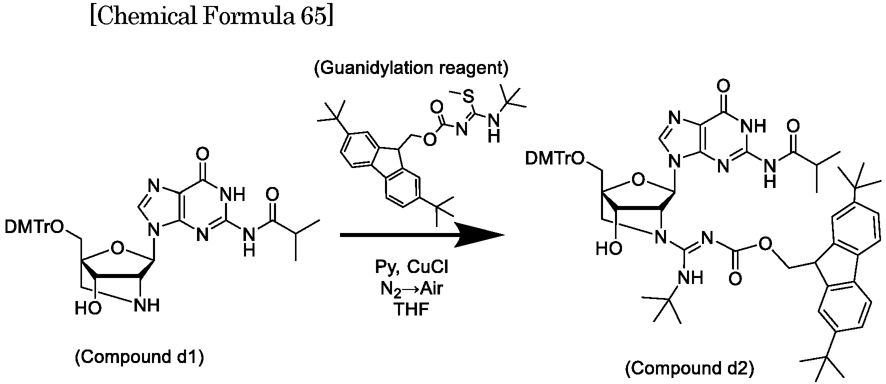

A solution of the compound d1 (100 mg) obtained in Examples described below in THF (1.5 mL) was stirred at room temperature under a nitrogen atmosphere for 30 minutes, pyridine (61 µL) and 18 mg of copper (I) chloride were added, and the resulting mixture was stirred for 40 minutes. A solution of the guanidinylation reagent (di-tBu-Fmoc-protected derivative) (79 mg) in THF (0.2 mL) was added, the resulting mixture was stirred at 60°C for 4 minutes, the nitrogen atmosphere was changed to air, and the mixture was further stirred at 60°C for 4 hours.

The reaction mixture was cooled to room temperature through air cooling, toluene (1.0 mL) was added, the resulting mixture was shaken well and then centrifuged, and the supernatant was removed. Toluene (2.0 mL) was added to the precipitate, and the resulting mixture was shaken well and centrifuged again, and the supernatant was removed. The two supernatants were combined, the resulting mixture was concentrated under slightly reduced pressure, and most of THF was distilled off. A solution prepared from EDTA (88 mg), a 1 M aqueous solution of sodium hydroxide (0.60 mL), and water (2.0 mL) was added to the residue, and the mixture was shaken well for 4 minutes and allowed to stand. The aqueous layer was then removed. Water (2.0 mL) was added to the organic layer, the resulting mixture was shaken well and allowed to stand. The aqueous layer was then removed. Water (2.0 mL) was added to the organic layer again, the resulting mixture was shaken well and allowed to stand. The aqueous layer was then removed, and the organic layer was concentrated under reduced pressure.

The concentrated residue was first purified through silica gel column chromatography (7.0 g of DIOL MB100-40/75 manufactured by Fuji Silysia Chemical Ltd., developing solvent: n-heptane/ethyl acetate = 50/50 (4 CV)→ 0/100 (4 CV)), and then further purified through silica gel column chromatography (5.0 g of triethylamine-treated silica gel, developing solvent: n-hexane/ethyl acetate = 50/50 (7 CV) → 0/100 (5 CV)) to obtain the title compound d2 (112 mg, yield was 68%) as a white amorphous solid. Table 6 shows data on physical properties of the compound d2 obtained in Example 4(4-1(2)).

**[Table 6]**

| Data on Physical Properties of Compound d2 obtained in This Example 4 (4-1(2)) |
|---|
| LC/MS: Measuring condition B, Retention time=4.16 min |
| LC/MS(ESI+) m/z; 1099.4[M+H]⁺ |
| LC purity 94.0% |
| ¹H-NMR (CDCl₃,400MHz) δ: 1.21 (3H, d, J = 6.8 Hz), 1.28 (9H, s), 1.29 (3H, d, J = 6.8 Hz), 1.35 (9H, s), 1.35 (9H, s), 2.70 (1H, septet, J = 6.8 Hz), 3.43-3.52 (3H, m), 3.69 (1H, d, J = 10.8 Hz), 3.78 (3H, s), 3.78 (3H, s), 4.03 (1H, t, J = 5.9 Hz), 4.21 (1H, dd, J = 10.8, 6.6 Hz), 4.21-4.26 (1H, m), 4.57 (1H, br s), 4.69 (1H, dd, J = 10.8, 5.9 Hz), 4.97 (2H, br s), 5.66 (1H, s), 6.84 (4H, d, J = 9.0 Hz), 7.22 (1H, t, J = 6.8 Hz), 7.31 (2H, t, J = 7.9 Hz), 7.34-7.41 (6H, m), 7.48 (2H, d, J = 8.4 Hz), 7.52 (1H, d, J = 0.9 Hz), 7.56 (1H, d, J = 1.3 Hz), 7.58-7.64 (3H, m), 10.56 (1H, br s), 11.93 (1H, br s). |

### (4-2: Amidite d3 Synthesis)

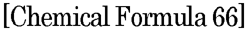

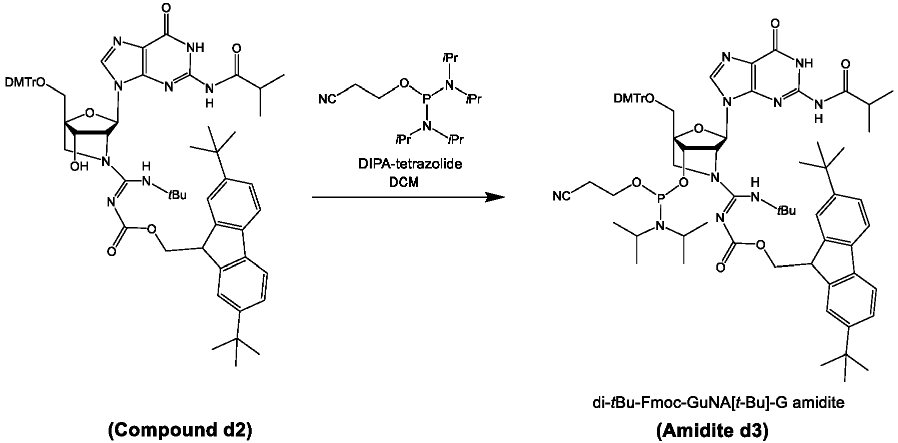

In an argon atmosphere, the compound d2 (13.6 g, 11.5 mmol (in terms of pure content)) obtained in Example 4(4-1(1)) above was charged into dry DCM (136 mL) and cooled to 4°C in an ice bath. Then, 2-cyanoethyl N,N,N',N'-tetraisopropylphosphorodiamidite (6.96 g, 23.1 mmol) and DIPA-tetrazolide (5.93 g, 34.6 mmol) were added, and the resulting mixture was stirred for 10 minutes while the mixture was cooled on ice, and the reaction solution was stirred for 2 hours and 45 minutes while the temperature of the reaction solution was increased to room temperature.

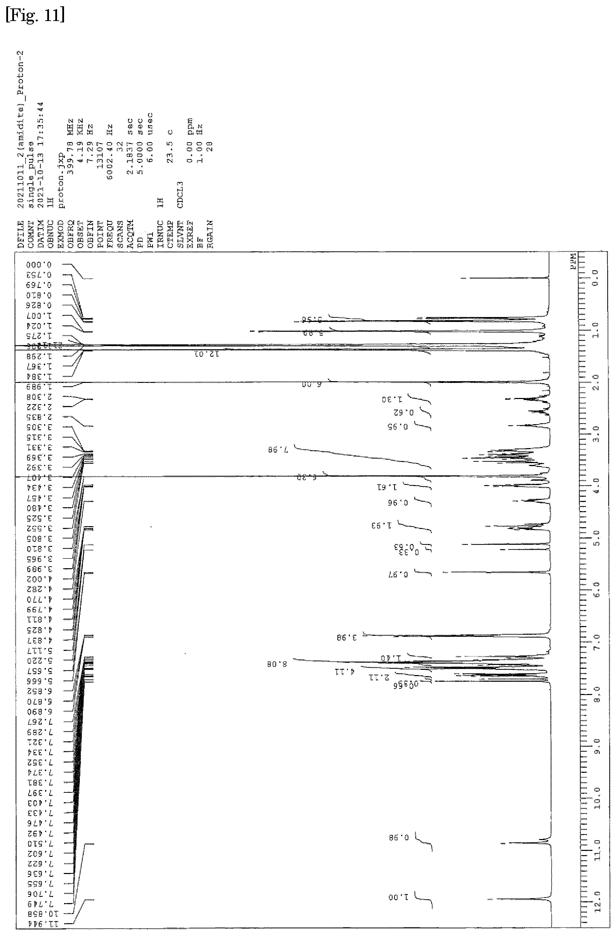

After the disappearance of the raw material was confirmed, the reaction was quenched by pouring the reaction solution into a mixture (300 mL) of ice and the saturated aqueous NaHCO₃ solution, CHCl₃ (300 mL) was added, and the resulting mixture was separated. The aqueous layer was extracted with CHCl₃ (200 mL). The organic layers were combined together, and washed with a saturated aqueous NaCl solution (300 mL). The organic layer was dried over Na₂SO₄, the drying agent was filtered off, and the filtrate was concentrated. The concentrated residue was purified through silica gel column chromatography (SI 50, SIZE 200 + 180 g of neutral silica gel, manufactured by Fuji Silysia Chemical Ltd., developing solvent: n-heptane/ethyl acetate (containing 1% pyridine) = 70/30 → 50/50 → 40/60 → 30/70). The crude product obtained by concentrating the fractions containing the target substance was dissolved in n-heptane/ethyl acetate = 1/1 (400 mL) and washed twice with water/DMF = 1/1 (300 mL) and twice with water (300 mL). The organic layer was dried over Na₂SO₄, the drying agent was filtered off, and the filtrate was concentrated. The concentrated residue obtained was purified again through silica gel column chromatography (200 g of DIOL MB 100-40/ 75 manufactured by Fuji Silysia Chemical Ltd., developing solvent: n-heptane/ethyl acetate (containing 1% pyridine) = 70/30 → 60/40 → 50/50 → 40/60). The fractions containing the target substance were concentrated and subjected to azeotropic distillation with toluene and acetonitrile to obtain the title compound amidite d3 (di-tBu-Fmoc-GuNA[t-Bu]-G amidite) (12.62 g, 9.14 mmol (in terms of pure content), containing acetonitrile in an amount of 5.9% by weight, yield was 79%) as a white amorphous solid. FIG. 11 shows a ¹H-NMR spectrum of the obtained amidite d3. Also, data on physical properties of the obtained amidite d3 were as follows: ³¹P-NMR (CDCl₃): δ149.4, 149.9 ppm.

### (Example 5: Stability Test of Amidite)

The amidite a3 (purity was 96.3%) obtained in Example 1, and an amidite a3' (purity was 90.9%) synthesized according to the method described in Patent Document 5 were each dissolved in acetonitrile to prepare a 0.1 M solution.

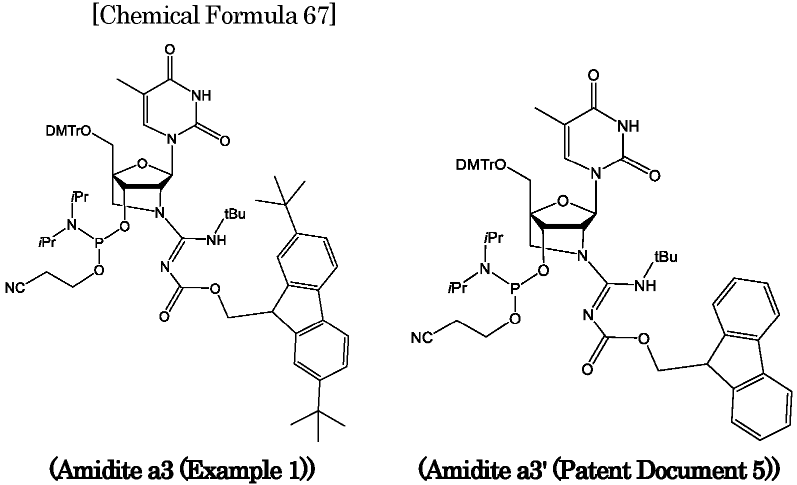

Of the two 0.1 M solutions obtained, the solution containing the amidite a3 of Example 1 was stored in the dark at room temperature for 7 days. On the other hand, the solution containing the amidite a3' of Patent Document 5 was stored in the dark at room temperature for 17 hours.

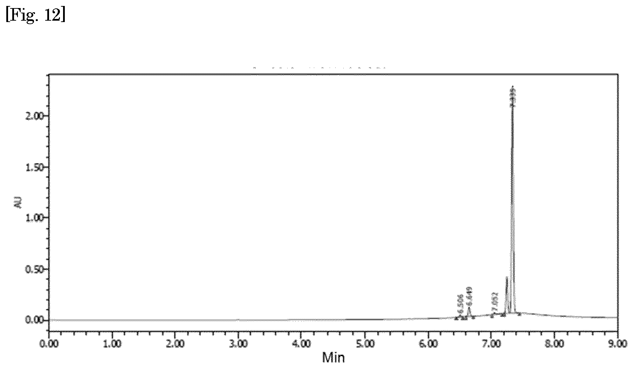

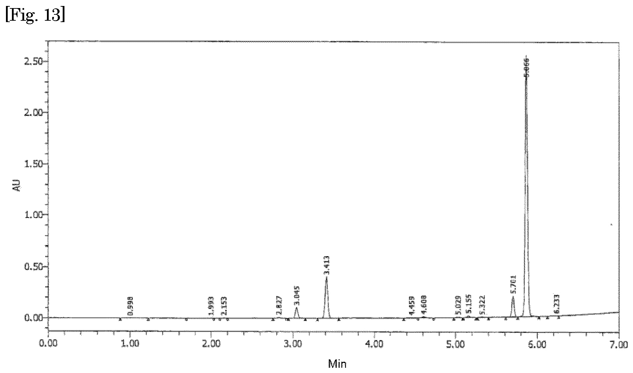

The UHPLC results of the amidites a3 and a3' contained respectively in the stored solutions are shown in Tables 7 and 8, and the UHPLC-MS results of the amidites a3 and a3' contained respectively in the stored solutions are shown in FIGS. 12 and 13.

**[Table 7]**

| UHPLC results for amidite a3 from Example 1 (stored for 7 days) | | | |
|---|---|---|---|
| | Retention time (min) | Area (µVsec) | %Area |
| 1 | 6.506 | 31547 | 0.64 |
| 2 | 6.649 | 170096 | 3.48 |
| 3 | 7.052 | 32605 | 0.67 |
| 4 | 7.335 | 4659890 | 95.21 |

**[Table 8]**

| UHPLC results for amidite a3' from Patent Document 5 (stored for 17 hours) | | | |
|---|---|---|---|
| | Retention time (min) | Area (µVsec | %Area |
| 1 | 0.998 | 1452 | 0.02 |
| 2 | 1.993 | 1071 | 0.02 |
| 3 | 2.153 | 1774 | 0.03 |
| 4 | 2.827 | 17516 | 0.25 |
| 5 | 3.045 | 223760 | 3.17 |
| 6 | 3.413 | 945245 | 13.39 |
| 7 | 4.459 | 9462 | 0.13 |
| 8 | 4.608 | 33474 | 0.47 |
| 9 | 5.029 | 13470 | 0.19 |
| 10 | 5.155 | 38486 | 0.55 |
| 11 | 5.322 | 8453 | 0.12 |
| 12 | 5.701 | 398065 | 5.64 |
| 13 | 5.866 | 5366414 | 76.00 |
| 14 | 6.233 | 2590 | 0.04 |

As is clear from Tables 7 and 8 and FIGS. 12 and 13, the amidite a3 obtained in Example 1, which was stored for 7 days, had fewer split peaks through UHPLC and was more stable than the amidite a3' of Patent Document 5, which was stored for 17 hours, even though the amidite 3 was stored for a longer period of time.

### (Example 6: Compound d1 Synthesis)

The compound d1 used above was synthesized as follows:

### (6-1: Compound e4 Synthesis)

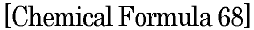

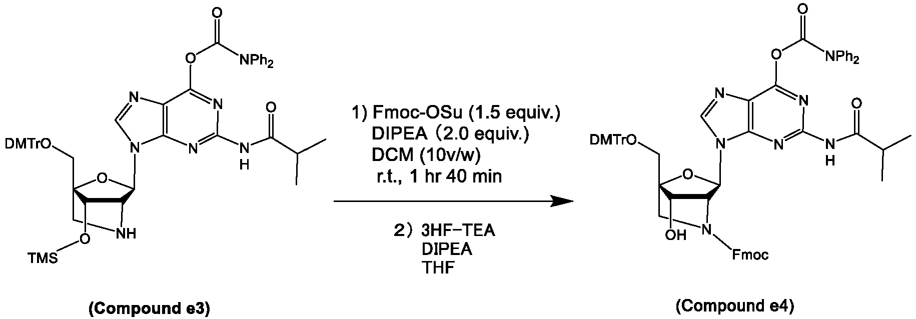

### (1) Fmoc protection

In an argon atmosphere, the compound e3 (1.00 g, 1.07 mmol) described in International Publication No. 2020/100826 was dissolved in dry DCM (10 mL), DIPEA (374 µL, 2.14 mmol) and N-[(9H-fluoren-9-ylmethoxy)carbonyloxy]succinimide (Fmoc-OSu) (542 mg, 1.61 mmol) were added, and the resulting mixture was stirred at room temperature for 1 hour and 40 minutes. After the disappearance of the raw material was confirmed, the reaction was quenched by adding the reaction solution to ethyl acetate/water = 1/1 (100 mL), and the resulting mixture was separated. The organic layer was washed with water (50 mL) and dried over Na₂SO₄. The drying agent was filtered off, and the filtrate was concentrated to obtain a crude product of the Fmoc protected derivative (1.51 g). The crude product of the Fmoc protected derivative was used as it was in the next step.

### (2) TMS deprotection

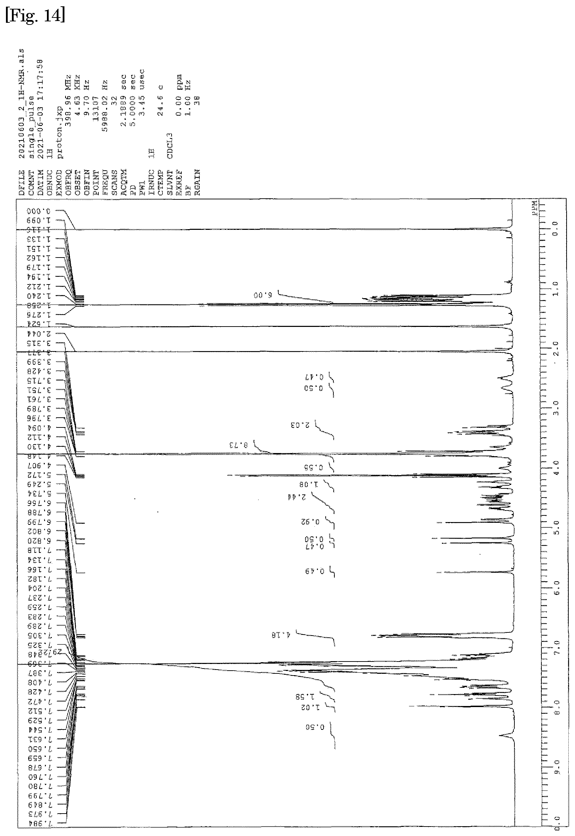

In an argon atmosphere, the crude product of the Fmoc protected derivative (1.51 g, 1.07 mmol (in terms of pure content)) obtained above was dissolved in dry THF (10 mL), and DIPEA (561 µL, 3.21mmol) and triethylamine trihydrofluoride (3 HF-TEA) (174 µL, 1.07 mmol) were added, and the resulting mixture was stirred at room temperature for 35 minutes. After the disappearance of the raw material was confirmed, the reaction was quenched by adding the reaction solution to the saturated aqueous NaCl solution / water = 1/1 (50 mL), and the resulting mixture was separated. The aqueous layer was re-extracted with ethyl acetate (50 mL), and the organic layers were combined together and washed with a saturated aqueous NaCl solution (50 mL), and dried over Na₂SO₄. The drying agent was filtered off, and the filtrate was concentrated. The concentrated residue was purified through silica gel column chromatography (SI 50, SIZE 60, manufactured by Fuji Silysia Chemical Ltd., developing solvent: n-heptane/ethyl acetate = 90/10 → 50/50 → 10/90). Fractions containing the title target substance were concentrated to obtain the compound e4 (0.89 g, 0.821 mmol, yield was 77% in two steps) as a pale yellow amorphous solid. FIG. 14 shows a ¹H-NMR spectrum of the compound e4 obtained.

### (6-2: Compound e5 Synthesis)

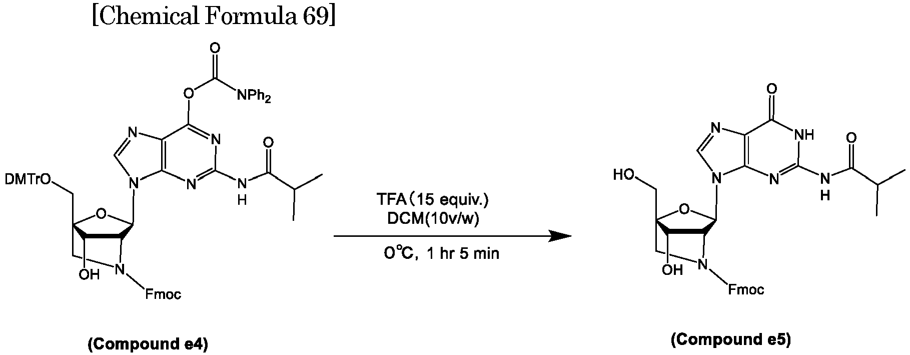

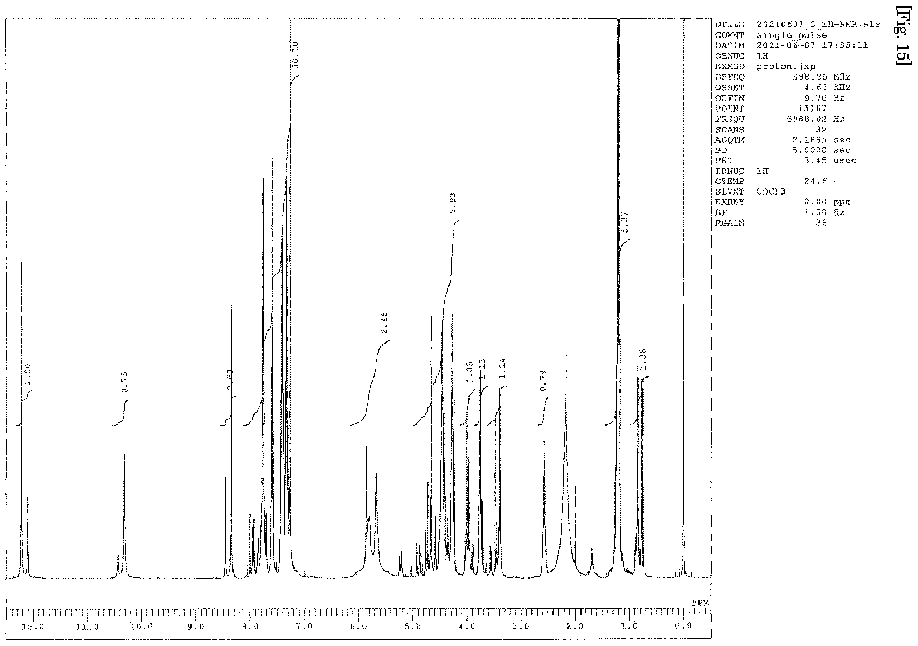

In an argon atmosphere, the compound e4 (0.89 g, 0.821 mmol) obtained above was dissolved in dry DCM (9.0 mL) and cooled to 5°C in an ice bath. TFA (949 µL, 12.3 mmol) was added, and the resulting mixture was stirred under ice cooling for 1 hour and 5 minutes. The formation of the target substance was confirmed, and then the reaction solution was concentrated. CHCl₃ (50 mL) and a saturated aqueous NaHCO₃ solution (50 mL) were added to the residue, and the resulting mixture was separated. The organic layers were washed with a saturated aqueous NaCl solution (50 mL) and dried over Na₂SO₄. The drying agent was filtered off, and the filtrate was concentrated. The concentrated residue was purified through silica gel column chromatography (SI 50, SIZE 60, manufactured by Fuji Silysia Chemical Ltd., developing solvent: CHCl₃/MeOH = 100/0 → 90/10). Fractions containing the title target substance were concentrated to obtain a crude product (453 mg) of the compound e5 as a white amorphous solid. The compound e5 was used directly in the next step without further purification. FIG. 15 shows a ¹H-NMR spectrum of the compound e5 obtained.

### (6-3: Compound e6 Synthesis)

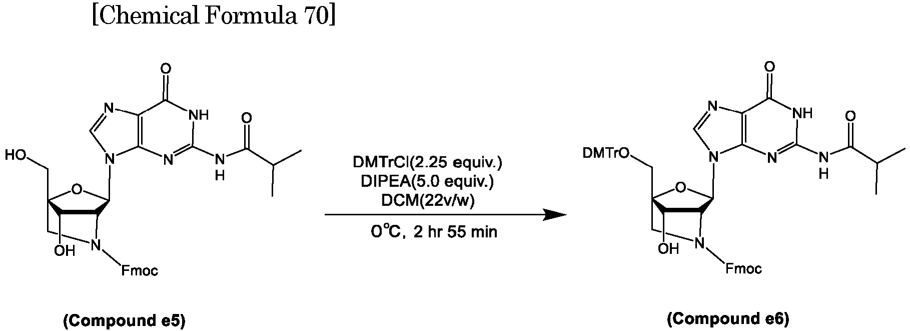

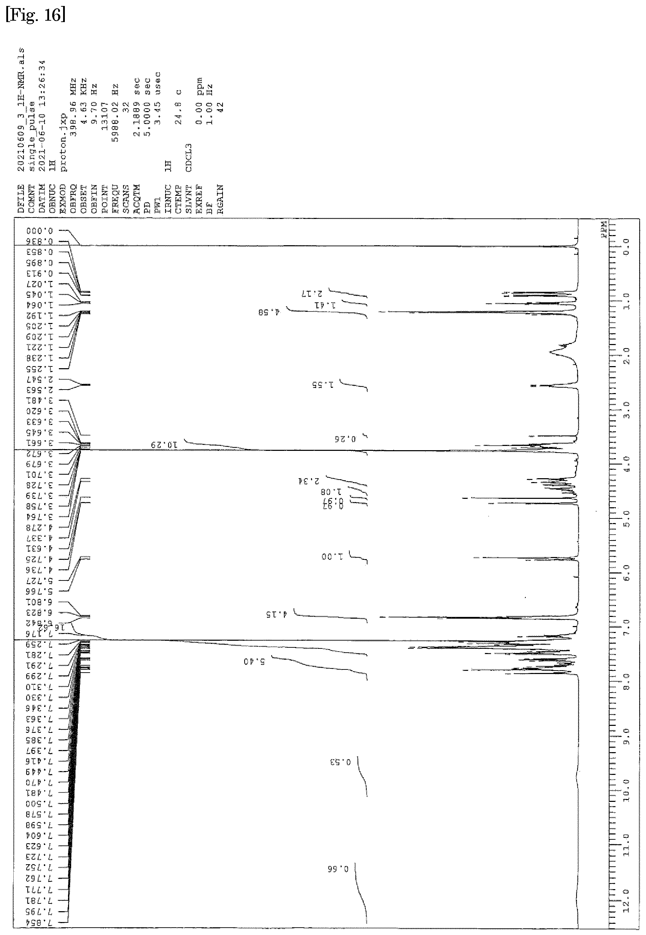

In an argon atmosphere, the compound e5 (450 mg, 0.767 mmol (in terms of pure content)) obtained above was dissolved in dry DCM (10 mL), DIPEA (670 µL, 3.84 mmol) was added, and the resulting mixture was cooled to 5°C in an ice bath. DMTrCl (389 mg, 1.15 mmol) was added, and the resulting mixture was stirred for 1 hour and 15 minutes while the temperature thereof was increased to room temperature. The residue of the raw material was confirmed, and thus DMTrCl (194 mg, 0.575 mmol) was added, and the resulting mixture was further stirred at room temperature for 1 hour and 40 minutes. After the disappearance of the raw material was confirmed, the reaction was quenched by pouring the reaction solution into a mixture (50 mL) of ice and the saturated aqueous NaHCO₃ solution, CHCl₃ (50 mL) was added, and the resulting mixture was separated. The aqueous layer was extracted with CHCl₃ (50 mL), and the combined organic layers were dried over Na₂SO₄. The drying agent was filtered off, and the filtrate was concentrated. The concentrated residue was purified through silica gel column chromatography (SI 50, SIZE 60, manufactured by Fuji Silysia Chemical Ltd., developing solvent: CHCl₃/MeOH = 100/0 → 90/10). Fractions containing the title target substance were concentrated to obtain the compound e6 (556 mg, 0.625 mmol, yield was 76% in two steps) as a pale yellow amorphous solid. FIG. 16 shows a ¹H-NMR spectrum of the compound e6 obtained.

### (6-4: Compound d1 Synthesis)

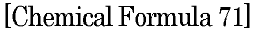

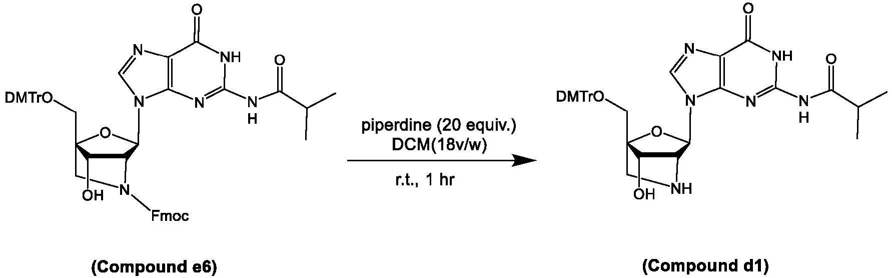

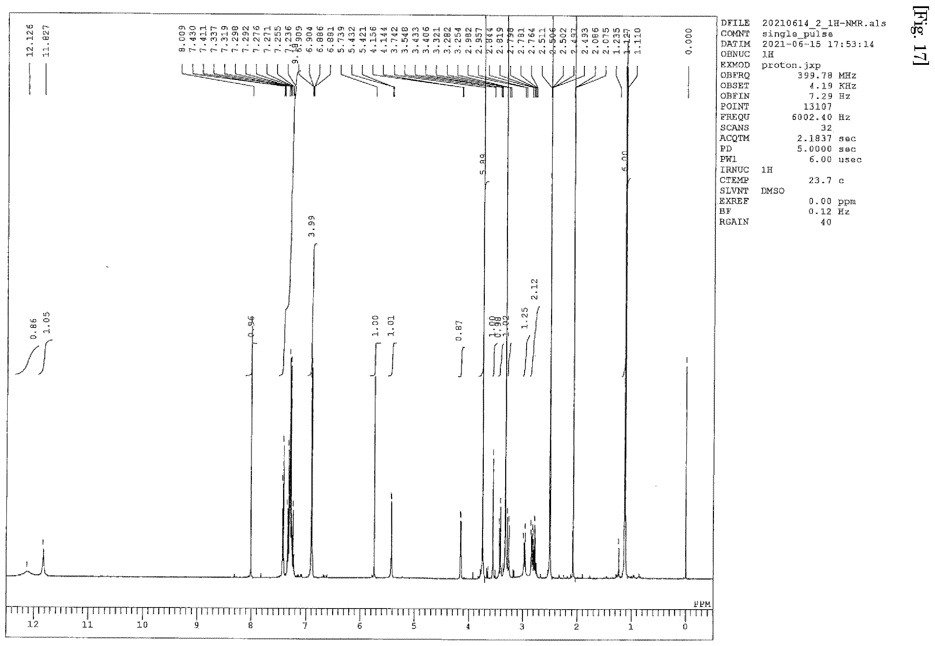

In an argon atmosphere, the compound e6 (550 mg, 0.619 mmol) obtained above was dissolved in dry DCM (10 mL), and piperdine (1.23 mL, 12.4 mmol) was added, and the resulting mixture was stirred at room temperature for 1 hour. The disappearance of the raw material was confirmed, and then the reaction solution was concentrated. The concentrated residue was suspended twice and washed twice with IPE, and then purified through silica gel column chromatography (SI 50, SIZE 60, manufactured by Fuji Silysia Chemical Ltd., developing solvent: CHCl₃/MeOH (containing 1% TEA) = 100/0 → 90/10). Fractions containing the title target substance were concentrated to obtain the compound d1 (340 mg, 0.493 mmol, yield was 80%) as a white amorphous solid. FIG. 17 shows a ¹H-NMR spectrum of the compound d1 obtained.

### (Example 7: Guanidinylation Reagent (di-tBu-Fmoc-protected derivative) Synthesis (A))

### (7-1) Synthesis of Compound A2 (N-(tert-butylcarbamothioyl)benzamide)

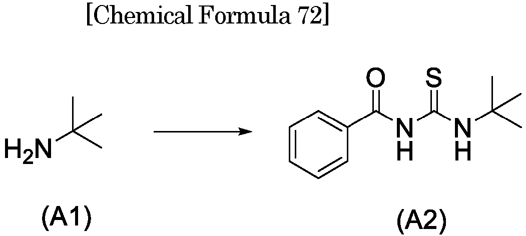

Tert-butylamine (A1) (9.37 mL) was added to dehydrated acetonitrile (26.0 mL), and the resulting mixture was stirred in an ice bath. Benzoyl isothiocyanate (13.23 g) was added dropwise over 8 minutes while the internal temperature was maintained at 35°C or less. The resulting mixture was stirred in an ice bath for 75 minutes, the resulting solid was then collected through filtration and washed with cold acetonitrile (6 mL × 2). The resulting solid was dried under reduced pressure to obtain the title compound A2 (N-(tert-butylcarbamothioyl)benzamide) as a white solid (11.6 g, yield was 61%). Table 9 shows data on physical properties of the compound A2 obtained.

**[Table 9]**

| Data on Physical Data of the obtained Compound A2 |
|---|
| LC/MS: Measuring condition A, Retention time=2.26 min |
| LC/MS(ESI+) m/z; 237.3[M+H]⁺ |
| LC purity 100% |
| ¹H-NMR (CDCl₃,400MHz) δ: 1.61 (9H, s), 7.51 (2H, dd, *J* = 8.4, 7.3 Hz), 7.62 (1H, t, *J* = 7.3 Hz), 7.82 (2H, d, *J* = 8.4 Hz), 8.78 (1H, brs), 10.91 (1H, brs). |

### (7-2) Synthesis of Compound A4 (1-(tert-butyl)-2-methyl isothiouronium iodide)

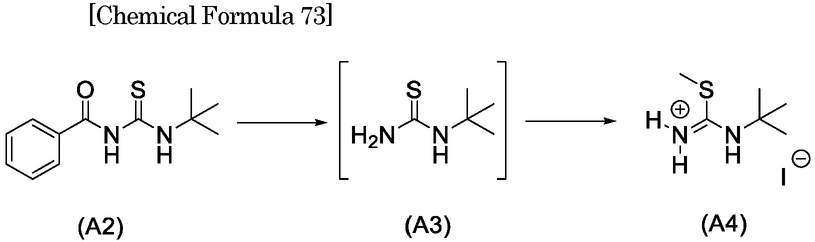

The compound A2 (N-(tert-butylcarbamothioyl)benzamide) (1.00 g) obtained above was suspended in methanol (5.0 mL), and the temperature thereof was then increased to 60°C. A28% by weight sodium methoxide-methanol solution (21 µL) was added at the same temperature, the resulting mixture was stirred for 40 minutes and cooled to room temperature over 1 hour and 50 minutes. Then, methyl iodide (395 µL) was added, and the resulting mixture was stirred at room temperature for 3 hours. The reaction mixture was concentrated to a mass of 1.76 g, ethyl acetate (5.0 mL) was then added, and the resulting mixture was stirred. The resulting solid was collected through filtration and washed with ethyl acetate. The resulting solid was dried under reduced pressure to obtain the title compound A4 (1-(tert-butyl)-2-methyl isothiouronium iodide) as a white solid (1.04 g, yield was 89%). Table 10 shows data on physical properties of the compound A4 obtained.

**[Table 10]**

| Data on Physical Data of the obtained Compound A4 |
|---|
| LC/MS Measuring condition A, Retention time =0.22 min |
| LC/MS(ESI+) m/z; 147.2[M+H]⁺ (Value of the cationic part) |
| ¹H-NMR(CDCl₃,400MHz)δ:1.52(9H,s),2.81(3H,s),7.49(1H,brs),8.84(1H,brs),8.98(1H,brs). |

### (7-3) Synthesis of Compound A7 ((2,7-di-tert-butyl-9H-fluoren-9-yl)methanol)

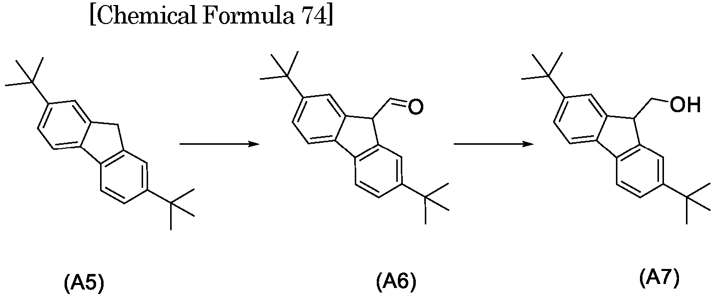

Potassium t-butoxide (10.9 g) was placed in a reaction vessel, which was purged with nitrogen. Dehydrated THF (81 mL) was added thereto, the resulting mixture was stirred for 5 minutes and cooled under ice cooling until the internal temperature reached 1°C. Then, 2,7-di-tert-butyl-9H-fluorene (A5) (13.5 g) dissolved in dehydrated THF (34 mL) was added dropwise over 11 minutes while the internal temperature was maintained at 3°C or less (rinsed with 7.0 mL of dehydrated THF), and the resulting mixture was stirred under ice cooling for 22 minutes. Ethyl formate (7.81 mL) was added dropwise over 21 minutes while the internal temperature was maintained at 5°C or less. Thereafter, the resulting mixture was stirred under ice cooling for 23 minutes, the ice bath was replaced with a water bath, and the mixture was stirred for 28 minutes. Then, 2 M hydrochloric acid (49 mL) was added over 3 minutes, the resulting mixture was stirred vigorously for 9 minutes. When stirring was stopped, the mixture was cleanly separated into two layers within 2 minutes. The lower layer (42 mL) was removed, a solution of sodium chloride (8.1 g) in water (80 mL) was added, and the resulting mixture was vigorously stirred for 36 minutes. When stirring was stopped, the mixture was cleanly separated into two layers within 1 minute. The lower layer (110 mL) was removed, the upper layer was then concentrated under reduced pressure, and most of THF was distilled off. Isopropyl alcohol (81 mL) and water (27 mL) were then added to obtain an orange transparent solution containing the compound A6.

A 1 M aqueous solution of sodium hydroxide (4.85 mL) was diluted with water (4.85 mL), and sodium borohydride (1.83 g) was dissolved therein. This solution was added dropwise to the orange solution containing the compound A6 obtained above over 10 minutes while the internal temperature was maintained at 28°C or less in an ice bath. Thereafter, the mixture was stirred in a water bath for 2 hours, acetone (12.8 mL) was added over 3 minutes in an ice bath, and the resulting mixture was stirred in a water bath for 14 minutes. Then, 2 M hydrochloric acid (29 mL) was added, the reaction mixture was stirred vigorously for 30 minutes and concentrated under reduced pressure, and most of isopropyl alcohol was distilled off. Toluene (135 mL) was added thereto, and the reaction mixture was stirred vigorously for 16 minutes. When stirring was stopped, the mixture was cleanly separated into two layers within 2 minutes. Then, the reaction mixture was allowed to stand overnight at room temperature in a bilayer state.

The lower layer (60 mL) was removed, the upper layer was then concentrated under reduced pressure, and most of toluene was distilled off. Thereafter, when toluene (100 mL) was added, the reaction mixture turned into an orange solution. (+)-10-camphorsulfonic acid (2.25 g) was added to this solution, the resulting mixture was stirred at an internal temperature of 90°C to 105°C for 2 hours, the warm bath was removed, and the mixture was further stirred for 30 minutes. A solution of potassium hydrogen carbonate (1.46 g) in water (40 mL) was added thereto, and the resulting mixture was vigorously stirred for 20 minutes. When stirring was stopped, the mixture was cleanly separated into two layers within 3 minutes. The lower layer was removed, water (40 mL) was added to the upper layer, and the resulting mixture was stirred vigorously for 10 minutes. When stirring was stopped, the mixture was cleanly separated into two layers within 4 minutes. The lower layer was removed, and the upper layer was concentrated under reduced pressure until the mass reached 26.0 g. n-heptane (135 mL) was added to the residue, and the resulting mixture was stirred to obtain amber transparent liquid. The solution was allowed to stand overnight at room temperature.

The next morning, the solution was concentrated under reduced pressure until the mass reached 16.7 g. n-heptane (68 mL) was added to the residue, and the resulting mixture was heated to 65°C to obtain a homogeneous solution. The solution was stirred for 18 minutes under natural cooling, then stirred in an ice bath for 2 hours, the resulting solid was then collected through filtration and washed with cold n-heptane (once with 14 mL and once with 7 mL). The resulting solid was dried under reduced pressure to obtain the title compound A7 ((2,7-di-tert-butyl-9H-fluoren-9-yl)methanol) as a white solid (13.6 g, yield was 90%). Table 11 shows data on physical properties of the compound A7 obtained.

**[Table 11]**

| Data on Physical Data of the obtained Compound A7 |
|---|
| LC/MS: Measuring condition A, Retention time =3.00 min |
| LC/MS(ESI+) m/z; 309.3[M+H]⁺ |
| LC purity 99.7% |
| ¹H-NMR(CDCl₃,400MHz)δ:1.38(18H,s),4.08(3H,s),7.42(2H,dd,*J*=7.9,1.8Hz),7.61(2H,d,*J* =1.8Hz),7.65(2H,d,*J*=7.9Hz). |

### (7-4) Synthesis of Compound A9 ((tert-butylamino)(methylthio)methylene)carbamic acid (2,7-di-tert-butyl-9H-fluoren-9-yl)methyl)

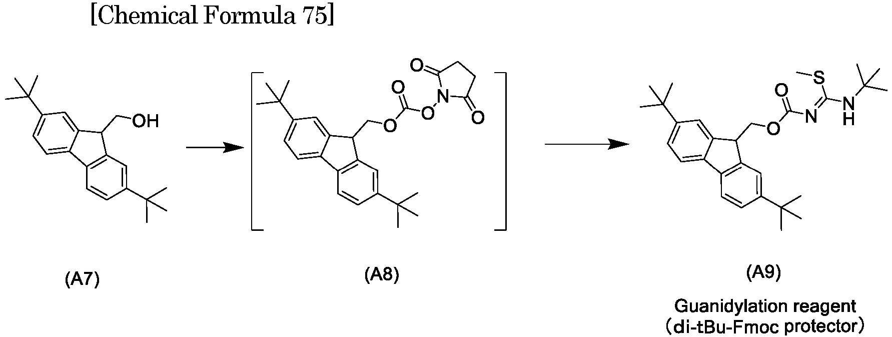

The compound A7 ((2,7-di-tert-butyl-9H-fluoren-9-yl)methanol) (1.36 g) obtained above and di(N-succinimi dyl)carbonate (1.36 g) were placed in a reaction vessel, dichloromethane (6.8 mL) was added, and pyridine (534 µL) was added at once while the mixture was stirred at room temperature. The mixture was stirred at room temperature for 3 hours, water (6.8 mL) was added at once, and the resulting mixture was vigorously stirred for 10 minutes.

To the resulting bilayer reaction mixture, a solution of the compound A4 (1-(tert-butyl)-2-methyl isothiouronium iodide) (1.21 g) obtained above in dichloromethane (2.7 mL) was added at once while the mixture was stirred at room temperature. Then, a solution of potassium carbonate (1.04 g) in water (2.7 mL) was added, and the resulting mixture was stirred for 2 hours. The reaction mixture was allowed to stand for three nights in a bilayer state.

The lower layer was taken out and the reaction mixture was concentrated under reduced pressure until the mass reached 3.48 g. Then, n-heptane (14 mL) was added to the residue, and the reaction mixture was concentrated under reduced pressure until the mass reached 3.06 g. When n-heptane (7.0 mL) was added to the residue and the resulting mixture was stirred at 45°C, a slightly cloudy solution was obtained. The solution was stirred for 40 minutes under natural cooling, then stirred in an ice bath for 60 minutes, the resulting solid was then collected through filtration and washed with heptane at about 0°C (2 mL × 2). The resulting solid was dried under reduced pressure to obtain the title compound A9 ((tert-butylamino)(methylthio)methylene)carbamic acid (2,7-di-tert-butyl-9H-fluoren-9-yl)methyl) (guanidinylation reagent (di-tBu-Fmoc protected derivative) as a white solid (1.85 g, yield was 87%). Table 12 shows data on physical properties of the compound A9 obtained.

**[Table 12]**

| Data on Physical Data of the obtained Compound A9 |
|---|
| LC/MS Measuring condition A, Retention time =3.63 min |
| LC/MS(ESI+) m/z; 481.6[M+H]⁺ |
| LC purity 98.7% |
| ¹H-NMR(CDCl₃,400MHz)δ:1.36(18H,s),1.48(9H,s),2.55(3H,s),4.26-4.35(3H,m),7.40(2H,d d,*J*=8.1,2.0Hz),7.63(2H,d,*J*=8.1Hz),7.70(2H,d,*J*=2.0Hz),9.97(1H,brs). |

### (Example 8: Guanidinylation Reagent (di-tBu-Fmoc-protected derivative) Synthesis (B))

### (8-1) Synthesis of Compound B2

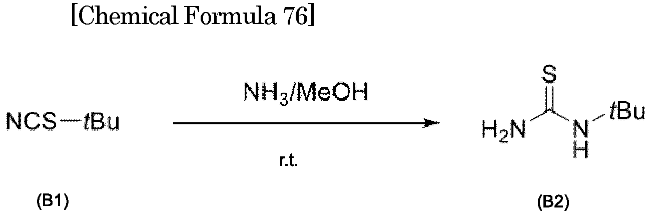

The compound B1 (t-butyl isothiocyanate) (305 g, 2.65 mol) was prepared, and 7 M ammonia/MeOH (1.51 L, 10.6 mol, 4.0 equivalents) was added while the mixture was cooled in an ice bath. The reaction solution was removed from the ice bath, the temperature of the reaction solution was increased to room temperature, and the reaction solution was stirred for 4 days.

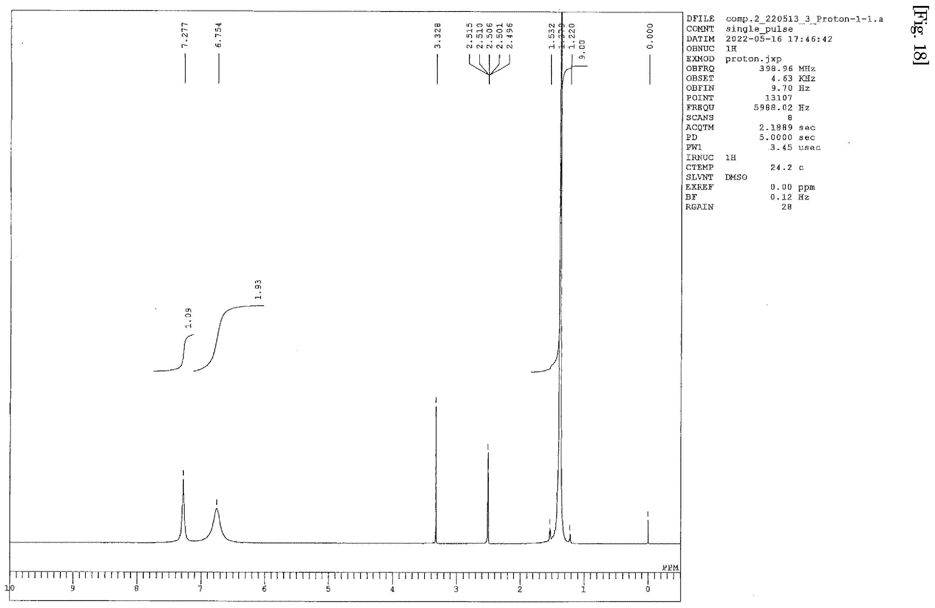

The disappearance of the raw material was confirmed by ¹H-NMR, and then the reaction solution was concentrated using a rotary evaporator. Methyl t-butyl ether (MTBE) (600 mL) was added to the concentrated residue, and the resulting mixture was stirred at room temperature. The suspension was filtered, the residue was washed with MTBE (400 mL), dried at 40°C under reduced pressure to obtain the title compound B2 (268 g, 2.03 mol, yield was 77%) as a white solid. FIG. 18 shows a ¹H-NMR spectrum of the compound B2 obtained.

### (8-2) Synthesis of Compound B3

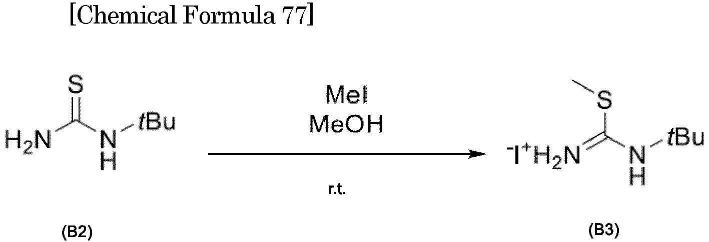

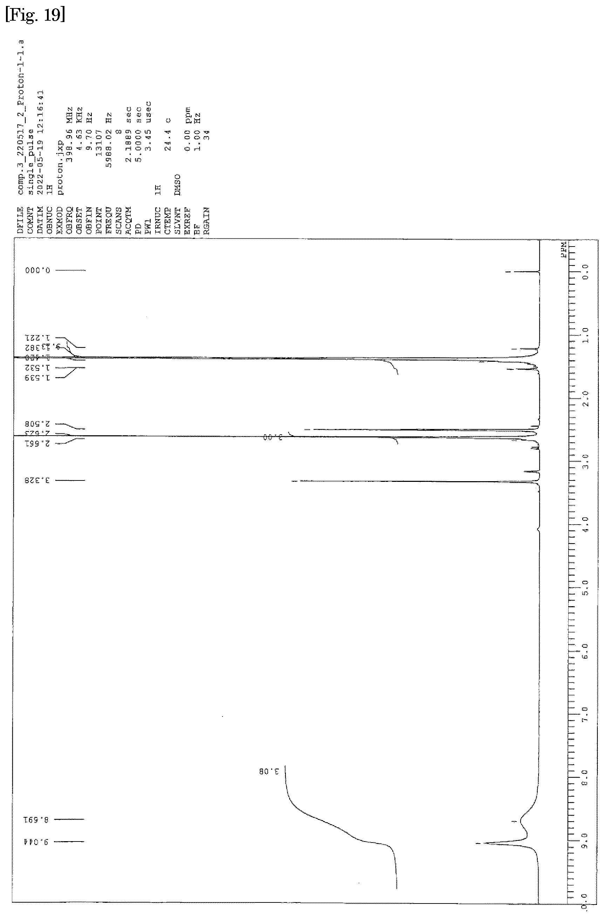

The compound B2 (267 g, 2.02 mol) obtained above was charged into MeOH (1.6 L) and cooled in an ice bath. MeI (430 g, 3.03 mol, 1.5 equivalents) was added, the reaction solution was removed from the ice bath, the temperature of the reaction solution was allowed to increase naturally to room temperature, and the reaction solution was stirred for 24 hours and 15 minutes. The disappearance of the raw material was confirmed by ¹H-NMR, and then the reaction solution was concentrated using a rotary evaporator. The concentrated residue was dried under reduced pressure to obtain the compound B3 (554 g, 2.02 mol, quantitative) as a yellow solid. FIG. 19 shows a ¹H-NMR spectrum of the compound B3 obtained.

### (8-3) Synthesis of Compound B5

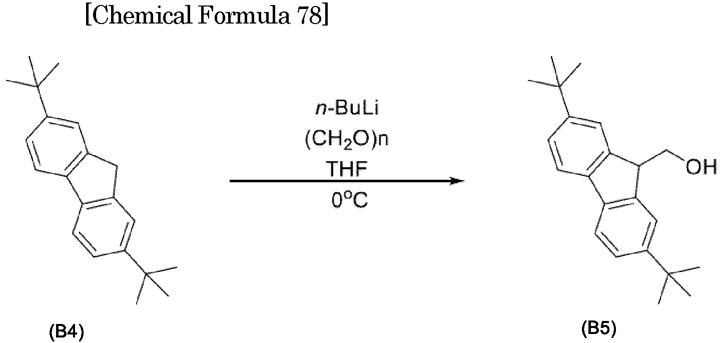

Under argon stream, the compound B4 (250 g, 898 mmol) was dissolved in dry THF (2.50 L) and cooled to an internal temperature of -2°C in an ice-salt bath. n-BuLi in n-hexane (1.6 M, 673 mL, 1.08 mol) was added dropwise over 62 minutes, and the resulting mixture was stirred for 40 minutes. (CH₂O)ₙ (40.4 g, 1.35 mol) was added, and the resulting mixture was stirred in an ice-salt bath for 30 minutes. The progress of the reaction was confirmed by TLC, and a saturated aqueous NaHCO₃ solution (1.00 L) was added dropwise over 20 minutes while the mixture was kept in the ice-salt bath. The reaction solution was extracted with ethyl acetate (1.00 L), and the aqueous layer was re-extracted with ethyl acetate (1.00 L). The organic layers were combined together, washed with a saturated aqueous NaCl solution (1.00 L), and dried over Na₂SO₄. The solid was filtered off, and the filtrate was concentrated.

The compound B4 (250 g, 898 mmol) was used again, and the same procedure as above was carried out to obtain a concentrated residue.

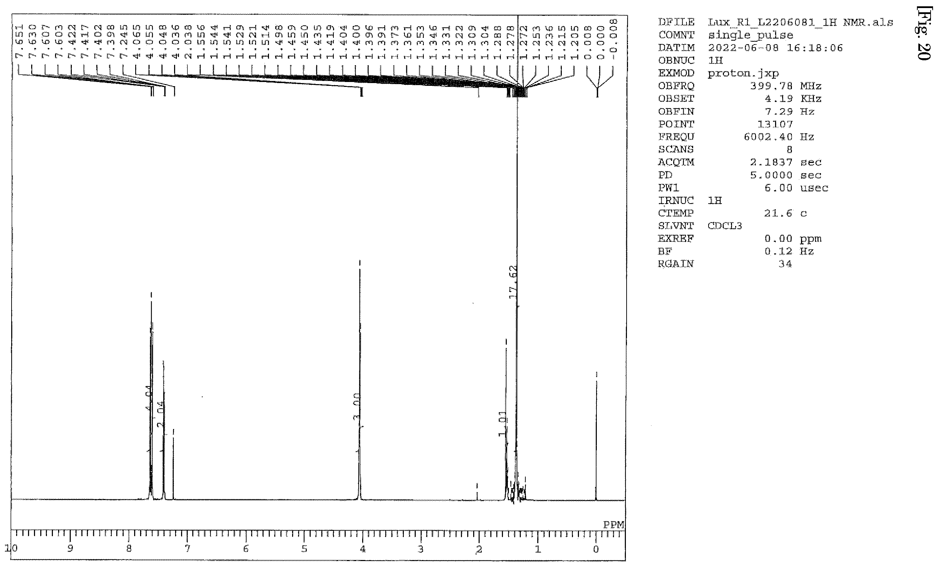

These concentrated residues were combined together and purified through silica gel column chromatography (5.5 kg of neutral silica gel, developing solvent: n-heptane/ethyl acetate = 100/0 → 95/5 → 90/10 → 50/50) to obtain the title compound B5 (383 g, 1.24 mol, yield was 69%) as a yellowish white solid. FIG. 20 is a ¹H-NMR spectrum of the compound B5 obtained.

### (8-4) Synthesis of Compound B6

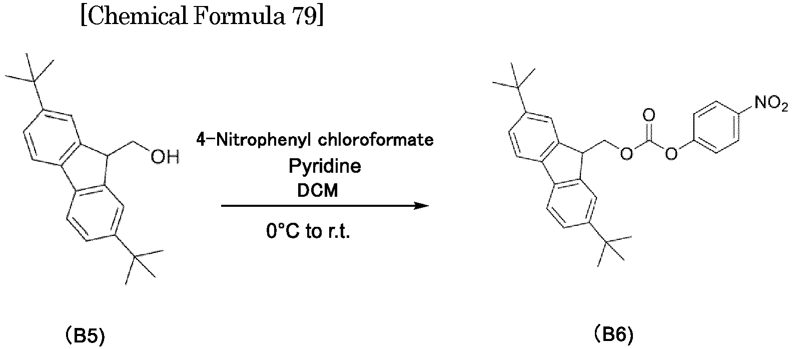

Under argon stream, the compound B5 (380 g, 1.23 mol) obtained above was dissolved in dry DCM (3.80 L) and cooled to 4°C in an ice bath. Pyridine (200 mL, 2.47 mol) was added, and the resulting mixture was stirred for 8 minutes. Then, 4-nitrophenyl chloroformate (372 g, 1.85 mol) dissolved in dry DCM (1.52 L) was added dropwise over 1 hour, and the resulting mixture was stirred at room temperature for 2 hours and 15 minutes. After the disappearance of the raw material was confirmed through UHPLC, the reaction solution was added to water (3.00 L), and the resulting mixture was extracted twice with DCM (3.00 L). The organic layers were combined together, washed with a saturated aqueous NaCl solution (3.00 L), and dried over Na₂SO₄. The solid was filtered off, and the filtrate was concentrated. The concentrated residue was dissolved in DCM (1.20 L), and n-heptane (3.80 L) was added dropwise. The resulting solid was filtered off, and the filtrate was concentrated. The filtered solid was referred to as solid (1), and the concentrated residue of the filtrate was referred to as residue (1).

The solid (1) was dissolved in DCM (2.00 L), and n-heptane (2.50 L) was added dropwise. The resulting solid was filtered off, and the filtrate was concentrated. The concentrated residue of the filtrate was referred to as residue (2).

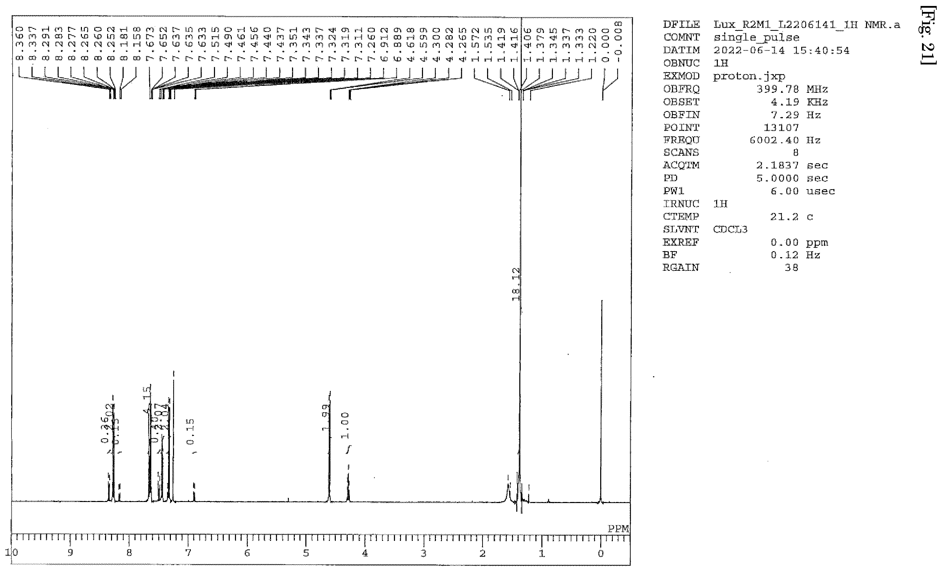

The resulting residues (1) and (2) obtained above were combined and washed through suspension in n-heptane (2.00 L), and the resulting solid was collected through filtration to obtain the title compound B6 (592 g, 1.18 mol, yield was 96%) as a yellow solid. FIG. 21 shows a ¹H-NMR spectrum of the compound B6 obtained.

### (8-5) Synthesis of Compound B7 (Guanidinylation Reagent (di-tBu-Fmoc-protected derivative))

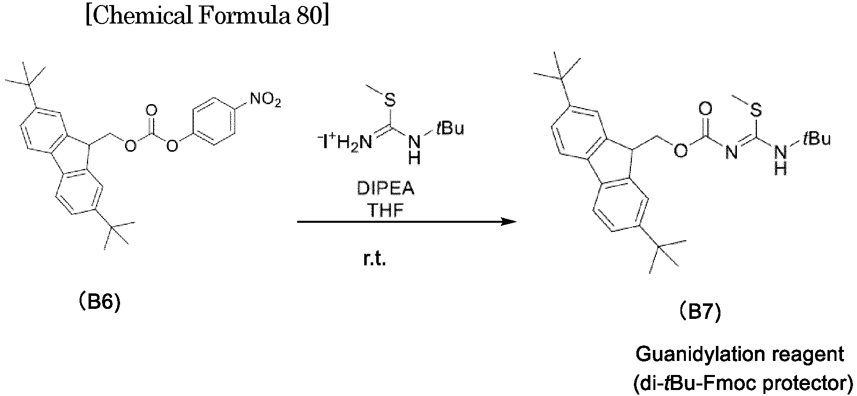

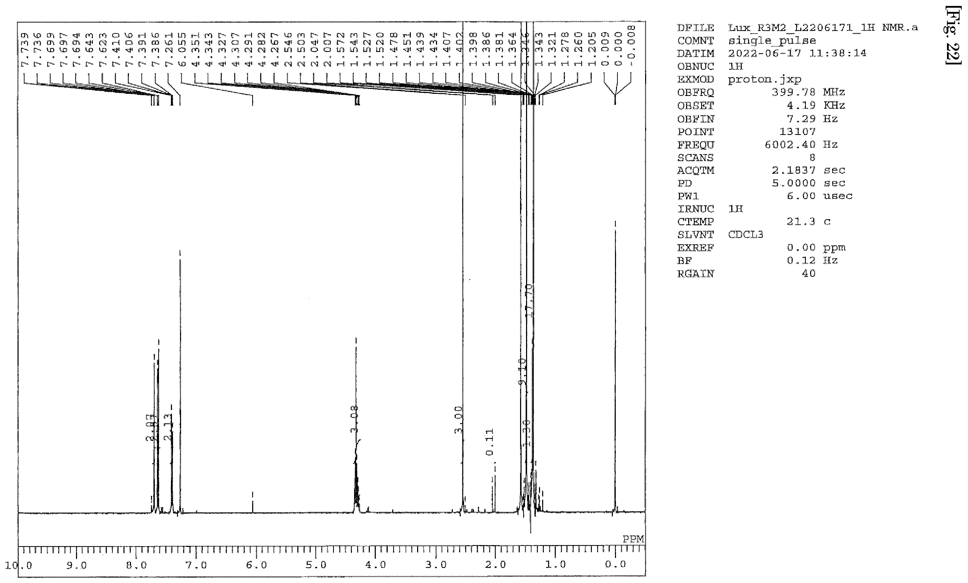

The compound B6 (590 g, 1.28 mol) obtained above was dissolved in THF (4.00 L). The compound B3 (484 g, 1.77 mol) obtained above was added, and the resulting mixture was washed down with THF (2.30 L). N,N-diisopropylethylamine (DIPEA) (1.03 L, 5.89 mol) was added dropwise over 25 minutes, and the resulting mixture was stirred at room temperature for 17 hours and 15 minutes. After the disappearance of the raw material was confirmed through UHPLC, the reaction solution was added to a saturated aqueous NaHCO₃ solution (6.00 L), and the resulting mixture was separated. The aqueous layer was re-extracted with ethyl acetate (6.00 L), and the organic layers were combined together and washed with a saturated aqueous NaCl solution (3.00 L), and dried over Na₂SO₄. The solid was then filtered off, and the filtrate was concentrated. The concentrated residue was purified through silica gel column chromatography (10 kg of amino silica gel NH-DM 1020 manufactured by Fuji Silysia Chemical Ltd., developing solvent: n-heptane/ethyl acetate = 100/0 → 90/10 → 80/20). As a result, the title compound B7 (479.85 g, 1.03 mol, yield was 80%), which is the guanidinylation reagent (di-tBu-Fmoc-protected derivative), was obtained. FIG. 22 shows a ¹H-NMR spectrum of the compound B7 obtained.

### Industrial Applicability

The present invention is useful, for example, as a material for nucleic acid drugs used in antisense therapies, antigene therapies, aptamer-based therapies, siRNA-based therapies, and the like, which are expected to be novel methods for treating or preventing a disease. Furthermore, the guanidinylation reagent according to the present invention can be used not only for production of the modified nucleoside of the present invention having a guanidino structure in a bridged part, but also for the production of various compounds having a guanidinyl structure (-N-C(=N)-N-).

## Claims

1. A compound represented by a formula (I) below or a salt thereof: 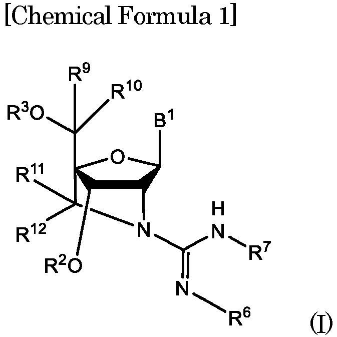 (where in the formula (I),
B¹ is
(a) a purin-9-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms; or
(b) a 1,2-dihydropyrimidin-1-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R² and R³ are each independently
(a) a hydrogen atom;
(b) a hydroxy group protecting group for nucleic acid synthesis;
(c) an alkyl group having 1 to 7 carbon atoms that may form a branch or a ring;
(d) an alkenyl group having 2 to 7 carbon atoms that may form a branch or a ring;
(e) an aryl group having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(f) a heteroaryl group having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(g) an aralkyl group with an aryl moiety having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(h) a heteroaralkyl group with a heteroaryl moiety having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(i) an acyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(j) a silyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(k) a phosphate group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(l) a phosphate group protected by a protecting group for nucleic acid synthesis; or
(m)-P(R⁴)R⁵,
(where
R⁴ and R⁵ are each independently
(a) a hydroxy group;
(b) a hydroxy group protected by a protecting group for nucleic acid synthesis;
(c) a mercapto group;
(d) a mercapto group protected by a protecting group for nucleic acid synthesis;
(e) an amino group;
(f) an alkoxy group having 1 to 5 carbon atoms;
(g) an alkylthio group having 1 to 5 carbon atoms;
(h) a cyanoalkoxy group having 1 to 6 carbon atoms;
(i) a dialkylamino group having alkyl groups that each have 1 to 6 carbon atoms and may be the same or different; or
(j) a cyclic alkylamino group having 3 to 6 carbon atoms; or
R⁴ and R⁵ together constitute a formula below: 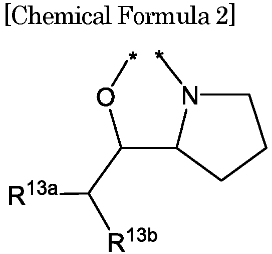
(where R^{13a} and R^{13b} are each independently a hydrogen atom, an alkyl group having 1 to 6 carbon atoms that may be branched, an aryl group having 6 to 12 carbon atoms, a nitro group, a halogen atom, a cyano group, Si(R^{a})₃ (where R^{a} is an alkyl group having 1 to 6 carbon atoms or an aryl group having 6 to 12 carbon atoms), a sulfonyl group, SO₂Ph, or SiMePh₂, and * is a bonding site); or 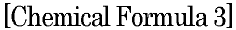 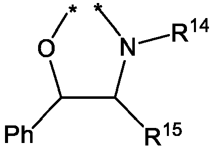
(where R¹⁴ and R¹⁵ are each independently a hydrogen atom, an alkyl group having 1 to 3 carbon atoms that may be branched, or a phenyl group, and * is a bonding site);
R⁶ is a group represented by a formula below: 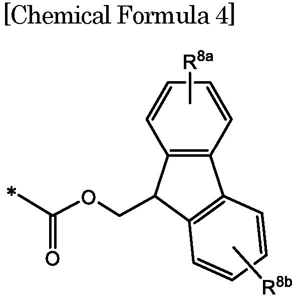 (where
R^{8a} and R^{8b} are each independently
(a) an alkyl group having 1 to 9 carbon atoms that may form a branch or a ring;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site); and
R⁷ is
(a) an alkyl group having 1 to 8 carbon atoms that may form a branch or a ring and may have undergone substitution by an alkoxy group having 1 to 8 carbon atoms that may form a branch or a ring;
(b) a cyanoalkoxy group having 1 to 8 carbon atoms;
(c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
(d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
(e) a group represented by a formula below: 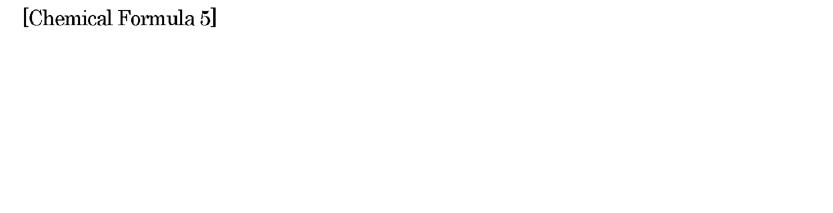 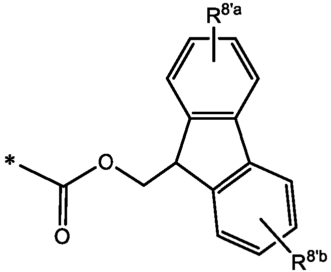 (where
R^{8'a} and R^{8'b} are each independently
(a) an alkyl group having 1 to 9 carbon atoms that may be branched;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site); or
R⁶ is:
(a) an alkyl group having 1 to 8 carbon atoms that may form a branch or a ring and may have undergone substitution by an alkoxy group having 1 to 8 carbon atoms that may form a branch or a ring;
(b) a cyanoalkoxy group having 1 to 8 carbon atoms;
(c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
(d) a heteroaralkyl group with a heteroaryl moiety having 3 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
(e) a group represented by a formula below: 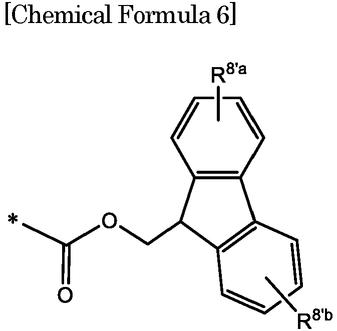 (where
R^{8'a} and R^{8'b} are each independently
(a) an alkyl group having 1 to 9 carbon atoms that may be branched;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, linear alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site); and
R⁷ is a group represented by a formula below: 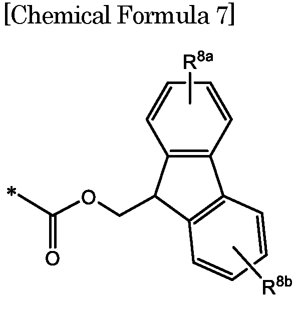 (where
R^{8a} and R^{8b} are each independently
(a) an alkyl group having 1 to 9 carbon atoms that may be branched;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site); and
R⁹ and R¹⁰ are each independently a hydrogen atom or an alkyl group having 1 to 6 carbon atoms that may form a branch or a ring, or R⁹ and R¹⁰ together constitute - (CH₂)ₘ- (where m is an integer from 2 to 7), and
R¹¹ and R¹² are each independently a hydrogen atom or an alkyl group having 1 to 6 carbon atoms that may form a branch or a ring, or
R¹¹ and R¹² together constitute an oxygen atom part (=O) of a carbonyl group, or
R¹¹ and R¹² together constitute -(CH₂)ₙ- (where n is an integer from 2 to 7).

2. The compound or salt thereof according to claim 1,
wherein in the formula (I), R⁶ is a group represented by a formula below: 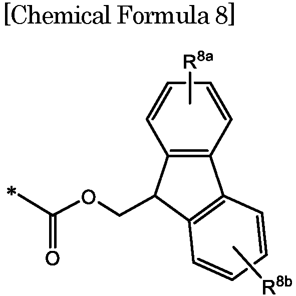 (where
R^{8a} and R^{8b} are each independently
(a) an alkyl group having 1 to 9 carbon atoms that may be branched;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site); and
R⁷ is
(a) an alkyl group having 1 to 8 carbon atoms that may form a branch or a ring and may have undergone substitution by an alkoxy group having 1 to 8 carbon atoms that may form a branch or a ring;
(b) a cyanoalkoxy group having 1 to 8 carbon atoms;
(c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
(d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
(e) a group represented by a formula below: 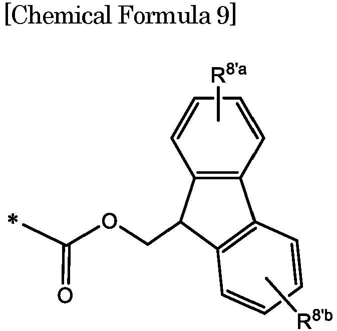 (where
R^{8'a} and R^{8'b} are each independently
(a) an alkyl group having 1 to 9 carbon atoms that may be branched;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site).

3. The compound or salt thereof according to claim 2,
wherein the formula (I) is represented by a formula (I') below:

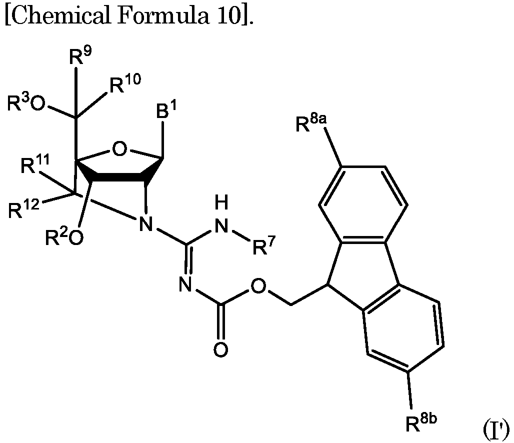

4. The compound or salt thereof according to claim 2,
wherein R⁷ in the formula (I) is an alkyl group having 1 to 8 carbon atoms that may form a branch or a ring.

5. The compound or salt thereof according to claim 2,
wherein both R^{8a} and R^{8b} in the formula (I) are each an alkyl group having 3 to 8 carbon atoms that may form a branch or a ring.

6. The compound or salt thereof according to claim 5,
wherein both R^{8a} and R^{8b} in the formula (I) are each a t-butyl group.

7. A method for producing an oligonucleotide or a pharmacologically acceptable salt thereof, the method comprising a step of synthesizing a nucleic acid using the compound or salt thereof according to any one of claims 1 to 6.

8. The method according to claim 7,
wherein the oligonucleotide is an oligonucleotide containing at least one phosphorothioate bond.

9. A compound represented by a formula (II) below or a salt thereof: 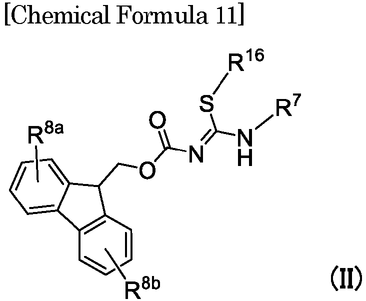 (where in the formula (II),
R⁷ is
(a) an alkyl group having 1 to 8 carbon atoms that may form a branch or a ring and may have undergone substitution by an alkoxy group having 1 to 8 carbon atoms that may form a branch or a ring;
(b) a cyanoalkoxy group having 1 to 8 carbon atoms;
(c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
(d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
(e) a group represented by a formula below: 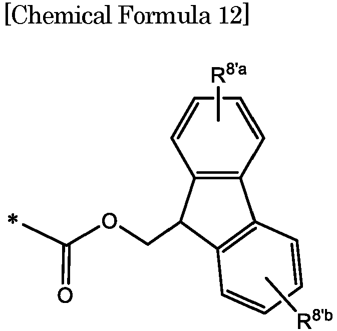 (where
R^{8'a} and R^{8'b} are each independently
(a) an alkyl group having 1 to 9 carbon atoms that may be branched;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site); and
R^{8a} and R^{8b} are each independently
(a) an alkyl group having 1 to 9 carbon atoms that may form a branch or a ring;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
R¹⁶ is a linear or branched alkyl group having 1 to 6 carbon atoms or a linear or branched alkenyl group having 2 to 6 carbon atoms).

10. A method for producing a compound represented by a formula (II) or a salt thereof, 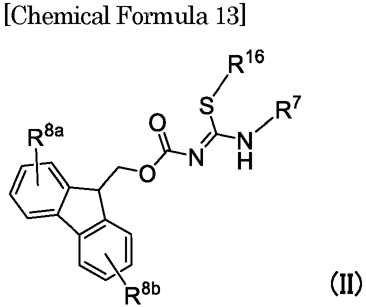 the method comprising a step of allowing a reaction product of a compound represented by a formula (III) below and a compound represented by a formula (IV) below to react with a compound represented by a formula (V) below: 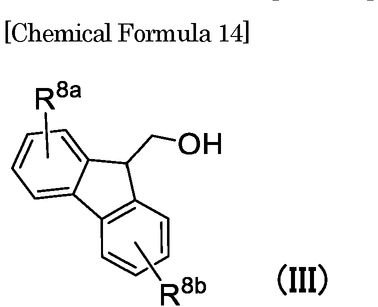 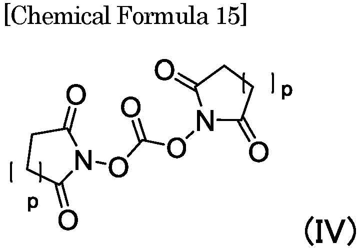 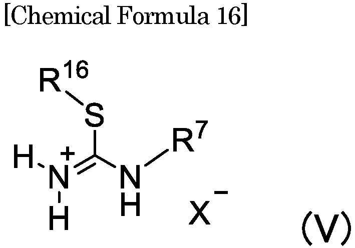 (where in the formula (III),
R^{8a} and R^{8b} are each independently
(a) an alkyl group having 1 to 9 carbon atoms that may form a branch or a ring;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis;
in the formula (IV),
p is independently an integer from 0 to 3,
in the formula (V),
R⁷ is
(a) an alkyl group having 1 to 8 carbon atoms that may form a branch or a ring and may have undergone substitution by an alkoxy group having 1 to 8 carbon atoms that may form a branch or a ring;
(b) a cyanoalkoxy group having 1 to 8 carbon atoms;
(c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
(d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
(e) a group represented by a formula below: 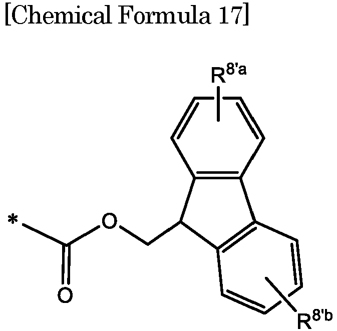 (where
R^{8'a} and R^{8'b} are each independently
(a) an alkyl group having 1 to 9 carbon atoms that may be branched;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site); and
R¹⁶ is a linear or branched alkyl group having 1 to 6 carbon atoms or a linear or branched alkenyl group having 2 to 6 carbon atoms, and
X⁻ is a counter anion.

11. A method for producing the compound represented by the formula (I) or salt thereof according to claim 1, the method comprising
a step of guanidinylating a compound represented by a formula (VI) below or a salt thereof: 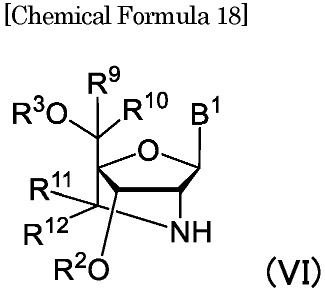 with a compound represented by a formula (II) or a salt thereof: 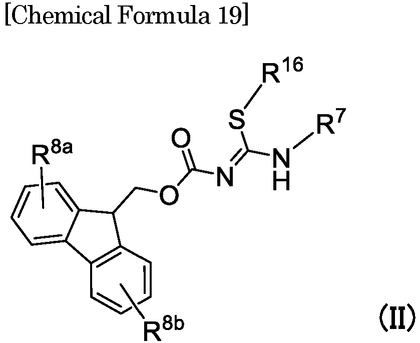 in the formula (VI),
B¹ is
(a) a purin-9-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms; or
(b) a 1,2-dihydropyrimidin-1-yl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms,
R² and R³ are each
(a) a hydrogen atom;
(b) a hydroxy group protecting group for nucleic acid synthesis;
(c) an alkyl group having 1 to 7 carbon atoms that may form a branch or a ring;
(d) an alkenyl group having 2 to 7 carbon atoms that may form a branch or a ring;
(e) an aryl group having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(f) a heteroaryl group having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(g) an aralkyl group with an aryl moiety having 6 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(h) a heteroaralkyl group with a heteroaryl moiety having 1 to 12 carbon atoms that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(i) an acyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(j) a silyl group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms;
(k) a phosphate group that may have at least one substituent selected from the group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms; or
(l) a phosphate group protected by a protecting group for nucleic acid synthesis;
R⁹ and R¹⁰ are each independently a hydrogen atom or an alkyl group having 1 to 6 carbon atoms that may form a branch or a ring, or R⁹ and R¹⁰ together constitute - (CH₂)ₘ- (where m is an integer from 2 to 7), and
R¹¹ and R¹² are each independently a hydrogen atom or an alkyl group having 1 to 6 carbon atoms that may form a branch or a ring, or
R¹¹ and R¹² together constitute an oxygen atom part (=O) of a carbonyl group, or
R¹¹ and R¹² together constitute -(CH₂)ₙ- (where n is an integer from 2 to 7), and
in the formula (II),
R⁷is
(a) an alkyl group having 1 to 8 carbon atoms that may form a branch or a ring and may have undergone substitution by an alkoxy group having 1 to 8 carbon atoms that may form a branch or a ring;
(b) a cyanoalkoxy group having 1 to 8 carbon atoms;
(c) an aralkyl group with an aryl moiety having 6 to 12 carbon atoms that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, an amino group, alkylamino groups having 1 to 8 carbon atoms that may form a branch or a ring, an amino group protected by a protecting group for nucleic acid synthesis, and halogen atoms;
(d) a heteroaralkyl group with a heteroaryl moiety having 1 to 12 carbon atoms that may have at least one substituent selected from the group consisting of a hydroxy group, a hydroxy group protected by a protecting group for nucleic acid synthesis, alkyl groups having 1 to 6 carbon atoms that may form a branch or a ring, alkoxy groups having 1 to 6 carbon atoms that may form a branch or a ring, a mercapto group, a mercapto group protected by a protecting group for nucleic acid synthesis, alkylthio groups having 1 to 6 carbon atoms that may form a branch or a ring, an amino group, alkylamino groups having 1 to 6 carbon atoms that may form a branch or a ring, an amino group protected by a protecting group for nucleic acid synthesis, and halogen atoms; or
(e) a group represented by a formula below: 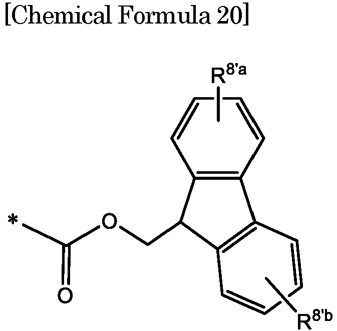 (where
R^{8'a} and R^{8'b} are each independently
(a) an alkyl group having 1 to 9 carbon atoms that may be branched;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, linear alkyl groups having 1 to 8 carbon atoms, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkoxy groups having 1 to 8 carbon atoms that may form a branch or a ring, alkylamino groups having 1 to 8 carbon atoms that may form a branch or a ring, and an amino group protected by a protecting group for nucleic acid synthesis; and
* is a bonding site); and
R^{8a} and R^{8b} are each independently
(a) an alkyl group having 1 to 9 carbon atoms that may form a branch or a ring;
(b) an alkoxy group having 1 to 8 carbon atoms that may form a branch or a ring;
(c) an alkylamino group having 1 to 8 carbon atoms that may form a branch or a ring; or
(d) a silyl group that may have at least one substituent selected from the group consisting of a hydroxy group, a hydroxy group protected by a protecting group for nucleic acid synthesis, alkyl groups having 1 to 8 carbon atoms that may form a branch or a ring, alkoxy groups having 1 to 8 carbon atoms that may form a branch or a ring, a mercapto group, a mercapto group protected by a protecting group for nucleic acid synthesis, alkylthio groups having 1 to 8 carbon atoms that may form a branch or a ring, alkylamino groups having 1 to 8 carbon atoms that may form a branch or a ring, and an amino group protected by a protecting group for nucleic acid synthesis; and
R¹⁶ is a linear or branched alkyl group having 1 to 6 carbon atoms or a linear or branched alkenyl group having 2 to 6 carbon atoms.

12. The production method according to claim 11,
wherein the guanidinylation step is carried out in the presence of a metal salt.

13. The production method according to claim 12,
wherein the metal salt is silver nitrate, silver chloride, silver bromide, silver iodide, silver trifluoromethanesulfonate, silver tetrafluoroborate, or silver hexafluorophosphate.

14. The production method according to claim 12,
wherein the metal salt is copper (I) chloride, copper (I) bromide, copper (I) iodide, copper (I) trifluoromethanesulfonate, copper (I) tetrafluoroborate, or copper (I) hexafluorophosphate.

15. The production method according to claim 11,
wherein R² in the formula (VI) is a hydrogen atom.

16. The production method according to any one of claims 11 to 15, comprising
a step of further converting, after the guanidinylation step, the resulting product into an amidite,
R² in the formula (I) being -P(R⁴)R⁵
(where
R⁴ and R⁵ are each independently
(a) a hydroxy group;
(b) a hydroxy group protected by a protecting group for nucleic acid synthesis;
(c) a mercapto group;
(d) a mercapto group protected by a protecting group for nucleic acid synthesis;
(e) an amino group;
(f) an alkoxy group having 1 to 5 carbon atoms;
(g) an alkylthio group having 1 to 5 carbon atoms;
(h) a cyanoalkoxy group having 1 to 6 carbon atoms;
(i) a dialkylamino group having alkyl groups that each have 1 to 6 carbon atoms and may be the same or different; or
(j) a cyclic alkylamino group having 3 to 6 carbon atoms; or
R⁴ and R⁵ together constitute a formula below: 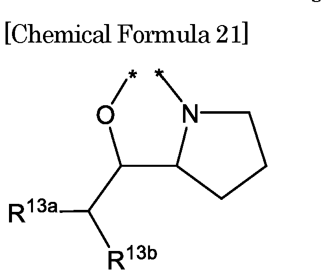
(where R^{13a} and R^{13b} are each independently a hydrogen atom, an alkyl group having 1 to 6 carbon atoms that may be branched, an aryl group having 6 to 12 carbon atoms, a nitro group, a halogen atom, a cyano group, Si(R^{a})₃ (where R^{a} is an alkyl group having 1 to 6 carbon atoms or an aryl group having 6 to 12 carbon atoms), a sulfonyl group, SO₂Ph, or SiMePh₂, and * is a bonding site); or 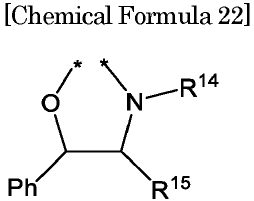
(where R¹⁴ and R¹⁵ are each independently a hydrogen atom, an alkyl group having 1 to 3 carbon atoms that may be branched, or a phenyl group, and * is a bonding site).

17. The production method according to claim 11, further comprising
a step of deprotecting a hydroxy group protecting group for nucleic acid synthesis, the protecting group being R² in the formula (VI).

18. The production method according to claim 17, comprising
a step of further converting, after the deprotection step, the resulting product into an amidite,
R² in the formula (I) being -P(R⁴)R⁵
(where
R⁴ and R⁵ are each independently
(a) a hydroxy group;
(b) a hydroxy group protected by a protecting group for nucleic acid synthesis;
(c) a mercapto group;
(d) a mercapto group protected by a protecting group for nucleic acid synthesis;
(e) an amino group;
(f) an alkoxy group having 1 to 5 carbon atoms;
(g) an alkylthio group having 1 to 5 carbon atoms;
(h) a cyanoalkoxy group having 1 to 6 carbon atoms;
(i) a dialkylamino group having alkyl groups that each have 1 to 6 carbon atoms and may be the same or different; or
(j) a cyclic alkylamino group having 3 to 6 carbon atoms; or
R⁴ and R⁵ together constitute a formula below: 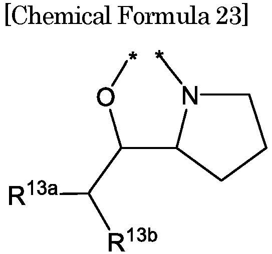
(where R^{13a} and R^{13b} are each independently a hydrogen atom, an alkyl group having 1 to 6 carbon atoms that may be branched, an aryl group having 6 to 12 carbon atoms, a nitro group, a halogen atom, a cyano group, Si(R^{a})₃ (where R^{a} is an alkyl group having 1 to 6 carbon atoms or an aryl group having 6 to 12 carbon atoms), a sulfonyl group, SO₂Ph, or SiMePh₂, and * is a bonding site); or 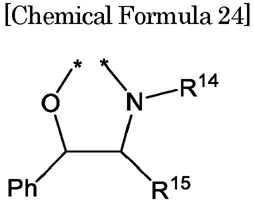
(where R¹⁴ and R¹⁵ are each independently a hydrogen atom, an alkyl group having 1 to 3 carbon atoms that may be branched, or a phenyl group, and * is a bonding site).
